(19) Europäisches Patentamt
European Patent Office
Office européen des brevets

(11) EP 2 117 517 B1

# (12) EUROPEAN PATENT SPECIFICATION

(45) Date of publication and mention of the grant of the patent:
**01.06.2011 Bulletin 2011/22**

(21) Application number: **08727550.9**

(22) Date of filing: **10.01.2008**

(51) Int Cl.:
***A61K 9/20*** *(2006.01)*

(86) International application number:
**PCT/US2008/050796**

(87) International publication number:
**WO 2008/086492 (17.07.2008 Gazette 2008/29)**

(54) **SUSTAINED RELEASE ORAL DOSAGE FORMS OF A PRODRUG OF R-BACLOFEN AND METHODS OF TREATMENT**

VERZÖGERT FREIGESETZTE ORALE DOSIERUNGSFORMEN EINES PRODRUGS VON R-BACLOFEN UND BEHANDLUNGSVERFAHREN DAMIT

FORMES GALÉNIQUES ORALES À LIBÉRATION PROLONGÉE D'UN PROMÉDICAMENT DE R-BACLOFÈNE ET PROCÉDÉS DE TRAITEMENT

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **11.01.2007 US 884598 P**

(43) Date of publication of application:
**18.11.2009 Bulletin 2009/47**

(73) Proprietor: **XenoPort, Inc.**
**Santa Clara, CA 95051 (US)**

(72) Inventors:
- **KIDNEY, David, J.**
  **San Jose, CA (US)**
- **CUNDY, Kenneth, C.**
  **Redwood City, CA 94062 (US)**
- **SASTRY, Srikonda**
  **Sunnyvale, CA 94087 (US)**
- **LEUNG, Manshiu**
  **Daly City, CA 94014 (US)**

(74) Representative: **Wytenburg, Wilhelmus Johannes et al**
**Mewburn Ellis LLP**
**33 Gutter Lane**
**London**
**EC2V 8AS (GB)**

(56) References cited:

**WO-A-2005/097079**
**WO-A-2008/011016**
**US-B2- 7 109 239**
**US-B2- 7 227 028**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 117 517 B1

## Description

### Field

The disclosure relates to sustained release oral dosage forms of *R*-baclofen and to methods of treating a disease comprising orally administering such dosage forms.

### Background

(±)-4-Amino-3-(4-chlorophenyl)butanoic acid (baclofen) is an analog of gamma-aminobutyric acid (i.e., GABA) that selectively activates $GABA_B$ receptors, resulting in neuronal hyperpolarization. $GABA_B$ receptors are located in laminae I-IV of the spinal cord, where primary sensory fibers end. These G-protein coupled receptors activate conductance by $K^+$-selective ion channels and can reduce currents mediated by $Ca^{2+}$ channels in certain neurons. Baclofen has a pre-synaptic inhibitory effect on the release of excitatory neurotransmitters and also acts postsynaptically to decrease motor neuron firing (see Bowery, Trends Pharmacol. Sci. 1989, 10, 401-407; and Misgeld et al., Prog. Neurobiol. 1995, 46, 423-462).

A principal pharmacological effect of baclofen in mammals is reduction of muscle tone and consequently the drug is frequently used in the treatment of spasticity. Spasticity is associated with damage to the corticospinal tract and is a common complication of neurological disease. Diseases and conditions in which spasticity may be a prominent symptom include cerebral palsy, multiple sclerosis, stroke, head and spinal cord injuries, traumatic brain injury, anoxia, and neurodegenerative diseases. Patients with spasticity complain of stiffness, involuntary spasm, and pain. These painful spasms may be spontaneous or triggered by a minor sensory stimulus, such as touching the patient.

Baclofen is also useful in controlling gastro-esophageal reflux disease (van Herwaarden et al., Aliment. Pharmacol. Ther. 2002, 16(9), 1655-62; Ciccaglione and Marzio, Gut 2003, 52(4), 464-70; Andrews et al., U.S. Patent No. 6,117,908; and Fara *et al.*, International Publication No. WO 02/096404); in promoting alcohol abstinence in alcoholics (Gessa *et al.*, International Publication No. WO 01/26638); in promoting smoking cessation (Gessa *et al.,* International Publication No. WO 01/08675); in reducing addiction liability of narcotic agents (Robson et al., U.S. Patent No. 4,126,684); in the treatment of emesis (Bountra et al., U.S. Patent No. 5,719,185); as an anti-tussive for the treatment of cough (Kreutner et al., U.S. Patent No. 5,006,560); in treating neuropathic pain (*see e.g.,* Fromm et al., Neurology 1981, 31 (6), 683-7; and Ringel and Roy, Ann Neurol 1987, 21(5), 514-5); and in treating musculoskeletal pain (*see e.g.,* Hering-Hanit, Cephalalgia 1999, 19(6), 589-591; Hering-Hanit and Gadoth, Headache 2000, 40(1), 48-51; Freitag, CNS Drugs 2003, 17(6), 373-81; Slonimski et al., Reg Anesth Pain Med 2004, 29(3), 269-76).

Baclofen may be administered orally or by intrathecal delivery through a surgically implanted programmable pump. The drug is rapidly absorbed from the gastrointestinal tract and has an elimination half-life of approximately 3-4 hours. Baclofen is partially metabolized in the liver but is largely excreted by the kidneys unchanged. The short half-life of baclofen necessitates frequent administration with typical oral dosing regimens ranging from about 10 mg to about 80 mg of three or four divided doses daily. Blood racemic baclofen concentrations of about 80 ng/mL to about 400 ng/mL result from these therapeutically effective doses in patients (Katz, Am. J. Phys. Med. Rehabil. 1988, 67(3), 108-16; and Krach, J Child Neurol. 2001, 16(1), 31-6). When baclofen is given orally, sedation is an adverse effect, particularly at elevated doses. Impairment of cognitive function, confusion, memory loss, dizziness, weakness, ataxia, and orthostatic hypotension are other commonly encountered adverse effects of baclofen therapy.

Intrathecal administration is often recommended for patients who find the adverse effects of oral baclofen intolerable. The intrathecal use of baclofen permits effective treatment of spasticity with doses less than $1/100^{th}$ of those required orally, because administration directly into the spinal subarachnoid space permits immediate access to the $GABA_B$ receptor sites in the dorsal horn of the spinal cord. Surgical implantation of a pump is, however, inconvenient and a variety of mechanical and medical complications can arise (e.g., catheter displacement, kinking or blockage, pump failure, sepsis, and deep vein thrombosis). Acute discontinuation of baclofen therapy (e.g., in cases of mechanical failure) may cause serious withdrawal symptoms such as hallucinations, confusion, agitation, and seizures (Sampathkumar et al., Anesth. Analg. 1998, 87, 562-563).

While the clinically prescribed baclofen product (Lioresal™) is available only as a racemate, the $GABA_B$ receptor agonist activity resides entirely in one enantiomer, *R*-(-)-baclofen (2) (also termed L-baclofen).

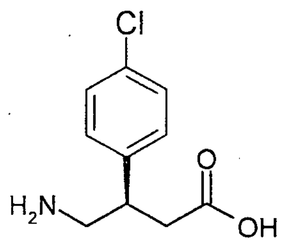

R-Baclofen (**2**)

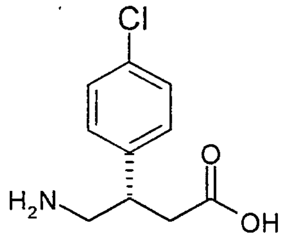

S-Baclofen (**3**)

The other isomer, *S*-baclofen, (**3**), antagonizes the action of *R*-baclofen at $GABA_B$ receptors and exhibits antinociceptive activity in the rat spinal cord (Terrence et al., Pharmacology 1983, 27, 85-94; and Sawynok et al., Pharmacology 1985, 31, 248-259). Orally administered *R*-baclofen is reported to be about 5 times more potent than orally administered racemic baclofen, with an *R*-baclofen regimen of 2 mg t.i.d being equivalent to racemic baclofen at 10 mg t.i.d. (Fromm et al., Neurology 1987, 37(11), 1725-8). Moreover, the adverse effect profile following administration of *R*-baclofen is significantly reduced relative to an equally efficacious dose of racemic baclofen.

As a zwitterionic amino acid, baclofen lacks the requisite physicochemical characteristics for effective passive permeability across cellular membranes. Passage of the drug across the gastrointestinal tract and the blood-brain barrier (BBB) is mediated primarily by active transport processes rather than by passive diffusion. Accordingly, baclofen is a substrate for active transport mechanisms shared by neutral α-amino acids such as leucine, and β-amino acids such as β-alanine and taurine (van Bree et al., Pharm. Res. 1988, 5, 369-371; Cercos-Fortea et al., Biopharm. Drug. Disp. 1995, 16, 563-577; Deguchi et al., Pharm. Res. 1995, 12, 1838-1844; and Moll-Navarro et al., J. Pharm. Sci. 1996, 85, 1248-1254). Transport across the BBB is stereoselective, with preferential uptake of the active *R*-enantiomer (**2**) being reported (van Bree et al., Pharm. Res. 1991, 8, 259-262). In addition, organic anion transporters localized in capillary endothelial cells of the BBB have been implicated in efflux of baclofen from the brain (Deguchi *et al.,* supra; and Ohtsuki et al., J Neurochem. 2002, 83, 57-66). 3-(*p*-Chlorophenyl)pyrrolidine has been described as a CNS-penetrable prodrug of baclofen (Wall et al., J. Med. Chem. 1989, 32, 1340-1348). Prodrugs of other $GABA_B$ agonists are described in Bryans *et al.,* International Publication No. WO 01/90052; Bryans et al., EP1178034; Cundy et al., U.S. Patent No. 6,992,076; Gallop et al., U.S. Patent Nos. 6,818,787, 6,927,036, and 6,972,341; and Raillard et al., U.S. Patent No. 7,232,924.

(3*R*)-4-{[(1*S*)-2-methyl-1-(2-methylpropanoyloxy)propoxy]carbonylamino}-3-(4-chlorophenyl)butanoic acid, (**1**),

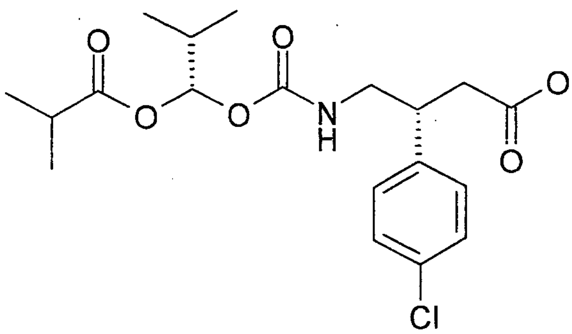

(**1**)

a prodrug of the $GABA_B$ agonist, *R*-baclofen (**2**) ((±)-4-amino-3-(4-chlorophenyl)butanoic acid), exhibits high bioavailability as *R*-baclofen when dosed either orally or directly into the colon of a mammal (Gallop et al., U.S. Patent Nos. 7,109,239 and 7,227,028).

The high *R*-baclofen oral bioavailability following administration of compound (**1**) favors the efficacious use of compound (**1**) in oral dosage forms, including sustained-release oral dosage forms, and the use of such oral dosage forms for treating diseases such as spasticity and gastro-esophageal reflux disease (van Herwaarden et al., Aliment. Pharmacol. Ther. 2002, 16(9), 1655-62; Ciccaglione and Marzio, Gut 2003, 52(4), 464-70; Andrews et al., U.S. Patent

No. 6,117,908; and Fara *et al.,* International Publication No. WO 02/096404); in promoting alcohol abstinence in alcoholics (Gessa *et al.,* International Publication No. WO 01/26638); in promoting smoking cessation (Gessa *et al.,* International Publication No. WO 01/08675); in reducing addiction liability of narcotic agents (Robson et al., U.S. Patent No. 4,126,684); in the treatment of emesis (Bountra et al., U.S. Patent No. 5,719,185); as an anti-tussive for the treatment of cough (Kreutner et al., U.S. Patent No. 5,006,560); as well as for treating movement disorders such as dystonia and hiccups; peripheral nerve disorders such as muscle stimulation disorders; spinal cord disorders such as spastic paraparesis; cranial nerve disorders such as glossopharyngeal neuralgia and trigeminal neuralgia; multiple sclerosis; and cerebral palsy.

The synthesis of compound (1) is described in Gallop et al., U.S. Patent Nos. 7,109,239 and 7,227,028. US Patent No 7,109,239 describes certain acyloxyalkyl carbamate prodrugs of (±)-4-amino-3-(4-chlorophenyl)butanoic acid and analogs thereof, pharmaceutical compositions comprising the prodrugs, and methods of treatment of, for example, spasticity and acid reflux disease.

US Patent No 7,227,028 describes methods of synthesis of certain 1-(acyloxy)-alkyl carbamate prodrugs of primary or secondary amine-containing drugs, as well as chemical intermediates used in such methods.

WO 2005/097079 describes certain pharmaceutical dosage forms having immediate release and controlled release properties that contain a GABA receptor agonist, for example, baclofen, for the treatment of conditions including spasms, cramping, and tightness of muscles associated with ailments such as multiple sclerosis and certain spinal injuries.

WO 2008/011016 describes certain methods of treatment of a gastrointestinal motility disorder, such as, gastroesophageal reflux disease, which involve co-administering a 5-HT3 receptor antagonist and a GABA receptor agonist.

## Summary of the Invention

A first aspect of the present invention is an oral dosage form comprising a tablet, wherein the tablet comprises (3*R*)-4-{[(1S)-2-methyl-1-(2-methylpropanoyloxy) propoxy]carbonylamino}-3-(4-chlorophenyl)butanoic acid or a pharmaceutically acceptable salt thereof, ammonioalkyl methacrylate copolymer, and hydroxypropylmethyl cellulose.

A second aspect of the present invention is an oral dosage form of the first aspect for use in a method of treatment of the human or animal body by therapy.

A third aspect of the present invention is an oral dosage form of the first aspect for use in a method of treatment of spasticity, gastro-esophageal reflux disease, emesis, cough, narcotic addiction or abuse, alcohol addiction or abuse, nicotine addiction or abuse, neuropathic pain, or musculoskeletal pain.

A fourth aspect of the present invention is use of (3*R*)-4-{[(1S)-2-methyl-1-(2-methylpropanoyloxy)propoxy]carbonylamino}-3-(4-chlorophenyl)butanoic acid or a pharmaceutically acceptable salt thereof in the manufacture of a dosage form of the first aspect for the treatment of spasticity, gastro-esophageal reflux disease, emesis, cough, narcotic addiction or abuse, alcohol addiction or abuse, nicotine addiction or abuse, neuropathic pain, or musculoskeletal pain.

The development of oral sustained release dosage forms comprising the *R*-baclofen prodrug (3*R*)-4-{[(1*S*)-2-methyl-1-(2-methylpropanoyloxy)propoxy]carbonylamino}-3-(4-chlorophenyl)butanoic acid (**1**) that provide enhanced bioavailability of *R*-baclofen can improve the convenience, efficacy, and adverse effect profile of *R*-baclofen therapy.

Described herein are oral dosage forms are provided comprising a tablet, wherein the tablet comprises (3*R*)-4-{[(1*S*)-2-methyl-1-(2-methylpropanoyloxy)propoxy]carbonylamino}-3-(4-chlorophenyl)butanoic acid or a pharmaceutically acceptable salt thereof, ammonioalkyl methacrylate copolymer and hydroxypropylmethyl cellulose.

Described herein are methods are provided for treating a disease such as spasticity, gastro-esophageal reflux disease, emesis, cough, narcotic addiction or abuse, alcohol addiction or abuse, nicotine addiction or abuse, neuropathic pain, or musculoskeletal pain in a patient comprising orally administering to a patient in need of such treatment an oral dosage form comprising the *R*-baclofen prodrug (3*R*)-4-{[(1*S*)-2-methyl-1-(2-methylpropanoyloxy)propoxy]carbonylamino}-3-(4-chlorophenyl)butanoic acid or a pharmaceutically acceptable salt thereof.

## Brief Description of the Drawings

Those skilled in the art will understand that the drawings, described herein, are for illustration purposes only.

**Figure 1** shows an *in vitro* dissolution profile for a controlled release capsule (CR) prepared according to Example 1.

**Figure 2** shows an *in vitro* dissolution profile for a sustained release tablet (SR1) prepared according to Example 2.

**Figure 3** shows an *in vitro* dissolution profile for a sustained release tablet (SR2) prepared according to Example 3.

**Figure 4** shows an *in vitro* dissolution profile for a sustained release tablet (SR3) prepared according to Example 4.

**Figure 5** shows the mean concentration of *R*-baclofen in blood of fasted dogs following oral administration of

CR, SR1, SR2, or SR3 dosage forms comprising compound (**1**) prepared according to Examples 1-4 at a dose of 10 mg compound (1**)**.

**Figure 6** shows the mean concentration of *R*-baclofen in blood of fasted healthy human patients following oral administration of CR dosage forms comprising 10 mg compound (**1**) prepared according to Example 1 at doses of 10 mg, 20 mg, 30 mg, 40 mg, 60 mg, or 80 mg compound (**1**).

**Figure 7** shows the correlation between the maximum blood concentration of *R*-baclofen and the dose of compound (**1**) administered as CR capsules to fasted healthy human patients.

**Figure 8** shows the correlation between the $AUC_{inf}$ of *R*-baclofen and the dose of compound (**1**) administered as CR capsules to fasted healthy human patients.

**Figure 9** shows the blood concentration of *R*-baclofen and compound (1) following administration of 80 mg compound (**1**) administered as CR capsules to fasted healthy human patients.

**Figure 10** shows the mean (SD) concentrations of *R*-baclofen in blood of fasted healthy human patients following oral adminstration of SR1, SR2, or SR3 tablet formulations at a dose of 20 mg (2 10 mg) compound (**1**).

**Figure 11** shows the mean (SD) concentrations of *R*-baclofen in blood of fed healthy human patients following oral adminstration of SR1, SR2, or SR3 tablet formulations at a dose of 20 mg (2 10 mg) compound (**1**).

**Figure 12** shows the mean (SD) concentration of *R*-baclofen in blood of fasted dogs and fasted humans following administration of sustained release tablet formulation SR3 at a dose of 10 mg compound (**1**) and a dose of 20 mg (2 10 mg) compound (**1**), respectively.

**Figure 13** shows the mean (SD) concentrations of *R*-baclofen in blood at steady state (Day 7) after once daily oral dosing of 30 mg (3 10 mg), 60 mg (6 10 mg), or 90 mg (9 10 mg) doses of compound (**1**) as SR3 tablet formulations in healthy human patients.

**Figure 14** shows the mean (SD) concentrations of *R*-baclofen in blood at steady state (Day 14) after twice daily oral dosing of 30 mg (3 10 mg), 60 mg (6 10 mg), or 90 mg (9 10 mg) doses of compound (**1**) as SR3 tablet formulations in healthy human patients.

**Figure 15** shows the correlation between the dose of compound (**1**) and the mean steady state $C_{max,\ ss}$ of *R*-baclofen in blood following once daily (QD) or twice daily (BID) oral dosing of 30 mg (3 10 mg), 60 mg (6 10 mg), 90 mg (9 10 mg), or 120 mg (12 10 mg) doses of compound (**1**) administered as SR3 tablet formulations in healthy human patients.

**Figure 16** shows the correlation between the dose of compound (**1**) and the mean steady state $AUC_{0-24}$ of *R*-baclofen in blood following once daily (QD) or twice daily (BID) oral dosing of 30 mg (3 10 mg), 60 mg (6 10 mg), 90 mg (9 10 mg), or 120 mg (12 10 mg) doses of compound (**1**) administered as SR3 tablet formulations in healthy human patients.

**Figure 17** shows the mean (SD) trough concentrations of *R*-baclofen in blood following once daily oral (QD) oral dosing of 30 mg (3 10 mg), 60 mg (6 10 mg), 90 mg (9 10 mg), or 120 mg (12 10 mg) doses of compound ( **1**) as SR3 tablet formulations in healthy human patients.

**Figure 18** shows the mean (SD) trough blood concentrations of *R*-baclofen in blood following twice daily (BID) oral dosing of 30 mg (3 10 mg), 60 mg (6 10 mg), 90 mg (9 10 mg), or 120 mg (12 10 mg) doses of compound ( **1**) as SR3 tablet formulations in healthy human patients.

## Detailed Description

### Definitions

"Adverse drug effects" refers to drug effects that are unwanted, unpleasant, noxious, or potentially harmful. Adverse drug effects can be mild such as digestive disturbance, headaches, fatigue, vague muscle aches, malaise, and changes in sleep patterns. Moderate adverse drug effects represent reactions that a person experiencing them considers annoying, distressing, or intolerable such as skin rashes, visual disturbances, muscle tremor, difficulty with urination, perceptible changes in mood or mental function, and certain changes in blood components. Examples of severe adverse drug effects include reactions that may be life threatening, that result in persistent or significant disability or hospitalization, and that cause a birth defect. Examples of adverse effects known to be associated with baclofen therapy include sedation, impairment of cognitive function, confusion, memory loss, dizziness, weakness, ataxia, blurred or double vision, nausea, shortness of breath, convulsions, and orthostatic hypotension.

"AUC" is the area under a curve representing the concentration of a compound or metabolite thereof in a biological fluid of a patient as a function of time following administration of the compound to the patient. For example, the administered compound can be the *R*-baclofen prodrug (**1**) and the corresponding metabolite *R* - baclofen. Examples of biological fluids include plasma and blood. The AUC may be determined by measuring the concentration of a compound or metabolite thereof in a biological fluid such as the plasma or blood using methods such as liquid chromatography-tandem mass spectrometry (LC/MS/MS), at various time intervals, and calculating the area under the plasma or blood

concentration-versus-time curve. The concentration versus time curve is also referred to as the pharmacokinetic profile. Suitable methods for calculating the AUC from a drug concentration-versus-time curve are well known in the art. For example, an AUC for *R* -baclofen may be determined by measuring the concentration of *R* -baclofen in the plasma or blood of a patient following administration of a *R* -baclofen prodrug, such as compound (**1**), to the patient. $AUC_{0-24}$ is the area under the curve from administration (time 0) to 24 hours following administration.

"Bioavailability" refers to the rate and amount of a drug that reaches the systemic circulation of a patient following administration of the drug or prodrug thereof to the patient and can be determined by evaluating, for example, the plasma or blood concentration-versus-time profile for a drug. Parameters useful in characterizing a plasma or blood concentration-versus-time curve include the area under the curve (AUC), the time to peak concentration ($T_{max}$), and the maximum drug concentration ($C_{max}$), where $C_{max}$ is the maximum concentration of a drug in the plasma or blood of a patient following administration of a dose of the drug or prodrug thereof to the patient, and $T_{max}$ is the time to the maximum concentration ($C_{max}$) of a drug in the plasma or blood of a patient following administration of a dose of the drug or prodrug thereof to the patient.

Absolute oral bioavailability is the bioavailability of a compound or metabolite thereof following oral administration compared to the bioavailability following intravenous administration of an equiavalent amout of the compound or metabolite thereof. Relative oral bioavailability of a compound or metabolite thereof is the bioavailability following oral administration of a compound or metabolite thereof relative to administration of an equivalent amount of the compound or metabolite thereof in another dosage form and/or route of adminsitraation. For example, in certain embodiments, relative oral bioavailability expressed as $\%F_{rel}$ is the bioavailability of *R*-baclofen determined by the $AUC_{0-24}$ following oral administration of compound (**1**) to a patient relative to the bioavailability of *R*-baclofen following oral administration of 20 mg compound (**1**) as a CR capsule.

"Bioequivalence" refers to equivalence of the rate and extent of absorption of a drug after administration of equal doses of the drug or prodrug to a patient. As used herein, two pharmacokinetic profiles are bioequivalent if the 90% confidence interval for the ratio of the mean response of the two profiles is within the limits of 0.8 and 1.25. The mean response includes at least one of the characteristic parameters of a profile such as $C_{max}$, $T_{max}$, and AUC.

"Compound (**1**)" includes the *R*-baclofen prodrug compound (**1**), (3*R*)-4-{[(1*S*)-2-methyl-1-(2-methylpropanoyloxy)propoxy]carbonylamino}-3-(4-chlorophenyl)butanoic acid, pharmaceutically acceptable salts thereof, and crystalline forms of any of the foregoing. Compound (**1**) is used interchangeably with *R*-baclofen prodrug (**1**). In certain embodiments, *R*-baclofen prodrug compound (**1**), (3*R*)-4-{[(1*S*)-2-methyl-1-(2-methylpropanoyloxy)propoxy]carbonylamino}-3-(4-chlorophenyl)butanoic acid, is the free acid. In certain embodiments, *R*-baclofen prodrug compound (1), (3*R*)-4-{[(1*S*)-2-methyl-1-(2-methylpropanoyloxy)propoxy]carbonylamino}-3-(4-chlorophenyl)butanoic acid, is the hydrochloride salt.

"$C_{max}$" is the maximum drug concentration observed in the blood or plasma of a patient following administration of a dose of drug or prodrug thereof to the patient. $C_{max,ss}$ is the maximum steady state concentration following a dosing regimen administered over a period of days.

"$C_{12}$" is the drug concentration observed in the blood or plasma of a patient twelve (12) hours after administration of a dose of a drug or prodrug thereof to the patient.

"$T_{max}$" is the time to the maximum concentration ($C_{max}$) of a drug in the plasma or blood of a patient following administration of a dose of the drug or prodrug thereof to the patient.

"$T_{1/2}$" is the time interval between $T_{max}$ and the time at which the drug concentration in the blood or plasma of a patient has decreased to one-half the maximum drug concentration.

"Controlled-release" refers to release of a drug from a dosage form in which the drug release is controlled or modified over a period of time. Controlled can mean, for example, sustained, delayed, or pulsed-release at a particular time. Controlled can also mean that release of the drug from the dosage form is extended for longer than it would be in an immediate-release dosage form, i.e., at least over several hours.

"Dosage form" refers to a form of a formulation that contains an amount of active agent or prodrug of an active agent, e.g., the *R* -baclofen prodrug (**1**), which can be administered to a patient to achieve a therapeutic effect. An oral dosage form is intended to be administered to a patient via the mouth and swallowed. Examples of oral dosage forms include capsules, tablets, and liquid suspensions. A dose of a drug may include one or more dosage forms administered simultaneously or over a period of time.

"Fasted patient" refers to a patient whose stomach is substantially free of food at the time a dose is administered to the patient and for at least 4 hours following administration. The time at which a patient's stomach becomes substantially free of food following a meal can depend on a number of factors including, for example, the size of the meal such as the number of calories, the content of the meal such as the fat content, the health of the patient, and the condition of the patient's gastrointestinal tract. The stomach of a healthy human subject is typically substantially free of food after about 4 to about 8 following ingestion of food. In certain embodiments, a fasted patient does not eat any food (but can ingest any amount of water or clear liquid) from about 10 hours prior to dosing until about 4 hours after dosing, drinks about 250 mL of water about 2 hours and about 1 hour prior to dosing and about 250 mL of water about 2 hours after dosing, eats a lunch about 4 hours after dosing, and eats a dinner about 10 hours after dosing.

"Fed patient" refers to a patient whose stomach contains food. In certain embodiments, a fed patient begins eating a test meal about 30 minutes prior to dosing and completes eating the test meal about 5 minutes prior to dosing, eats a lunch 4 hours after dosing, and eats a dinner about 10 hours after dosing. A test meal may comprise a high fat (about 50% of the total number of calories in the test meal) and high calorie (about 1000 total calories) breakfast such as, for example, 2 eggs fried in butter, 2 strips of bacon, 2 slices of wheat toast with butter, 4 ounces of hash brown potatoes, and 8 ounces of whole milk. A test meal may contain about 150 protein calories, 250 carbohydrate calories, and about 500 to 600 fat calories.

"Immediate release" refers to formulations or dosage forms that rapidly dissolve *in vitro* and *in vivo* and are intended to be completely dissolved and absorbed in the stomach or upper gastrointestinal tract. Immediate release formulations can release at least 90% of the active ingredient or precursor thereof within about 15 minutes, within about 30 minutes, within about one hour, or within about two hours of administering an immediate release dosage form.

"Minimum adverse concentration" refers to the minimum concentration of a therapeutic compound in, for example, the blood or plasma of a patient, which does not produce an unacceptable adverse drug effect. The unacceptability of an adverse drug effect can be determined, for example, by the patient and/or the prescribing physician based at least in part on the severity of the adverse drug effect and/or the perceived risk in view of the therapeutic benefits of the compound being administered to the patient. The minimum adverse concentration may also depend, at least in part, on the age, weight and health of the patient being treated, the disease being treated, the frequency and severity of the symptoms, and the judgment of the prescribing physician.

"Minimum therapeutically effective concentration" refers to the minimum concentration of a therapeutic compound in, for example, the blood or plasma of a patient that produces an intended therapeutic effect.

"Patient" includes mammals, such as for example, humans.

"Pharmaceutically acceptable" refers to approved or approvable by a regulatory agency of a federal or a state government, listed in the U.S. Pharmacopeia, or listed in other generally recognized pharmacopeia for use in mammals, including humans.

"Pharmaceutically acceptable salt" refers to a salt of a compound such as compound (**1**) that is pharmaceutically acceptable and that possesses the desired pharmacological activity of the parent compound. Such salts include: (1) acid addition salts, formed with inorganic acids such as hydrochloric acid, hydrobromic acid, sulfuric acid, nitric acid, phosphoric acid, and the like; or formed with organic acids such as acetic acid, propionic acid, hexanoic acid, cyclopentanepropionic acid, glycolic acid, pyruvic acid, lactic acid, malonic acid, succinic acid, malic acid, maleic acid, fumaric acid, tartaric acid, citric acid, benzoic acid, 3-(4-hydroxybenzoyl) benzoic acid, cinnamic acid, mandelic acid, methanesulfonic acid, ethanesulfonic acid, 1,2-ethane-disulfonic acid, 2-hydroxyethanesulfonic acid, benzenesulfonic acid, 4-chlorobenzenesulfonic acid, 2-naphthalenesulfonic acid, 4-toluenesulfonic acid, camphorsulfonic acid, 4-methylbicyclo [2.2.2]-oct-2-ene-1-carboxylic acid, glucoheptonic acid, 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, and the like; and (2) salts formed when an acidic proton present in the parent compound either is replaced by a metal ion, e.g., an alkali metal ion, an alkaline earth metal ion, or an aluminum ion; or coordinates with an organic base such as ethanolamine, diethanolamine, triethanolamine, *N*-methylglucamine, and the like. In certain embodiments, a salt of compound (**1**) is the hydrochloride salt, and in certain embodiments, the sodium salt.

"Pharmaceutically acceptable vehicle" refers to a pharmaceutically acceptable diluent, a pharmaceutically acceptable adjuvant, a pharmaceutically acceptable excipient, a pharmaceutically acceptable carrier, or a combination of any of the foregoing with which a compound such as the *R*-baclofen prodrug, (3*R*)-4-{[(1*S*)-2-methyl-1-(2-methylpropanoyloxy)propoxy]carbonylamino}-3-(4-chlorophenyl)butanoic acid (**1**), may be administered to a patient, which does not destroy the pharmacological activity thereof, and which is nontoxic when administered in doses sufficient to provide a therapeutically effective amount of the *R*-baclofen prodrug or *R*-baclofen metabolite.

"Pharmaceutical composition" refers to a composition comprising the *R*-baclofen prodrug (**1**) or a pharmaceutically acceptable salt thereof and at least one pharmaceutically acceptable vehicle, with which the prodrug is to be administered to a patient.

"Prodrug" refers to a derivative of an active compound (drug) that undergoes a transformation under the conditions of use, such as within the body, to release an active drug. Prodrugs are frequently, but not necessarily, pharmacologically inactive until converted into the active drug. Prodrugs can be obtained by bonding a promoiety (defined herein), typically via a functional group, to a drug. For example, *R*-baclofen prodrug (1) is metabolized within a patient's body to form the parent drug *R*-baclofen.

"Promoiety" refers to a group bonded to a drug, typically to a functional group of the drug, via bond(s) that are cleavable under specified conditions of use. The bond(s) between the drug and promoiety may be cleaved by enzymatic or non-enzymatic means. Under the conditions of use, for example following administration to a patient, the bond(s) between the drug and promoiety may be cleaved to release the parent drug. The cleavage of the promoiety may proceed spontaneously, such as via a hydrolysis reaction, or it may be catalyzed or induced by another agent, such as by an enzyme, by light, by acid, or by a change of or exposure to a physical or environmental parameter, such as a change

of temperature, pH, etc. The agent may be endogenous to the conditions of use, such as an enzyme present in the systemic circulation of a patient to which the prodrug is administered or the acidic conditions of the stomach, or the agent may be supplied exogenously. For example, for *R*-baclofen prodrug (**1**), the drug is *R*-baclofen and the promoiety has the structure:

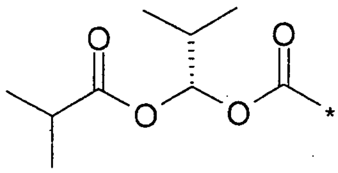

"Sedation" as used herein refers to minimal sedation and/or moderate sedation (*see e.g.,* American Society of Anesthesiologists, Anesthesiology 2002, 96, 1004-17). Minimal sedation, also referred to as anxiolysis, is a minimally depressed level of consciousness that retains the patient's ability to independently and continuously maintain an airway and respond appropriately to physical stimulation or verbal command that is produced by a pharmacological or non-pharmacological method or combination thereof. Although cognitive function and coordination may be modestly impaired, ventilatory and cardiovascular functions are unaffected. When the intent is minimal sedation in adults, the appropriate dosing is no more than the maximum recommended dose that can be prescribed for unmonitored home use, e.g., a maximum recommended therapeutic dose. Moderate sedation is a drug-induced depression of consciousness during which patients respond purposefully to verbal commands, either alone or accompanied by light tactile stimulation. No intervention is required to maintain a patient's airway. Sedation is a continuum and it is not always possible to predict how an individual patient will respond. A sedative dose can be determined by incremental dosing, administering multiple doses of a drug, such as *R*-baclofen prodrug (**1**), until a desired effect is reached. A variety of scales can be used to assess sedation including, for example, the Ramsay scale (Ramsay et al., Br Med J 1974, 2, 656-659), the Observer's Assessment of Alertness/Sedation scale (Chernik et al., J Clin Psychopharmacol 1990, 10, 244-251), and others (*see e.g.,* Sessler, Chest 2004, 126, 1727-1730). Objective measures of sedation include measurement of electroencephalogram parameters such as the Bispectral Index version XP and the Patient State Analyzer (*see, e.g.,* Chisholm et al., Mayo Clin Proc 2006, 81(1), 46-52; and Tonner et al., Best Pract Res Clin Anaesthesiol 2006, 20(1), 191-2000). In certain embodiments, sedation refers to minimal sedation, and in certain embodiments, to moderate sedation.

"Solvate" refers to a molecular complex of a compound with one or more solvent molecules in a stoichiometric or non-stoichiometric amount. Such solvent molecules are those commonly used in the pharmaceutical arts, e.g., water, ethanol, and the like. A molecular complex of a compound or moiety of a compound and a solvent can be stabilized by non-covalent intra-molecular forces such as, electrostatic forces, van der Waals forces, and hydrogen bonds. The term "hydrate" refers to a complex in which the one or more solvent molecules are water including monohydrates and hemi-hydrates.

"Sustained release" refers to release of a compound from a dosage form at a rate effective to achieve a therapeutic amount of the compound, or active metabolite thereof, in the systemic blood circulation over a prolonged period of time relative to that achieved by oral administration of an immediate formulation of the compound. In some embodiments, *in vivo* release of the compound occurs over a period of at least about 4 hours, in some embodiments, over a period of at least about 8 hours, in some embodiments over a period of at least about 12 hours, in some embodiments, over a period of at least about 16 hours, in some embodiments, over a period of at least about 20 hours, and in some embodiments, over a period of at least about 24 hours.

"Therapeutically effective amount" refers to the amount of a compound that, when administered to a subject for treating a disease or disorder, or at least one of the clinical symptoms of a disease or disorder, is sufficient to affect such treatment of the disease, disorder, or symptom. The therapeutically effective amount may vary depending, for example, on the compound, the disease, disorder, and/or symptoms of the disease, severity of the disease or disorder, and/or symptoms of the disease or disorder, the age, weight, and/or health of the patient to be treated, and the judgment of the prescribing physician. A therapeutically effective amount may be ascertained by those skilled in the art or capable of determination by routine experimentation.

"Treating" or "treatment" of any disease refers to arresting or ameliorating a disease or at least one of the clinical symptoms of a disease or disorder, reducing the risk of acquiring a disease or at least one of the clinical symptoms of a disease, reducing the development of a disease or at least one of the clinical symptoms of the disease or reducing the risk of developing a disease or at least one of the clinical symptoms of a disease. "Treating" or "treatment" also refers to inhibiting the disease, either physically, (e.g., stabilization of a discernible symptom), physiologically, (e.g., stabilization of a physical parameter), or both, and to inhibiting at least one physical parameter that may or may not be discernible

to the patient. In certain embodiments, "treating" or "treatment" refers to delaying the onset of the disease or at least one or more symptoms thereof in a patient which may be exposed to or predisposed to a disease or disorder even though that patient does not yet experience or display symptoms of the disease.

Reference is now made in detail to certain embodiments of dosage forms and methods. The disclosed embodiments are not intended to be limiting of the claims. To the contrary, the claims are intended to cover all alternatives, modifications, and equivalents.

**Sustained Release Oral Dosage Forms**

Dosage forms provided by the present disclosure comprise the *R*-baclofen prodrug (**1**). Dosage forms are tablets.

Controlled release capsules comprise a plurality of controlled release particles, each of the controlled release particles comprising immediate release particles, which comprise a core and a coating comprising compound (**1**) and a binder. Immediate release particles release compound (**1**) upon contact with gastrointestinal fluid more rapidly than controlled release particles and in a manner that does not affect the rate of absorption of compound (**1**) from the gastrointestinal tract.

Cores may be inert or active. Active cores comprise compound (1) and optionally additional components such a binder.

In embodiments in which cores are inert, inert cores are coated with an immediate release coating comprising compound (**1**) to form immediate release particles. In embodiments in which cores are active, cores comprising compound (**1**) may be coated with an immediate release coating to provide immediate release particles, or cores comprising compound (**1**), with or without a coating, may be used as immediate release particles. In certain embodiments, immediate release particles may comprise uncoated granules or powders of compound (**1**), may comprise inert cores having a coating of compound (**1**), or may include granules or pellets of compound (**1**) coated with a highly soluble immediate release coating.

Inert cores may comprise any appropriate type of core material useful in pharmaceutical applications, and which may be water insoluble, such as cellulose spheres or silicon dioxide, or may be water soluble such as starch and/or sugar. Cores may comprise beads, ion-exchange resin beads, spheroids, spheres, seeds, pellets, granules, or other particulate form. Cores may comprise a material such as sugar, starch, sugar and starch, sucrose crystals, extruded and dried spheres comprising excipients such as microcrystalline cellulose and lactose, or an acidic or alkaline buffer crystal such as calcium carbonate, sodium bicarbonate, fumaric acid, tartaric acid, or combinations of any of the foregoing, which may alter the microenvironment of the compound (**1**) to facilitate release of the compound (**1**) and/or impact the chemical stability of compound (**1**). In certain embodiments, inert cores comprise sugar (Sugar Sphere NF). Inert cores may have any appropriate dimension suitable for oral delivery. For example, inert cores may have a diameter ranging from about 15 mesh to about 50 mesh, from about 20 mesh to about 25 mesh, or from about 30 mesh to about 35 mesh. In certain embodiments, inert cores may have a diameter ranging from about 0.25 mm to about 3 mm, and in certain embodiments, from about 0.5 mm to about 1 mm.

To prepare immediate release particles, inert cores may be coated with a formulation comprising compound (**1**) that provides for immediate release of compound (**1**). Coatings may further comprise a binding agent to provide adhesion between cores and compound (**1**). Binding agents may be water-soluble and may include any appropriate binding agent such as polyvinylpyrrolidone, hydroxyethyl cellulose, hydroxypropyl cellulose, low molecular weight hydroxypropylmethyl cellulose (HPMC), polymethacrylate, ethyl cellulose, or combinations of any of the foregoing. In certain embodiments; a binding agent is a polyvinylpyrrolidone polymer such as Plasdone® K29/32 Povidone USP/NF. Plasdone K29-32 is a linear homopolymer of vinyl pyrrolidone having a K-value from about 29 to about 32, a viscosity in 5% aqueous solution of about 2.5 cp at 25 °C, a nominal molecular weight of 58×103, and a glass transition temperature, Tg, of 164 °C. In certain embodiments, coating compositions comprise an amount of binder ranging from about 2 wt% to about 10 wt%, and in certain embodiments from about 4 wt% to about 6 wt% based on the total solids weight of the coating composition.

Coatings comprising compound (**1**) may be applied to inert cores by any appropriate method known in the pharmaceutical industry such as fluidized bed coating, rotor granulation, pan coating, or spray coating. Suspensions of compound (**1**) and binder may be formed in a low viscosity solvent such as isopropyl alcohol, ethanol, acetone, water, or mixtures of any of the foregoing. The suspensions may then be applied to inert cores to provide a coating thickness sufficient to provide a desired amount of compound (**1**). These compound (**1**) coated cores may then be used as immediate release particles.

In certain embodiments, immediate release particles may comprise particles having an active core comprising compound (**1**). Active cores comprising compound (**1**) may further comprise any appropriate vehicle, for example, any of those disclosed herein. Cores comprising compound (**1**) may be granules formed by granulation methods known to those skilled in the art. Cores comprising compound (**1**) may be coated with a coating that may or may not comprise

compound (**1**).

In certain embodiments, one or more coatings may be applied to immediate release particles having a coating comprising compound (**1**) or that comprise a core of compound (**1**). The purpose of the one or more additional coatings may be for physical protection and/or to facilitate further processing of the particles. These sealant or barrier coatings are not intended to modify or affect the release of compound (**1**) from the immediate release particles or from dosage forms comprising the particles. These additional coatings may be applied to the particles comprising compound (**1**) by methods known to those skilled in the art and as disclosed herein. Examples of materials useful in coatings to provide physical protection include permeable or soluble materials such as hydroxypropyl methylcellulose, hydroxypropyl cellulose, hydroxypropyl ethylcellulose, and xanthan gum. Examples of materials useful in coatings to facilitate processing include talc, colloidal silica, polyvinyl alcohol, titanium dioxide, micronized silica, fumed silica, glycerol monostearate, magnesium trisilicate, magnesium stearate, and combinations of any of the foregoing.

In certain embodiments, one or more additional coatings that impart desired release properties may be applied to immediate release particles comprising compound (**1**). Such particles are referred to as controlled-release particles. Controlled release particles have a controlled-release coating surrounding the immediate release particles. Controlled-release coatings modify or control release of compound (**1**) from a controlled-release particle in the gastrointestinal tract. Useful controlled-release coating materials include bioerodible, gradually hydrolysable, gradually water-soluble, enzymatically degradable polymers, and/or enteric polymers.

Examples of coating materials for effecting controlled or modified release include, but are not limited to, cellulosic polymers such as hydroxypropyl cellulose, hydroxyethyl cellulose, hydroxymethyl cellulose, hydroxypropyl methyl cellulose, hydroxypropyl methyl cellulose acetate succinate, hydroxypropyl methyl cellulose phthalate, methylcellulose, ethyl cellulose, cellulose acetate, cellulose acetate phthalate, cellulose acetate trimellitate, and carboxymethylcellulose sodium; acrylic acid and methacrylic acid copolymers, methyl methacrylate copolymers, ethoxyethyl methacrylates, cyanoethyl methacrylate, poly(acrylic acid), poly(methacrylic acid), methacrylic acid alkylamide copolymer, poly(methyl methacrylate), polymethacrylate, poly(methyl methacrylate) copolymers, polyacrylamide, aminoalkyl methacrylate copolymer, poly(methacrylic acid anhydride), glycidyl methacrylate copolymers, ammonioalkyl methacrylate copolymers, and methacrylic resins commercially available under the tradename Eudragit® including Eudragit® L, Eudragit® S, Eudragit® E, Eudragit® RL, and Eudragit® RS; vinyl polymers and copolymers such as polyvinylpyrrolidone, vinyl acetate, vinylacetate phthalate, vinylacetate crotonic acid copolymer, and ethylene-vinyl acetate copolymer; enzymatically degradable polymers such as azo polymers, pectin, chitosan, amylase, and guar gum; and shellac. Combinations of any of the foregoing polymers may also be used to form controlled-release coatings. In certain embodiments, more than one controlled release coating may be applied to immediate release particles to modify the release properties of compound (**1**) from controlled release particles, each controlled release coating comprising one or more controlled release polymers.

In certain embodiments, controlled-release coatings may provide pH-independent release of compound (**1**). pH-Independent release coatings release compound (**1**) from controlled release particles at a rate independent of the pH of the fluid in which the particles are immersed. Ammonioalkyl methacrylate copolymers such as Eudragit® RS and Eudragit® RL are examples of useful pH-independent release polymers. Eudragit® RL and Eudragit® RS are acrylic resins comprising copolymers of acrylic and methacrylic acid esters with a low content of quaternary ammonium groups. The ammonium groups are present as salts and impart permeability to the lacquer films formed by the cured resins. Eudragit® RL and Eudragit® RS swell in water and gastrointestinal fluids in a pH-independent manner. In the swollen state the coatings are permeable to water and release or dissolve active compounds. Eudragit® RL and Eudragit® RS may be used alone in pH-independent release coatings, combined together, combined with other ammonioalkyl methacrylate copolymers, or combined with other methacrylic acid copolymers to achieve a desired release property. In certain embodiments, pH independent release polymers are ammonioalkyl methacrylate copolymers such as Eudragit® RL 100.

Coatings used to form immediate- and controlled-release particles provided by the present disclosure may comprise one or more pharmaceutically acceptable vehicles such as, for example, surfactants, lubricants, diluents, plasticizers, anti-adherents, glidants, buffers, disintegrants, fillers, wetting agents, emulsifying agents, pH buffering agents, pH-modifying agents, stabilizing agents, chelating agents, binders, thickening agents, coloring agents, or combinations of any of the foregoing.

Examples of surfactants useful in pharmaceutically acceptable coatings provided by the present disclosure include pharmaceutically acceptable anionic surfactants, cationic surfactants, amphoteric (amphiphatic/ampophilic) surfactants, non-ionic surfactants, polyethyleneglycol esters or ethers, and combinations of any of the foregoing. Examples of useful pharmaceutically acceptable anionic surfactants include monovalent alkyl carboxylates, acyl lactylates, alkyl ether carboxylates, *N*-acyl sarcosinates, polyvalent alkyl carbonates, *N*-acyl glutamates, fatty acid-polypeptide condensates, sulfuric acid esters, alkyl sulfates such as sodium lauryl sulfate, ethoxylated alkyl sulfates, ester linked sulfonates such as docusate sodium and dioctyl sodium succinate, alpha olefin sulfonates, or phosphated ethoxylated alcohols. Examples of pharmaceutically acceptable cationic surfactants useful in coatings provided by the present disclosure include monoalkyl quaternary ammonium salts, dialkyl quaternary ammonium compounds, amidoamines, and aminimides. Examples of useful pharmaceutically acceptable amphoteric surfactants include *N*-substituted alkyl amides, *N*-alkyl betaines, sulfobetaines, and *N*-alkyl-6-aminopropionates. Examples of pharmaceutically acceptable polyethyleneglycol esters or ethers useful in coatings provided by the present disclosure include polyethoxylated castor oil, polyethoxylated hydrogenated castor oil, and hydrogenated castor oil.

Lubricants and anti-static agents may be included in a pharmaceutically acceptable coating to aid in processing. Examples of lubricants useful in coatings provided by the present disclosure include calcium stearate, glycerol behenate, glyceryl monostearate, magnesium stearate, mineral oil, polyethylene glycol, sodium stearyl fumarate, sodium lauryl sulfate, stearic acid, talc, vegetable oil, zinc stearate, and combinations of any of the foregoing. In certain embodiments, the lubricant is glyceryl monostearate. In certain embodiments, coatings may comprise an amount of lubricant ranging from about 1 wt% to about 15 wt% based on the total solids weight of the coating.

Plasticizers may be included in pharmaceutically acceptable coatings to improve the physical properties of the cured coating. For example, plasticizers may increase the flexibility or elasticity of a coating comprising a film-forming material having a relatively high glass transition temperature such as ethyl cellulose. Examples of plasticizers useful in coatings of the present disclosure include adipates, azelate, benzoates, citrates, isoebucates, phthalates, sebacates, stearates, glycols, polyethylene glycol, propylene glycol, triacetin, dimethyl phthalate, diethyl phthalate, dibutyl phthalate, dibutyl sebacate, triethyl citrate, tributyl citrate, triethyl acetyl citrate, acetyltributylcitrate, glyceryl triacetate, castor oil, acetylated monoglycerides, and combinations of any of the foregoing. In certain embodiments, coatings may comprise an amount of lubricant ranging from about 1 wt% to about 15 wt% based on the total solids weight of the coating.

Glidants may be included in pharmaceutically acceptable coatings to reduce sticking effects during processing, film formation, and/or drying. Examples of glidants useful in coatings provided by the present disclosure include talc, magnesium stearate, glycerol monostearate, colloidal silicon dioxide, precipitated silicon dioxide, and combinations of any of the foregoing.

Examples of pH-buffering agents useful in coatings provided by the present disclosure include citric acid, sodium citrate, fumaric acid, sodium fumarate, and the like.

pH modifying agents may create a microenvironment around released compound (**1**) or *R*-baclofen metabolite when exposed to aqueous fluids. For example, pH-modifying agents may drive the prodrug or the *R*-baclofen metabolite to its net neutral form, thereby enhancing its absorption through the intestinal epithelia.

Examples of stabilizers useful in coatings provided by the present disclosure include anti-oxidants such as 3,5-di-*tert*-butyl-4-hydroxytoluene (BHA), 3-(or 2)-*tert*-butyl-4-hydroxyanisole (BHT), ascorbic acids, tocopherols, and the like.

Binders may be included in coating compositions to hold the components of a coating together. Examples of binders useful in coatings provided by the present disclosure include, for example, polyvinylpyrrolidone, hydroxypropyl cellulose, hydroxypropyl methylcellulose, methylcellulose, hydroxyethyl cellulose, sugars, dextran, cornstarch, and combinations of any of the foregoing. In certain embodiments, the binder is polyvinylpyrrolidone such as Plasdone® K29/32 Plasdone (ISP Technologies, Wayne, NJ).

Anti-foaming agents may also be included in pharmaceutically acceptable coatings. Examples of anti-foaming agents useful in coatings provided by the present disclosure include silicone and simethicone.

Examples of pigments useful in coatings provided by the present disclosure include titanium dioxide, food color lakes, and iron oxides.

Controlled release coatings of the present disclosure may also comprise erosion-promoting agents such as starch and gums, materials useful for making microporous lamina in the use environment such as polycarbonates characterized by linear polyesters of carbonic acid in which carbonate groups reoccur in the polymer chain, and/or semi-permeable polymers such as hydroxypropylmethyl cellulose, lactose, and metal stearates.

Release of compound (**1**) from controlled release particles may further be influenced, for example, adjusted to a desired release rate, by providing one or more pores or passageways through one or more coatings. Release-modifying materials that may be incorporated into controlled release coatings and function as pore-formers may be organic or inorganic, include materials that can be dissolved, extracted, or leached from the coating in the environment of use, e.g., the gastrointestinal tract or in a certain region or regions of the gastrointestinal tract, such as hydrophilic materials, for example, hydroxypropylmethyl cellulose.

The combination of all solid components of the coatings forming controlled release coatings, including co-polymers, fillers, plasticizers, and optional excipients and processing aids, may provide a weight gain to the cores from about 10% to about 450%. In certain embodiments, the weight gain may be from about 30% to about 160%.

Controlled release particles comprising compound (**1**) and a release rate modifying coating may be incorporated into a number of oral dosage forms including capsules, tablets, and liquid suspensions. In certain embodiments, controlled release particles may be placed in a hard or soft gelatin capsule in an amount sufficient to provide a therapeutically effective concentration of *R*-baclofen in the blood of a patient when orally ingested. Capsules comprising controlled release particles are referred to herein as controlled release (CR) capsules. In certain embodiments, controlled release capsules comprise a therapeutically effective amount of compound (**1**) such as for example, from about 1 mg to about

100 mg of compound (**1**). In certain embodiments, controlled release capsules may comprise less than a therapeutically effective amount of compound (**1**), in which case multiple capsules may be administered simultaneously or over a period of time to provide a therapeutically effective amount of compound (**1**).

In certain embodiments, controlled release particles and optional vehicles may be compressed into a tablet using conventional tableting equipment and standard techniques. Techniques and compositions for making tablets (compressed and molded), capsules (hard and soft gelatin), and pills are known in the art. When controlled release particles are incorporated into tablets, the particles are compressed lightly so as not to break. Disintegrants may be included in tablets comprising controlled release particles to facilitate release of the particles from the tablet following ingestion.

Dosage forms comprising controlled release particles may comprise a suspension in which controlled release particles comprising compound (**1**) are dispersed in a pharmaceutically acceptable solvent formulation. Solvent formulations may include water, ethanol, flavorings, colorings, or combinations of any of the foregoing. Liquid oral dosage forms can include aqueous and non-aqueous solutions, emulsions, suspensions, and solutions and/or suspensions reconstituted from non-effervescent granules, containing suitable solvents, emulsifying agents, suspending agents, diluents, sweeteners, coloring agents, and flavoring agents, preservatives, and combinations of any of the foregoing. The solvent of an aqueous-based orally acceptable pharmaceutical carrier is entirely or predominantly water and can include a suspending agent. Examples of carriers include aqueous solutions, syrups, elixirs, dispersions, suspensions, emulsions such as oil-in-water emulsions, and microemulsions. Examples of suspending agents include microcrystalline cellulose/sodium carboxymethyl cellulose, guar gum, and the like. Cosolvents useful to solubilize and incorporate water-insoluble ingredients into a suspension include propylene glycol, glycerin, sorbitol solution, and the like. In addition, a liquid formulation may include vehicles such as wetting agents, emulsifying and suspension agents, sweetening, flavoring, coloring, perfuming, and preserving agents. Examples of useful suspension agents include ethoxylated isostearyl alcohols, polyoxyethylene sorbitol and sorbitan esters, microcrystalline cellulose, aluminum metahydroxide, bentonite, agar-agar, tragacanth, and combinations of any of the foregoing.

Dosage forms are in the form of tablets comprising compound (**1**). Tablet dosage forms may be of any shape suitable for oral administration of a drug such as spheroidal, cube-shaped oval, or ellipsoidal. In certain embodiments, tablet dosage forms, e.g., an oral dosage form in the form of a tablet, provided by the present disclosure are matrix systems in which the *R*-baclofen prodrug (**1**) is dispersed in a matrix comprising at least one release-rate modifying compound. Matrix systems are well-known in the art as described, for example, in "Handbook of Pharmaceutical Controlled Release Technology," ed. D.L. Wise, Marcel Dekker, Inc. (2000) and "Treatise on Controlled Drug Delivery, Fundamentals, Optimization, and Applications," ed. A. Kydonieus, Marcel Dekker, Inc. (1992).

Release rate modifying compounds can retard the release of compound (**1**) from a dosage form. Examples of release rate modifying compounds include, but are not limited to, pH dependent release polymers, pH independent release polymers, hydrophilic polymers that have a high degree of swelling when in contact with water or aqueous media, polymers that form a gel on contact with water or aqueous media, polymers that exhibit both swelling and gelling characteristics in contact with water or aqueous media, fatty compounds such as waxes, and biodegradable polymers.

Examples of pH dependent release rate modifying polymers useful in tablet dosage forms provided by the present disclosure include acrylic acid and methacrylic acid polymers and copolymers, methyl methacrylate copolymers, ethoxyethyl methacrylates, cyanoethyl methacrylate, poly(acrylic acid), poly(methacrylic acid), methacrylic acid alkylamide copolymer, poly(methyl methacrylate), polymethacrylate, poly(methyl methacrylate) copolymers, polyacrylamide, aminoalkyl methacrylate copolymer, poly(methacrylic acid anhydride), glycidyl methacrylate co-polymers, ammonioalkyl methacrylate copolymers, and combinations of any of the foregoing. In certain embodiments, a pH dependent polymer may be a copolymer synthesized from diethylaminoethyl methacrylate and other neutral methacrylic esters, also known as methacrylic acid copolymers or polymer methacrylates, commercially available as Eudragit® (Rohm Pharma).

Examples of pH independent release polymers useful in tablet dosage forms provided by the present disclosure include ammonioalkyl methacrylate copolymers such as Eudragit® RS and Eudragit® RL, which are acrylic resins comprising copolymers of acrylic and methacrylic acid esters with a low content of quaternary ammonium groups.

Examples of hydrophilic release rate modifying polymers that exhibit a high degree of swelling useful in tablet dosage forms provided by the present disclosure include cross linked sodium carboxymethyl cellulose, cross-linked hydroxypropyl cellulose, high-molecular weight hydroxypropylmethyl cellulose, carboxymethylamide, potassium methacrylatedivinylbenzene co-polymer, polymethylmethacrylate, polyvinylpyrrolidone, high-molecular weight polyvinylalcohols, methyl cellulose, vinyl acetate copolymers, and combinations of any of the foregoing.

Examples of release rate modifying polymers that gel in contact with water useful in tablet dosage forms provided by the present disclosure include methylcellulose, carboxymethyl cellulose, low-molecular weight hydroxypropylmethyl cellulose, low-molecular weight polyvinylalcohols, polyoxyethyleneglycols, non-cross linked polyvinylpyrrolidone, xanthan gum, and combinations of any of the foregoing.

Examples of release rate modifying polymers that exhibit both swelling and gelling properties useful in tablet dosage forms provided by the present disclosure include medium-viscosity hydroxypropylmethyl cellulose and medium-viscosity polyvinylalcohols.

In certain embodiments, release rate modifying compounds useful in tablet dosage forms provided by the present disclosure may be chosen from glyceryl esters such as glyceryl monostearate, glyceryl behenate, glyceryl palmitostearate, lauroyl macrogol glyceride, stearoyl macrogol glyceride, and combinations of any of the foregoing. Other fatty and/or waxy release rate modifying compounds include lauryl alcohol, myristyl alcohol, stearyl alcohol, cetyl alcohol, cetostearyl alcohol, palmitoyl alcohol, ouricury wax, hydrogenated vegetable oil, candelilla wax, esparto wax, stearic acid, paraffin wax, beeswax, glycowax, castor wax, carnauba wax, and combinations of any of the foregoing.

Examples of bioerodible polymers include collagen, gelatin, polyvinyl alcohols, polyorthoesters, polyacetyls, polyorthocarbonates, polyamides, polyaminoacids, polyesters, polylactic acids, polyglycolic acids, polycarbohydrates, polyorthoesters, polyorthocarbonates, polyacetyls, polyanhydrides, polydehydropyrans, polydioxinones, and combinations of any of the foregoing.

Other useful release-rate modifying compounds that may be incorporated into tablet dosage forms provided by the present disclosure include hydrocolloids such as natural or synthetic gums, carbohydrate-based substances such as acacia, gum tragacanth, locust bean gum, guar gum, agar, pectin, carageenin, soluble and insoluble alginates, carboxypolymethylene, casein, zein, polyethylene oxide, maleic anhydride/methyl vinyl ether copolymers, and proteinaceous substances such as gelatin.

Release rate modifying polymers or compounds may be used alone or in combination with one or more other release rate modifying polymers or compounds and/or can be a copolymer of more than one release rate modifying polymer.

Tablet dosage forms comprise as a release rate modifying polymer an ammonioalkyl methacrylate copolymer. The release rate modifying polymer is an ammonioalkyl methacrylate copolymer such as Eudragit RL® 30D (Type A) (Degussa), which is characterized by a molecular weight from about 125,000 Daltons to about 175,000 Daltons. Eudragit® RL 30D is an aqueous dispersion of Eudragit® RL 100, which is characterized by from about 10.18% to about 13.73% ammoniomethacrylate units on a dry substrate. Eudragit® RL 100 is a copolymer of acrylic and methacrylic acid esters with low quaternary ammonium groups and having an average molecular weight of about 150,000 Daltons.

Tablet dosage forms, e.g., an oral dosage form in the form of a tablet, provided by the present disclosure further comprise a second release rate modifying polymer. The second release rate modifying polymer is hydroxypropylmethyl cellulose. In certain embodiments, the hydroxypropylmethyl cellulose is chosen from hydroxypropylmethyl cellulose 2910 and hydroxypropylmethyl cellulose 2208. Hydroxypropylmethyl cellulose 2910 is commercially available as Methocel® E4M (Dow Chemical Co.) and is characterized by a methoxyl content from about 28 wt% to about 30 wt%, a hydroxypropyl content from about 7 wt% to about 12 wt%, and a viscosity in 2% aqueous solution at 37 °C from about 3,000 mPa-sec to about 5,600 mPa-sec. Hydroxypropylmethyl cellulose 2208 is commercially available as Methocel® K4M and is characterized by a methoxyl content from about 19 wt% to about 24 wt%, a hydroxypropyl content from about 7 wt% to about 12 wt%, and a viscosity in 2% aqueous solution at 37 °C from about 2,308 mPa-sec to about 3,757 mPa-sec.

Tablet dosage forms provided by the present disclosure comprise hydroxypropylmethyl cellulose and an ammonioalkyl methacrylate copolymer. In certain embodiments, tablet dosage forms provided by the present disclosure comprise from about 3 wt% to about 5 wt% of (3*R*)-4-{[(1*S*)-2-methyl-1-(2-methylpropanoyloxy)propoxy]carbonylamino}-3-(4-chlorophenyl)butanoic acid, about 12 wt% to about 22 wt% of ammonioalkyl methacrylate copolymer, and from about 30 wt% to about 40 wt% of hydroxypropylmethyl cellulose, and in certain embodiments, from about 3.5 wt% to about 4.5 wt% of (3*R*)-4-{[(1*S*)-2-methyl-1-(2-methylpropanoyloxy)propoxy]carbonylamino}-3-(4-chlorophenyl)butanoic acid, from about 14.5 wt% to about 21.5 wt% of ammonioalkyl methacrylate copolymer, and from about 32.5 wt% to about 37.5 wt% of hydroxypropylmethyl cellulose.

Tablet dosage forms comprising compound (**1**) and one or more release rate modifying compounds may be prepared using standard techniques well known in the art such as wet granulation, fluid-bed granulation, dry granulation, or direct compression. In wet granulation, specified quantities of drug and one or more vehicles are mixed using a mechanical powder blender or mixer until uniform. A liquid binder is added to the powder mixture to facilitate adhesion of the powder particles. The wet mass is pressed through a sieve to provide granules, which are then dried. The dried granules are passed through a screened to reduce the particle size. A dry lubricant is added to the dried granulate and the resulting blend compressed into tablets. In fluid-bed granulation, particles comprising a drug are suspended in an air stream. A solution comprising a granulating material is sprayed into the air stream to coat the particles. After drying and the addition of vehicles, the granulated material is compressed into tablets. In dry granulation, the drug and vehicles such as binder, diluent, and/or lubricant is blended and compressed by roller compaction or slugging. The compressed material is sieved through a mesh screen to provide a granulate. Additional vehicles may be blended with the granulate and the blend compressed into tablets. Tablets may also be formed by direct compression of compounds having sufficient coadhesive properties.

Matrix systems in which compound (**1**) is dispersed in a matrix comprising at least one release rate modifying compound may be prepared by dry blending a release-modifying polymer, filler, compound (**1**), and vehicles followed

by granulating the mixture using an alcohol until proper granulation is obtained. Granulation may be accomplished by methods known in the art. The wet granules may be dried in a fluid bed dryer, sifted, and ground to an appropriate size. Lubricating agents may be mixed with the dried granulation to obtain a final formulation. In certain embodiments, such formulations may be compressed into tablet dosage forms by methods well known in the art.

In certain embodiments, the amount of compound (**1**) in a dosage form provided by the present disclosure ranges from about 0.1 mg to about 200 mg, in certain embodiments, from about 1 mg to about 100 mg, from about 5 mg to about 80 mg, and in certain embodiments, from about 5 mg to about 50 mg. For dosage forms comprising a pharmaceutically acceptable salt and/or solvate of compound (**1**), the amount of compound (**1**) in a dosage form refers to the mass equivalent weight of compound (**1**) comprising the salt and/or hydrate. In certain embodiments, tablet dosage forms may comprise a therapeutically effective amount of compound (**1**). A therapeutically effective amount of compound (**1**) can comprise from about 1 mg-equivalents to about 100 mg-equivalents *R*-baclofen, from about 2 mg-equivalents to about 80 mg-equivalents *R*-baclofen, from about 2 mg-equivalents to about 40 mg equivalent *R*-baclofen, or from about 5 mg-equivalent *R*-baclofen to about 20 mg-equivalent *R*-baclofen. One (1) mg compound (**1**) comprises 0.535 mg-equivalents of *R*-baclofen. In certain embodiments, the amount of compoound (**1**) in a dosage form provided by the present disclosure is less than an amount that causes moderate sedation and impairment of motor activity in a patient. In certain embodiments, a therapeutically effective amount of compound (**1**) is less than an amount that causes moderate sedation and impairment of motor activity in a patient.

In certain embodiments in which tablet dosage forms comprise less than a therapeutically effective amount of compound (**1**), multiple tablet dosage forms may be administered to a patient simultaneously, or over a period of time to provide a therapeutically effective amount of compound (**1**).

In addition to compound (**1**) and the release rate modifying compounds disclosed herein, tablet dosage forms may also comprise one or more pharmaceutically acceptable vehicles such as surfactants, lubricants, plasticizers, binding agents, diluents, anti-adherents, glidants, buffers, dyes, wetting agents, emulsifying agents, pH buffering agents, stabilizing agents, thickening agents, disintegrants, and coloring agents.

Diluents, or fillers, may be added to increase the bulk to make dosage forms a practical size for compression. Examples of diluents useful in tablet dosage forms provided by the present disclosure include dibasic calcium phosphate, dibasic calcium phosphate dihydrate, calcium sulfate, dicalcium phosphate, tricalcium phosphate, lactose, cellulose including microcrystalline cellulose, kaolin, mannitol, sodium chloride, dry starch, pregelatinized starch, compressible sugar, mannitol, and combinations of any of the foregoing. In certain embodiments, a diluent is selected from dibasic calcium phosphate and microcrystalline cellulose. Fillers may be water insoluble, water soluble, or combinations thereof. Examples of useful water insoluble fillers include silicon dioxide, titanium dioxide, talc, alumina, starch, kaolin, polacrilin potassium, powdered cellulose, microcrystalline cellulose, fumed silica, glyceryl monostearate, magnesium stearate, calcium stearate, colloidal silica, micronized silica, magnesium trisilicate, gypsum, and combinations of any of the foregoing. Examples of water-soluble fillers include water soluble sugars and sugar alcohols, such as lactose, glucose, fructose, sucrose, mannose, dextrose, galactose, the corresponding sugar alcohols and other sugar alcohols, such as mannitol, sorbitol, xylitol, and combinations of any of the foregoing. In certain embodiments wherein the diluent is microcrystalline cellulose, a tablet dosage form may comprise an amount of diluent ranging from about 25 wt% to about 60 wt%, and in certain embodiments, from about 30 wt% to about 55 wt%.

Glidants may be included in dosage forms provided by the present disclosure to reduce sticking effects during processing, film formation, and/or drying. Examples of useful glidants include talc, magnesium stearate, glycerol monostearate, colloidal silicon dioxide, precipitated silicon dioxide, fumed silicon dioxide, and combinations of any of the foregoing. In certain embodiments, a glidant is colloidal silicon dioxide. Tablet dosage forms may comprise less than about 2 wt% of a glidant, and in certain embodiments, less than about 1 wt% of a glidant.

Binding agents may be included in dosage forms to facilitate adhesion of the constituents. Examples of binding agents useful in tablet dosage forms provided by the present disclosure include polyvinyl acetate phthalate, molasses, methylcellulose, carboxymethyl cellulose, microcrystalline cellulose, and polyvinyl pyrrolidone. In certain embodiments provided by the present disclosure, a binder is microcrystalline cellulose such as Avicel® PH200 (FMC Corporation).

Plasticizers may be included in tablet dosage forms provided by the present disclosure. Examples of plasticizers useful in tablet dosage forms provided by the present disclosure include alkyl citrates such as triethyl citrate, acetyl triethyl citrate, tributyl citrate, acetyl triethyl citrate, and acetyl tributyl citrate; acetates such as triethyl acetate; sucrose fatty acid esters; glycerin mono-, di- and tri-fatty acid esters such as triacetin, glycerin mono-fatty acid esters, glycerin monostearate and acetylated monoglyceride; polyglycerin fatty acid esters; polyethylene glycols such as macrogol 400, macrogol 600, macrogol 1500, macrogol 4000 and macrogol 6000; dibutyl sebacate; tributyl sebacate; vinyl pyrrolidone; propylene glycol; sesame oil; castor oil; glycerin; silicone resins; D-sorbitol; phytosterol; alkyl phthalates such as diethyl phthalate, dibutyl phthalate and dioctyl phthalate; adipate polyesters; isopropyl myristate; medium chain triglyceride; butyl phthalyl butyl glycolate; polyoxyethylene polyoxypropylene glycol; and combinations of any of the foregoing. Tablet dosage forms may comprise an amount of plasticizer ranging from about 0.1 wt% to about 10 wt%, from about 1 wt% to about 8 wt %, and in certain embodiments, from about 2 wt% to about 6 wt%. In certain embodiments of dosage forms

provided by the present disclosure, the dosage form comprises from about 2 wt% to about 6 wt% of a plasticizer chosen from triethyl citrate and triethyl acetate.

Lubricants and anti-adherents may be included in tablet dosage forms provided by the present disclosure to aid in processing. Examples of lubricants and/or anti-adherents useful in tablet dosage forms provided by the present disclosure include calcium stearate, glyceryl behenate, glyceryl monostearate, magnesium stearate, mineral oil, polyethylene glycol, sodium stearyl fumarate, sodium lauryl sulfate, sodium dodecyl sulfate, stearic acid, talc, hydrogenated vegetable oil, zinc stearate, and combinations of any of the foregoing. In certain embodiments, a lubricant is glyceryl monostearate. In certain embodiments, a lubricant is magnesium stearate. Tablet dosage forms may comprise an amount of lubricant and/or anti-adherent ranging from about 0.1 wt% to about 5 wt%, and in certain embodiments, from about 0.1 wt% to about 1 wt%.

Examples of surfactants useful in tablet dosage forms provided by the present disclosure include pharmaceutically acceptable anionic surfactants, cationic surfactants, amphoteric (amphiphatic/amphiphilic) surfactants, non-ionic surfactants, polyethyleneglycol esters or ethers, and combinations of any of the foregoing. Examples of useful pharmaceutically acceptable anionic surfactants include monovalent alkyl carboxylates, acyl lactylates, alkyl ether carboxylates, *N*-acyl sarcosinates, polyvalent alkyl carbonates, *N*-acyl glutamates, fatty acid-polypeptide condensates, sulfuric acid esters, alkyl sulfates such as sodium lauryl sulfate and sodium dodecyl sulfate, ethoxylated alkyl sulfates, ester linked sulfonates such as docusate sodium and dioctyl sodium succinate, alpha olefin sulfonates, or phosphated ethoxylated alcohols. Examples of useful pharmaceutically acceptable cationic surfactants include monoalkyl quaternary ammonium salts, dialkyl quaternary ammonium compounds, amidoamines, and aminimides. Examples of useful pharmaceutically acceptable amphoteric surfactants include, *N*-substituted alkyl amides, *N*-alkyl betaines, sulfobetaines, and *N*-alkyl-6-aminopropionates. Examples of useful pharmaceutically acceptable polyethyleneglycol esters or ethers include polyethoxylated castor oil, polyethoxylated hydrogenated castor oil, and hydrogenated castor oil. In certain embodiments, a surfactant is chosen from sodium lauryl sulfate and sodium dodecyl sulfate. In certain embodiments, tablet dosage forms may comprise less than about 3 wt% of a surfactant, and in certain embodiments, less than about 2 wt% of a surfactant.

Disintegrants may be included in a tablet formulation to cause a tablet to break apart, for example, by expansion of a disintegrants when exposed to water. Examples of useful disintegrants include water swellable substances such as low-substituted hydroxypropyl cellulose, cross-linked sodium carboxymethylcellulose (sodium croscarmellose), sodium starch glycolate, sodium carboxymethylcellulose, sodium carboxymethyl starch, ion-exchange resins, microcrystalline cellulose, cross-linked polyvinyl pyrrolidone, starches and pregelatinized starch, formalin-casein, alginic acid, certain complex silicates, and combinations of any of the foregoing.

Tablet dosage forms provided by the present disclosure may further comprise one or more coatings, which may partially or fully cover the tablets. While certain coatings may be applied to modify or affect the release of compound (*1*) from a tablet dosage form in the gastrointestinal tract, others may have no such effect. For example, one or more additional coatings may be for physical protection, aesthetics, ease in swallowing, identification, and/or to facilitate further processing of the tablets. Coatings may be impermeable to moisture or moisture permeable. Moisture permeable exterior tablet coatings may be useful for maintaining low moisture content in a dosage form that is packaged in the presence of a desiccant and may thereby enhance, for example, the storage stability of a tablet dosage form. Examples of materials useful in coatings for physical protection include permeable or soluble materials such as hydroxypropyl methylcellulose, hydroxypropyl cellulose, hydroxypropyl ethylcellulose, and xanthan gum. Examples of materials useful in external tablet coatings to facilitate further processing include talc, colloidal silica, polyvinyl alcohol, titanium dioxide, micronized silica, fumed silica, glycerol monostearate, magnesium trisilicate, and magnesium stearate. An external tablet coating may further include one or more vehicles such as plasticizers, binders, fillers, lubricants, compression aides, and combinations of any of the foregoing. The one or more additional coatings may comprise a single material or a combination of more than one material including any of those disclosed herein. These additional coatings may be applied to tablet dosage forms by methods known to those skilled in the art.

In certain embodiments, dosage forms provided by the present disclosure are substantially free of lactam side products formed by intramolecular cyclization of compound (1) and/or *R*-baclofen. Dosage forms may be stable to extended storage, such as for example, greater than one year, without substantial lactam formation such as less than 0.5% lactam by weight, less than 0.2% lactam by weight, or less than 0.1% lactam by weight.

**Release Characteristics of Dosage Forms**

Sustained release dosage forms comprising compound (**1**) exhibit enhanced oral bioavailability as *R*-baclofen compared to the oral bioavailability of *R*-baclofen when administered in an equivalent dosage form of *R*-baclofen and/or racemate. The enhanced oral bioavailability of compound (**1**) is believed to be due the efficient absorption of compound (**1**) throughout the gastrointestinal tract, including the colon, via passive and/or active transport mechanisms. Dosage forms provided by the present disclosure provide for the release of compound (**1**) from the dosage form during passage

of the dosage form through the gastrointestinal tract.

Following oral administration to a patient, sustained release dosage forms comprising compound (**1**) provide *R*-baclofen in the systemic circulation of a patient. Compound (**1**) may be absorbed from the gastrointestinal tract and enter the systemic circulation where the promoiety is cleaved to release *R*-baclofen. The promoiety of compound (**1**) may be cleaved either chemically and/or enzymatically. For example, one or more enzymes, such as esterases, present in the stomach, intestinal lumen, intestinal tissue, blood, liver, brain, or any other suitable tissue of a mammal can enzymatically cleave the promoiety of compound (**1**).

When administered orally to a patient, i.e., by a patient swallowing a dosage form provided by the present disclosure, dosage forms provide a sustained therapeutically effective concentration of *R*-baclofen in the blood of the patient during a continuous period of time. In certain embodiments, dosage forms may provide a concentration of *R*-baclofen in the blood of a patient that is greater than a minimum therapeutically effective concentration and less than a minimum adverse concentration of *R*-baclofen in the blood of the patient. In certain embodiments, dosage forms provided by the present disclosure provide a therapeutically effective concentration *R*-baclofen in the blood of a patient for a continuous period of time without exceeding the minimum adverse concentration of *R*-baclofen. In certain embodiments, the concentration of *R*-baclofen in the blood of a patient does not exceed a minimum adverse concentration at any time after the dosage form is orally administered to the patient. Dosage forms provided by the present disclosure can provide a therapeutically effective concentration of *R*-baclofen in the blood of a patient for a continuous period of time while reducing or eliminating adverse drug effects associated with high blood concentrations of *R*-baclofen, e.g., at concentrations above the minimum adverse concentration, observed following oral dosing of forms comprising *R*-baclofen. The high bioavailability of *R*-baclofen achievable using dosage forms comprising compound (**1**) may facilitate the use of lower mass equivalents of *R*-baclofen in a dose to achieve a sustained therapeutically effective concentration of *R*-baclofen in the blood of a patient compared to the amount of *R*-baclofen in an oral dosage form comprising *R*-baclofen.

Sustained release dosage forms provided by the present disclosure are capable of providing a sustained therapeutically effective concentration of *R*-baclofen in the blood of a patient following oral administration. For example, dosage forms may provide a sustained therapeutically effective concentration of *R*-baclofen in the blood of a patient during a continuous time period selected from at least about 4 hours, at least about 8 hours, at least about 12 hours, at least about 16 hours, at least about 20 hours, or at least about 24 hours, after oral administration to a patient. In certain embodiments, the concentration of *R*-baclofen in the blood of a patient will not exceed a minimum adverse concentration at any time after the dosage form is orally administered to the patient, e.g., will not reach a concentration that causes adverse events in the patient. A therapeutically effective concentration of *R*-baclofen in the blood of a patient may range from about 50 ng/mL to about 1,000 ng/mL, and in certain embodiments, from about 100 ng/mL to about 500 ng/mL. The pharmacokinetic profile of the blood *R*-baclofen concentration can be characterized by a lower $C_{max}/C_{12}$ ratio, and a lower $C_{max}$/dose, compared to immediate release and sustained release oral formulations comprising *R*-baclofen that provide a similar *R*-baclofen blood $AUC_{inf}$.

In certain embodiments, following oral administration to a human patient, particle dosage forms comprising (3*R*)-4-{[(1*S*)-2-methyl-1-(2-methylpropanoyloxy)propoxy]carbonylamino}-3-(4-chlorophenyl)butanoic acid and at least one pH independent release polymer such as an ammonioalkyl methacrylate copolymer, provide a blood (*R*)-3-amino-3-(4-chlorophenyl)butanoic acid concentration characterized by: a $C_{max}$ / $C_{12}$ ratio ranging from about 1 to about 6; a $C_{max}$ / dose ratio ranging from about 1.25 $(10^6{\cdot}mL)^{-1}$ to about 3.25 $(10^6{\cdot}mL)^{-1}$; and an $AUC_{inf}$ / dose ratio ranging from about 13 (hr/$10^6$·mL) to about 33 (hr/$10^6$·mL). In certain embodiments, following oral administration to a human patient, particle dosage forms comprising (3*R*)-4-{[(1*S*)-2-methyl-1-(2-methylpropanoyloxy)propoxy]carbonylamino}-3-(4-chlorophenyl) butanoic acid and at least one pH independent release polymer such as an ammonioalkyl methacrylate copolymer, provide a blood (*R*)-3-amino-3-(4-chlorophenyl)butanoic acid concentration characterized by: a $C_{max}$ / $C_{12}$ ratio ranging from about 2.3 to about 4.3; a $C_{max}$ / dose ratio ranging from about 1.75 $(10^6{\cdot}mL)^{-1}$ to about 2.75$(10^6{\cdot}mL)^{-1}$; and an $AUC_{inf}$/ dose ratio ranging from about 18 (hr/$10^6$·mL) to about 28 (hr/$10^6$·mL). In certain embodiments, following oral administration to a human patient, particle dosage forms comprising (3*R*)-4-{[(1*S*)-2-methyl-1-(2-methylpropanoyloxy) propoxy]carbonylamino}-3-(4-chlorophenyl)butanoic acid and at least one pH independent release polymer such as an ammonioalkyl methacrylate copolymer, provide a blood (*R*)-3-amino-3-(4-chlorophenyl)butanoic acid concentration characterized by: a $C_{max}$ / $C_{12}$ ratio ranging from about 2.8 to about 3.8; a $C_{max}$ / dose ratio ranging from about 2.0 $(10^6{\cdot}mL)^{-1}$ to about 2.5 $(10^6{\cdot}mL)^{-1}$; and an $AUC_{inf}$ / dose ratio ranging from about 18 (hr/$10^6$·mL) to about 28 (hr/$10^6$·mL). In certain of the above embodiments, tablet dosage forms comprising an ammonioalkyl methacrylate copolymer and hydroxypropylmethyl cellulose provide an oral bioavailability of *R*-baclofen ranging from about 80% to about 100% following oral administration to dogs, where the oral bioavailabiity is determined relative to an intravenous dose of 10 mg *R*-baclofen ($AUC_{inf}$= 1.98 μg·hr/mL). In certain of the above embodiments, the tablet dosage forms administered comprise an amount of compound (**1**) ranging from about 1 mg to about 100 mg, and in certain of the above embodiments, the tablet dosage forms comprise about 10 mg of compound (**1**).

In certain embodiments, following oral administration to dogs, tablet dosage forms comprising compound (**1**) dispersed in a polymer matrix comprising an ammonioalkyl methacrylate copolymer, and hydroxypropylmethyl cellulose

provide a pharmacokinetic profile for *R*-baclofen in the blood characterized by a $C_{max}/C_{12}$ ratio ranging from about 1 to about 6; a $C_{max}$/dose ratio ranging from about 1.25 $(10^6{\cdot}mL)^{-1}$ to about 6.4 $(10^6{\cdot}mL)^{-1}$; and an $AUC_{inf}$/dose ratio ranging from about 13 (hr/$10^6$·mL) to about 33 (hr/$10^6$·mL). In certain of the above embodiments, tablet dosage forms provide an oral bioavailability of *R*-baclofen ranging from about 24% to 72%, and in certain embodiments, from about 36% to about 60% following oral administration to dogs, where the oral bioavailabiity is determined relative to an intravenous dose of 10 mg *R*-baclofen ($AUC_{inf}$= 1.98 μg·hr/mL). In certain of the above embodiments, the tablet dosage forms administered comprise an amount of compound (**1**) ranging from about 1 mg to about 100 mg, and in certain of the above embodiments, the tablet dosage forms comprise about 10 mg of compound (**1**).

In certain embodiments, oral administration of at least one oral dosage form comprising (3*R*)-4-{[(1*S*)-2-methyl-1-(2-methylpropanoyloxy)propoxy]carbonylamino}-3-(4-chlorophenyl)butanoic acid, ammonioalkyl methacrylate copolymer, and hydroxypropylmethyl cellulose to a fasted human patient provides a relative oral bioavailability of (*R*)-3-amino-3-(4-chlorophenyl)butanoic acid ranging from about 100% to about 200%, wherein the oral bioavailability is relative to that following oral administration of an equivalent amount of (*R*)-3-amino-3-(4-chlorophenyl)butanoic acid in at least one immediate release dosage form.

In certain embodiments, oral administration of at least one oral dosage form comprising (3*R*)-4-{[(1*S*)-2-methyl-1-(2-methylpropanoyloxy)propoxy]carbonylamino}-3-(4-chlorophenyl)butanoic acid, ammonioalkyl methacrylate copolymer, and hydroxypropylmethyl cellulose to a fasted human patient provides a pharmacokinetic profile of (*R*)-3-amino-3-(4-chlorophenyl)butanoic acid in the blood of a fasted human patient characterized by: a $C_{max}$ / dose ratio ranging from about 1.0$(10^6{\cdot}mL)^{-1}$ to about 2.2 $(10^6{\cdot}mL)^{-1}$; a $C_{max}$ / $C_{12}$ ratio ranging from about 1.3 to about 2.9; and an $AUC_{0\text{-}inf}$/dose ratio ranging from about 21 h/$10^6$·mL to about 34 h/$10^6$·mL.

In certain embodiments, oral administration of at least one oral dosage form comprising (3*R*)-4-{[(1*S*)-2-methyl-1-(2-methylpropanoyloxy)propoxy]carbonylamino}-3-(4-chlorophenyl)butanoic acid, ammonioalkyl methacrylate copolymer, and hydroxypropylmethyl cellulose to a fed human patient provides a relative oral bioavailability of (*R*)-3-amino-3-(4-chlorophenyl)butanoic acid ranging from about 170% to about 370%, wherein the oral bioavailability is relative to that following oral administration of an equivalent amount of (*R*)-3-amino-3-(4-chlorophenyl)butanoic acid in at least one immediate release dosage form.

In certain embodiments, oral administration of at least one oral dosage form comprising (3*R*)-4-{[(1*S*)-2-methyl-1-(2-methylpropanoyloxy)propoxy]carbonylamino}-3-(4-chlorophenyl)butanoic acid, ammonioalkyl methacrylate copolymer, and hydroxypropylmethyl cellulose to a fed human patient provides a pharmacokinetic profile of (*R*)-3-amino-3-(4-chlorophenyl)butanoic acid in the blood of a fed human patient characterized by: a $C_{max}$ / dose ratio ranging from about 1.9 $(10^6{\cdot}mL)^{-1}$ to about 4.2 $(10^6{\cdot}mL)^{-1}$; a $C_{max}$ / $C_{12}$ ratio ranging from about 0.9 to about 2.3; and an $AUC_{0\text{-}inf}$/dose ratio ranging from about 29 h/$10^6$·mL to about 58 h/$10^6$·mL.

In certain embodiments, once daily oral administration of at least one oral dosage form comprising (3*R*)-4-{[(1*S*)-2-methyl-1-(2-methylpropanoyloxy)propoxy]carbonylamino}-3-(4-chlorophenyl)butanoic acid, ammonioalkyl methacrylate copolymer, and hydroxypropylmethyl cellulose to a patient provides a steady state pharmacokinetic profile of (*R*)-3-amino-3-(4-chlorophenyl)butanoic acid in the blood of the human patient characterized by: a $C_{SS,\ max}$ / dose ratio ranging from about 1.4 μg/mL to about 3.0 μg/mL; a $C_{ss,}$min / dose ratio ranging from about 0.26 $(10^6{\cdot}mL)^{-1}$ to about 0.70 $(10^6{\cdot}mL)^{-1}$; a $C_{ss,\ max}$ / $C_{SS,\ min}$ ratio ranging from about 1.1 to about 9.1; and an $AUC_{0\text{-}i24}$/dose ratio ranging from about 16 h/$10^6$·mL to about 35 h/$10^6$·mL.

In certain embodiments, once daily oral administration of at least one oral dosage form comprising (3*R*)-4-{[(1*S*)-2-methyl-1-(2-methylpropanoyloxy)propoxy]carbonylamino}-3-(4-chlorophenyl)butanoic acid, ammonioalkyl methacrylate copolymer, and hydroxypropylmethyl cellulose to a patient provides a steady state pharmacokinetic profile of (*R*)-3-amino-3-(4-chlorophenyl)butanoic acid in the blood of the human patient characterized by: the $C_{SS,\ max}$ / dose ratio ranges from about 1.8 $(10^6{\cdot}mL)^{-1}$ to about 2.6 $(10^6{\cdot}mL)^{-1}$; the $C_{SS,min}$ / dose ratio ranges from about 0.37 $(10^6{\cdot}mL)^{-1}$ to about 0.59 $(10^6{\cdot}mL)^{-1}$; the $C_{ss,\ max}$ / $C_{SS,\ min}$ ratio ranges from about 3.1 to about 7.1; and the $AUC_{0\text{-}i24}$ / dose ratio ranges from about 21 h/$10^6$·mL to about 30 h/$10^6$·mL.

In certain embodiments, twice daily oral administration of at least one oral dosage form comprising (3*R*)-4-{[(1*S*)-2-methyl-1-(2-methylpropanoyloxy)propoxy]carbonylamino}-3-(4-chlorophenyl)butanoic acid, ammonioalkyl methacrylate copolymer, and hydroxypropylmethyl cellulose to a patient provides a steady state pharmacokinetic profile of (*R*)-3-amino-3-(4-chlorophenyl)butanoic acid in the blood of the human patient characterized by: a $C_{SS,\ max}$ / dose ratio ranging from about 2.2 $(10^6{\cdot}mL)^{-1}$ to about 5.2 $(10^6{\cdot}mL)^{-1}$; a $C_{SS,min}$ / dose ratio ranging from about 1.2 $(10^6{\cdot}mL)^{-1}$ to about 2.2 $(10^6{\cdot}mL)^{-1}$; a $C_{ss,\ max}$ / $C_{SS,\ min}$ ratio ranging from about 1.1 to about 3.5; and an $AUC_{0\text{-}i24}$ / dose ratio ranging from about 42 h/$10^6$·mL to about 76 h/$10^6$·mL.

In certain embodiments, twice daily oral administration of at least one oral dosage form comprising (3*R*)-4-{[(1*S*)-2-methyl-1-(2-methylpropanoyloxy)propoxy]carbonylamino}-3-(4-chlorophenyl)butanoic acid, ammonioalkyl methacrylate copolymer, and hydroxypropylmethyl cellulose to a patient provides a steady state pharmacokinetic profile of (*R*)-3-amino-3-(4-chlorophenyl)butanoic acid in the blood of the human patient characterized by: the $C_{SS,\ max}$ / dose ratio ranges from about 3.0 $(10^6{\cdot}mL)^{-1}$ to about 4.4 $(10^6{\cdot}mL)^{-1}$; the $C_{SS,min}$ / dose ratio ranges from about 1.4 $(10^6{\cdot}mL)^{-1}$

to about 2.0 $(10^6 \cdot mL)^{-1}$; the $C_{ss, max}$ / $C_{SS, min}$ ratio ranges from about 1.7 to about 2.9; and the $AUC_{0-i24}$ / dose ratio ranges from about 51 h/$10^6 \cdot$mL to about 67 h/$10^6 \cdot$mL.

In certain of the preceding embodiments, the at least one oral dosage form is administered to a human patient at a dose of (3*R*)-4-{[(1*S*)-2-methyl-1-(2-methylpropanoyloxy)propoxy]carbonylamino}-3-(4-chlorophenyl)butanoic acid ranging from about 5 mg to about 140 mg, and in certain of the preceding embodiments, from about 10 mg to about 120 mg. In certain of the preceding embodiments, the dose of (3*R*)-4-{[(1*S*)-2-methyl-1-(2-methylpropanoyloxy)propoxy]carbonylamino}-3-(4-chlorophenyl)butanoic acid administered is less than a dose of (3*R*)-4-{[(1*S*)-2-methyl-1-(2-methylpropanoyloxy)propoxy]carbonylamino}-3-(4-chlorophenyl)butanoic acid that causes moderate sedation and impairment of motor activity in a patient.

A dosage regimen employing oral administration of dosage forms provided by the present disclosure may be developed to maintain a concentration of *R*-baclofen in the blood of a patient, which is greater than a minimum therapeutically effective concentration and less than a minimum adverse concentration for a prolonged period of time. In certain embodiments, a minimum therapeutically effective concentration of *R*-baclofen may range from about 1 ng/mL to about 200 ng/mL, and in certain embodiments, can range from about 10 ng/mL to about 100 ng/mL. In certain embodiments, a minimum adverse concentration can range from about 200 ng/mL to about 2,000 ng/mL, and in certain embodiments, can range from about 500 ng/mL to about 1,000 ng/mL. A minimum therapeutic concentration and a minimum adverse concentration will depend on a number of factors such as the disease being treated, the severity of the disease, the intended clinical outcome, the condition of the patient being treated, and so forth. Such regimens may employ repeated dosing of one or more dosage forms provided by the present disclosure. An appropriate interval of dosing may depend, for example, on the amount of compound (**1**) in the dosage form, the composition of the dosage form, the release characteristics of compound (**1**) from the dosage form, the disease being treated, the condition of the patient, the potential adverse effects, and the judgment of the prescribing physician. Dosage regimens may include repeated administration of the same dosage form at each interval or different dosage forms at different intervals. For example, a twice-daily dosage regimen can include the administration of a first dosage form in the morning, and a second dosage form in the evening.

Dosage forms provided by the present disclosure further include dosage forms that are bioequivalent to the dosage forms disclosed herein, in terms of both rate and extent of absorption, for example as defined by the U.S. Food and Drug Administration and discussed in "Guidance for Industry - Bioavailability and Bioequivalence Studies for Orally Administered Drug Products" (2003).

**Dissolution Profiles**

Dosage forms provided by the present disclosure comprising compound (**1**) may be characterized, in part, by the *in vitro* dissolution profile. Methods for determining dissolution profiles of dosage forms are well known to those skilled in the pharmaceutical arts. Standard methodologies set forth in the U.S. Pharmacopeia may be used. For example, a dissolution profile may be measured in either U.S. Pharmacopeia Type I Apparatus (baskets) or a U.S. Pharmacopeia Type II Apparatus (paddles).

Using the latter method, dissolution, or release, profiles of dosage forms provided by the present disclosure may be determined by immersing dosage forms in a 10 mM monobasic potassium phosphate buffer ($KH_2PO_4$) at pH 7.4, and a temperature of 37 °C. The dissolution medium is agitated at 75 rpm (USP, Type II). Samples are withdrawn from the dissolution medium at intervals and the content of compound (**1**) and or *R*-baclofen in the dissolution medium determined using reverse phase HPLC.

In certain embodiments, release of compound (**1**) from controlled release particles exhibits an *in vitro* dissolution profile in 10 mM monobasic potassium phosphate buffer at pH 7.4 and 37°C agitated at 75 rpm (USP, Type II) in which from about 35% to about 45% of compound (**1**) is released within about 4 hours; from about 60% to about 80% of compound (**1**) is released within about 7.6 hours; and from about 85% to about 95% of compound (**1**) is released within about 13 hours. In certain embodiments, release of compound (**1**) from controlled release particles exhibits an *in vitro* dissolution profile in 10 mM monobasic potassium phosphate buffer at pH 7.4 and 37 °C agitated at 75 rpm (USP, Type II) in which from about 20% of compound (**1**) is released within about 4 hours; from about 70% of compound (**1**) is released within about 7.6 hours; and from about 90% of compound (**1**) is released within about 13 hours.

In certain embodiments, release of compound (**1**) from tablet dosage forms comprising an ammonioalkyl methacrylate copolymer and hydroxypropylmethyl cellulose exhibits an *in vitro* dissolution profile in 10 mM monobasic potassium phosphate buffer at pH 7.4 and 37 °C agitated at 75 rpm (USP, Type II) in which from about 9% to about 11 % of compound (**1**) is released within about 2 hours; from about 19% to about 21% of compound (**1**) is released within 7 hours; and from about 28% to about 32% of compound (**1**) is released within about 11 hours. In certain embodiments, release of compound (**1**) from tablet dosage forms comprising an ammonioalkyl methacrylate copolymers and hydroxypropylmethyl cellulose exhibits an *in vitro* dissolution profile in 10 mM monobasic potassium phosphate buffer at pH 7.4 and 37°C agitated at 75 rpm (USP, Type II) in which from about 10% of compound (**1**) is released within about 2

hours; from about 20% of compound (**1**) is released within 7 hours; and from about 30% of compound (**1**) is released within about 11 hours.

## Therapeutic Uses

Sustained release oral dosage forms provided by the present disclosure may be administered to a patient suffering from any disease or disorder for which the parent drug, *R*-baclofen, is known, believed to be, or hereafter determined to be therapeutically effective. Indications for which *R*-baclofen has been prescribed, and hence for which the dosage forms of the present disclosure are also effective, include spasticity, gastro-esophageal reflux disease, narcotic addiction or abuse, alcohol addiction or abuse, nicotine addiction or abuse, emesis, cough, neuropathic pain, and musculoskeletal pain.

The suitability of dosage forms provided by the present disclosure in treating the above-listed diseases may be determined by methods described in the art (see, e.g., Bowery, Trends Pharmacol. Sci. 1989, 10, 401-407; Misgeld et al., Prog. Neurobiol. 1995, 46, 423-462; van Herwaarden et al., Aliment. Pharmacol. Ther. 2002, 16(9), 1655-62; Ciccaglione and Marzio, Gut 2003, 52(4), 464-470; Andrews et al., U.S. Patent No. 6,117,908; Fara *et al.,* International Publication No. WO 02/096404; Gessa *et al.,* International Publication No. WO 01/26638; Gessa *et al.,* International Publication No. WO 01/08675); Robson et al., U.S. Patent No. 4,126,684; Bountra et al., U.S. Patent No. 5,719,185; and Kreutner et al., U.S. Patent No. 5,006,560; Katz, Am. J. Phys. Med. Rehabil. 1988, 67(3), 108-16; Krach, J. Child Neurol. 2001, 16(1), 31-6; Bryans *et al.*, International Publication No. WO 01/90052; Bryans et al., EP 1178034; Cundy et al., U.S. Application Publication No. 2002/0151529; Gallop et al., U.S. Application Publication No. 2003/0176398; Gallop et al., U.S. Application Publication No. 2003/0171303; Gallop et al., U.S. Application Publication No. 2004/0006132; and Raillard et al., U.S. Application Publication No. 2004/0014940).

A suitable dose of compound (**1**) to be administered to a patient in need of *R*-baclofen therapy may be estimated based on the mass equivalent of *R*-baclofen and the enhanced oral bioavailability of *R*-baclofen provided by compound (**1**). *Spasticity*

Spasticity is estimated to affect about 500,000 people in the United States and more than 12 million people worldwide. Spasticity is an involuntary, velocity-dependent, increased resistance to stretch. Spasticity is characterized by muscle hypertonia in which there is increased resistance to externally imposed movement with increasing speed of stretch (Lance et al., Trans Am. Neurol. Assoc. 1970, 95, 272-274; and Sanger et al., Pediatrics 2003, 111, e89-e97). Spasticity can be caused by lack of oxygen to the brain before, during, or after birth (cerebral palsy); physical trauma (brain or spinal cord injury); blockage of or bleeding from a blood vessel in the brain (stroke); certain metabolic diseases; adrenolekodystrophy; phenylketonuria; neurodegenerative diseases such as Parkinson's disease and amyotrophic lateral sclerosis; and neurological disorders such as multiple sclerosis. Spasticity is associated with damage to the corticospinal tract and is a common complication of neurological disease. Diseases and conditions in which spasticity may be a prominent symptom include cerebral palsy, multiple sclerosis, stroke, head and spinal cord injuries, traumatic brain injury, anoxia, and neurodegenerative diseases. Patients with spasticity complain of stiffness, involuntary spasm, and pain. These painful spasms may be spontaneous or triggered by a minor sensory stimulus, such as touching the patient.

Symptoms of spasticity can include hypertonia (increased muscle tone), clonus (a series of rapid muscle contractions), exaggerated deep tendon reflexes, muscle spasms, scissoring (involuntary crossing of the legs), deformities with fixed joints, stiffness, and/or fatigue caused by trying to force the limbs to move normally. Other complications include urinary tract infections, chronic constipation, fever or other systemic illnesses, and/or pressure sores. The degree of spasticity can vary from mild muscle stiffness to severe, painful, and uncontrollable muscle spasms. Spasticity may coexist with other conditions but is distinguished from rigidity (involuntary bidirectional non-velocity-dependent resistance to movement), clonus (self-sustaining oscillating movements secondary to hypertonicity), dystonia (involuntary sustained contractions resulting in twisting abnormal postures), athetoid movement (involuntary irregular confluent writhing movements), chorea (involuntary, abrupt, rapid, irregular, and unsustained movements), ballisms (involuntary flinging movements of the limbs or body), and tremor (involuntary rhythmic repetitive oscillations, not self-sustaining). Spasticity can lead to orthopedic deformity such as hip dislocation, contractures, or scoliosis; impairment of daily living activities such as dressing, bathing, and toileting; impairment of mobility such as inability to walk, roll, or sit; skin breakdown secondary to positioning difficulties and shearing pressure; pain or abnormal sensory feedback; poor weight gain secondary to high caloric expenditure; sleep disturbance; and/or depression secondary to lack of functional independence.

Treatment of spasticity includes physical and occupational therapy such as functional based therapies, rehabilitation, facilitation such as neurodevelopmental therapy, proprioceptive neuromuscular facilitation, and sensory integration; biofeedback: electrical stimulation; and orthoses. Oral medications useful in treating spasticity include baclofen, benzodiazepines such as diazepam, dantrolene sodium; imidazolines such as clonidine and tizanidine; and gabapentin. Intrathecal medications useful in treating spasticity include baclofen. Chemodenervation with local anesthetics such as lidocaine and xylocaine; type A botulinum toxin and type B botulinum toxin; phenol and alcohol injection can also be useful in treating spasticity. Surgical treatments useful in treating spasticity include neurosurgery such as selective dorsal

rhizotomy; and orthopedic operations such as contracture release, tendon or muscle lengthening, tendon transfer, osteotomy, and arthrodesis.

The efficacy of dosage form provided by the present disclosure for the treatment of spasticity can be assessed using animal models of spasticity and in clinically relevant studies of spasticity of different etiologies. Animal models of spasticity are known *(see e.g.,* Eaton, J Rehab Res Dev 2003, 40(4), 41-54*;* Kakinohana et al., Neuroscience 2006, 141, 1569-1583; Ligresti et al., British J Pharm 2006, 147, 83-91; Zhang et al., Chinese J Clin Rehab, 2006, 10(38), 150-151; Hefferan et al., Neuroscience Letters 2006, 403, 195-200; and Li et al., J Neurophysiol 2004, 92, 2694-2703). For example, animal models of spasticity include (a) the mutant spastic mouse (Chai et al., Proc. Soc. Exptl. Biol. Med. 1962, 109, 491); (b) the acute/chronic spinally transected rat and the acute decerebrate rat *(see e.g.,* Wright and Rang, Clin Orthop Relat Res 1990, 253, 12-19; Shimizu et al., J Pharmacol Sci 2004, 96, 444-449; and Li et al., J Neurophysiol 2004, 92, 2694-2703); (c) primary observation Irwin Test in the rat (Irwin, Psychopharmacologia 1968, 13, 222-57); and d) Rotarod Test in the rat and mouse (Dunham et al., J. Am. Pharm. Assoc. 1957, 46, 208-09). Other animal models include spasticity induced in rats following transient spinal cord ischemia (Kakinohana et al., Neuroscience 2006, 141, 1569-1583*;* and Hefferan et al., Neuroscience Letters 2006, 403, 195-200), spasticity in mouse models of multiple sclerosis (Ligresti et al., British J Pharmacol 2006, 147, 83-91); and spasticity in rat models of cerebral palsy (Zhang et al., Chinese J Clin Rehabilitation 2006, 10(38), 150-151). The maximal electroshock seizure (MES) threshold test in rodents is sensitive for detecting potential anticonvulsant properties (Loscher and Schmidt, Epilepsy Res 1988, 2(3), 145-181). In this model, anticonvulsant agents elevate the threshold to electrically-induced seizures while proconvulsants lower the seizure threshold.

The efficacy of dosage forms provided by the present disclosure for treating spasticity may also be assessed in humans using double blind placebo-controlled clinical trials (*see e.g.,* Priebe et al., Spinal Cord 1997, 35(3), 171-5; Gruenthal et al., Spinal Cord 1997, 35(10), 686-9; Tuszynski et al., Spinal Cord 2007, 45, 222-231 and Steeves et al., Spinal Cord 2007, 45, 206-221 for examples of the conduct and assessment of clinical trials for spasticity caused by spinal cord injury). Clinical trial outcome measures for spasticity include the Ashworth Scale, the modified Ashworth Scale, muscle stretch reflexes, presence of clonus and reflex response to noxious stimuli. Spasticity can be assessed using methods and procedures known in the art such as a combination of clinical examination, rating scales such as the Ashworth Scale, the modified Ashworth scale the spasm frequency scale and the reflex score, biomechanical studies such as the pendulum test, electrophysiologic studies including electromyography, and functional measurements such as the Fugl-Meyer Assessment of Sensorimotor Impairment scale. Other measures can be used to assess spasticity associated with a specific disorder such as the Multiple Sclerosis Spasticity Scale (Hobart et al., Brain 2006, 129(1), 224-234).

### *Gastroesphageal Reflux Disease*

Gastroesophageal reflux disease (GERD) is defined as chronic symptoms or mucosal damage produced by the abnormal relfux in the esophagus. Symptoms of GERD include heartburn, esophagitis, stictures, dysphagia, chronic chest pain, cough, hoarsness, voice changes, chronic ear ache, burning chest pains, nausea, and sinusitis.

Tonic contraction of the lower esophageal sphincter is the principal factor preventing the reflux of gastric contents into the esophagus. Transient lower esophageal sphincter relaxation (TLESR) is the major mechanism underlying reflux in normal subjects and patients with GERD. $GABA_B$ agonists such as *R*-baclofen have been shown to reduce TLESRs in humans (Lidums et al., Gastroenterology 2000, 118(1), 7-13; Vela et al., Aliment Pharmacol Ther 2003, 17 (2), 243-51; Ciccaglione and Marzio, Gut 2003, 52(4), 464-70; and Zhang et al., Gut 2002, 50(1), 19-24). Reduction of the frequency of TLESRs by baclofen is believed to be due to inhibition of vagal afferents, information transfer between the nucleus tractus solitarious and dorsal moter nucleus of the vagus, and vagal efferent outflow (Hornby et al., Gastroenterol Clin N Am 2002, 31(4 Suppl), S11-S20).

More specifically, (3*R*)-4-{[(1*S*)-2-methyl-1-(2-methylpropanoyloxy)propoxy]carbonylamino}-3-(4-chlorophenyl)butanoic acid, compound (**1**), has been shown to reduce reflux episodes in clinical trials (Castell et al., Am J. Gastroenterology 2006, 101(suppl 2)(52), S59 and poster presentation at American College of Gastroenterology 2006 Annual Meeting, October 20-25, 2006, Las Vegas, NV; and Castell et al., Gastroenterology 2007, suppl. A, 486 and poster presentation at Digestive Disease Week Meeting, May 19-24, 2007, Washington D.C).

The efficacy for treating GERD may be assessed using animal models such as those described by Blackshaw et al., Am. J. Physiol. 1999, 277, G867-G874; Lehmann et al., Gastroenterology 1999, 117, 1147-1154; and Stakeberg and Lehmann, Neurogastroenterol. Mot. 1999, 11, 125-132; and in clinical trials.

### *Emesis*

Nausea, vomiting, and retching are basic human protective reflexes against the absorption of toxins as well as responses to certain stimuli. Nausea is a subjectively unpleasant wavelike sensation in the back of the throat or epigastrium associated with pallor or flushing, tachycardia, and an awareness of the urge to vomit. Sweating, excess salivation, and a sensation of being cold or hot may also occur. Vomiting is characterized by contraction of the abdominal muscles, descent of the diaphragm, and opening of the gastric cardia, resulting in forceful expulsion of stomach contents from the mouth. Retching involves spasmodic contractions of the diaphragm and the muscles of the thorax and abdominal wall without expulsion of gastric contents. Emesis is used herein to refer to nausea, vomiting, and/or retching.

Baclofen has been shown to suppress the retching and vomiting induced by morphine, thereby indicating the involvement of the $GABA_B$ receptor in the emetic control pathway (Suzuki et al., Neuropharmacology 2005, 49(8), 1121-31). Baclofen has also been shown to antagonize emesis induced by nicotine and motion in animal models (Chan et al., Eur J Pharmacology 2007, 559(2-3), 196-201).

Efficacy in treating emesis can be assessed using appropriate animal models and using clinical trials. For example, efficacy in treating emesis induced by chemotherapeutic agents can be determined based on effects indicative of emesis such as pica, gastric stasis, and reduced food intake in rats, mice, or ferrets (*see, e.g.,* Liu et al., Physiology & Behavior, 2005, 85, 271-277; Endo et al., Biogenic Amines, 2004, 18(3-6), 419-434; and Malik et al., Eur. J. Pharmacol, 2007, 555, 164-173. In clinical trials, assessment instruments such as the Duke Descriptive Scale, Visual Analog Scales, Morrow Assessment of Nausea and Emesis, Rhodes Index of Nausea and Vomiting Form-2, and Functional Living Index Emesis can be used to measure efficacy (*see, e.g.,* Rhodes et al., CA Cancer J Clin, 2001, 51, 232-248 and references therein). In general, adequately controlled, double blind placebo controlled trails may be used to evaluate efficacy in humans.

*Cough*

Cough reflex, elicited by activation of cough receptors located in the respiratory tract, clears inhaled irritants and foreign substances from the respiratory tract and in conjunction with the mucociliary system can expel excessive airway secretion produced under abnormal conditions from the respiratory tract. Cough can be caused by mild acuate upper respiratory tract infections, allergies, asthma, chronic obstructive pulmonary disease, lung cancer, gastroesophageal reflux disease, post-nasal drip, and heart or ear disorders. However, chronic non-productive cough having no identifiable cause accounts for a significant percent of patients presenting with cough. Chronic cough is associated with exacerbation of asthmatic symptoms, rib fractures, breathlessness, ruptured abdominal muscles, pneumothorax, syncope, second and third degree heart block, and loss of consciousness. Persistent and uncontrollable cough can lead to morbidity and severely impairs the quality of life of these patients.

Cough includes acute and chronic cough of any type, etiology, or pathogenesis, and in particular cough associated with laryngeal sensory neuropathy.

The anti-tussive effects of baclofen are well-known (*see e.g.,* Dicpinigaitis and Dobkin, Chest 1997, 111(4), 996-9; Dicpinigaitis and Rauf, Respiration 1998, 65(1), 86-8; Dicpinigaitis et al., J Clin Pharmacol 1998, 38(4), 364-7; and Kreutner et al., U.S. Patent No. 5,006,560 and International Publication No. WO 91/08740).

Efficacy in treating cough can be assessed using appropriate animal models and using clinical trials. Animal models of cough are reviewed by Lewis et al., Pulmonary Pharmacology & Therapeutics 2007, 20, 325-333.

*Substance Addiction or Abuse*

In clinical trials, *R*-baclofen has been shown to be effective in treating cocaine addiction (Brebner et al., Alcohol Alcohol 2002, 37(5), 478-84; and Haney et al., Neuropsychopharmacology 2006, 31, 1814-21); methamphetamine dependence (Heinzerling et al., Drug Alcohol Depend 2006, 85(3), 177-84); opioid dependence (Assadi et al., BMC Psychiatry 2003, 3(16*);* and Ahmadi-Abhari et al., J Clin Pharm Therapeutics 2001, 26(1), 67-71*);* alcohol craving and intake (Addolorato et al., Alcohol Alcohol 2002, 37(5), 504-8*;* and Flannery et al., Alcohol Clin Exp Res 2004, 28(10), 1517-23); nicotine use (Markou et al., Ann N.Y. Acad Sci 2004, 1025, 491-503); and drug addiction generally (Cousins et al., Drug Alcohol Dependence 2002, 65(3), 209-20*).*

Efficacy for treating substance addiction and abuse can be assessed using animal models and in clinical trials. Animal models of substance abuse disorders are known (*see e.g.,* Fattore et al., Alcohol & Alcoholism 2002, 37(5), 495-498 (nicotine); Spano et al., Neuropharmacology 2007, 52, 1555-1562 (opiate addiction); and Maccioni et al., Alcohol 2005, 36, 161-168 (alcohol abuse).

*Neuropathic Pain*

It is estimated that neuropathic pain affects over 6 million patients in the U.S. and Europe and over 26 million patients worldwide. Neuropathic pain involves an abnormal processing of sensory input usually occurring after direct injury or damage to nerve tissue. Neuropathic pain is a collection of disorders characterized by different etiologies including infection, inflammation, disease such as diabetes and multiple sclerosis, trauma or compression to major

peripheral nerves, and chemical or irradiation-induced nerve damage (Jensen et al., Eur J Pharmacol 2001, 429, 1-11). Neuropathic pain typically persists long after tissue injury has resolved. Prodrugs of $GABA_B$ agonists provided by the present disclosure can be used to treat neuropathic pain. In certain embodiments, prodrugs of $GABA_B$ agonists provided by the present disclosure can be used to treat neuropathic pain including, for example, post-herpetic neuralgia, peripheral neuropathy, trigeminal neuralgia, painful diabetic neuropathy, HIV-related neuropathic pain, cancer-related pain, or fibromyalgia.

International Association for the Study of Neuropathic Pain defines neuropathic pain states as disorders that are characterized by lesions or dysfunction of the neural system(s) that under normal conditions transmit noxious information to the central nervous system. The mechanisms underlying neuropathic pain conditions are highly heterogeneous, however, all types of neuropathic pain are presumed to involve nerve injury and certain common aberrations in somatosensory processing in the central and/or peripheral nervous system (Baron, Nat Clin Pract Neurol 2006, 2, 95-106; and Beggs and Salter, Drug Dev Res 2006, 67, 289-301). Potential causes of neuropathic pain include physical damage, infection, and chemical exposure. Neuropathic pain can be generally classified as a focal/multifocal lesion of the peripheral nervous system, *e.g.*, post-herpetic neuralgia, a generalized lesion of the peripheral nervous system, *e.g.*, painful diabetic neuropathy, HIV-related NP), a lesion of the central nervous system, or a more complex neuropathic disorder. Peripheral neuropathic pain can arise as a consequence of trauma and surgery related nerve injury, *e.g.*, brachial plexus injury; entrapment neuropathies such as lumbar disc compression, carpal tunnel syndrome; disease-related neuropathies, *e.g.*, diabetes and HIV-AIDS; radiculopathy; complex regional pain syndrome; and/or tumor growth leading to nerve compression or infiltration. Central neuropathic pain can be the result of stroke, multiple sclerosis, post-ischemic myelopathy; post-herpetic neuralgia; and/or post-traumatic spinal cord injury.

An essential part of neuropathic pain is a partial or complete loss of afferent sensory function and the paradoxical presence of certain hyperphenomena in the painful area. The nerve tissue lesion may be found in the brain, spinal cord, or the peripheral nervous system. Symptoms vary depending on the condition and can manifest as hyperalgesia (the lowering of pain threshold and an increased response to noxious stimuli), allodynia (the evocation of pain by non-noxious stimuli such as cold, warmth, or touch), hyperpathia (an explosive pain response that is suddenly evoked from cutaneous areas with increased sensory detection threshold when the stimulus intensity exceeds sensory threshold), paroxysms (a type of evoked pain characterized by shooting, electric, shock-like or stabbing pain that occur spontaneously, or following stimulation by an innocuous tactile stimulus or by a blunt pressure), paraesthesia (abnormal but non-painful sensations, which can be spontaneous or evoked, often described as pins and needles), dysesthesia (abnormal unpleasant but not necessarily painful sensation, which can be spontaneous or provoked by external stimuli), referred pain and abnormal pain radiation (abnormal spread of pain), and wind-up like pain and aftersensations (the persistence of pain long after termination of a painful stimulus).

Patients with neuropathic pain typically describe burning, lancinating, stabbing, cramping, aching, and sometimes vice-like pain. The pain can be paroxysmal or constant. Pathological changes to the peripheral nerve(s), spinal cord, and brain have been implicated in the induction and maintenance of chronic pain. Patients suffering from neuropathic pain typically endure chronic, debilitating episodes that are refractory to current pharmacotherapies and profoundly affect their quality of life. Currently available treatments for neuropathic pain, including tricyclic antidepressants and gabapentin, typically show limited efficacy in the majority of patients (Sindrup and Jensen, Pain 1999, 83, 389-400).

There are several types of neuropathic pain. A classification that relates to the type of damage or related pathophysiology causing a painful neuropathy includes neuropathies associated with mechanical nerve injury such as carpal tunnel syndrome, vertebral disk herniation, entrapment neuropathies, ulnar neuropathy, and neurogenetic thoracic outlet syndrome; metabolic disease associated neuropathies such as diabetic polyneuropathy; neuropathies associated with neurotropic viral disease such as herpes zoster and human immunodeficiency virus (HIV) disease; neuropathies associated with neurotoxicity such as chemotherapy of cancer or tuberculosis, radiation therapy, drug-induced neuropathy, and alcoholic neuropathy; neuropathies associated with inflammatory and/or immunologic mechanisms such as multiple sclerosis, anti-sulfatide antibody neuropathies, neuropathy associated with monoclonal gammopathy, Sjogren's disease, lupus, vasculitic neuropathy, polyclonal inflammatory neuropathies, Guillain-Barre syndrome, chronic inflammatory demyelinating neuropathy, multifocal motor neuropathy, paraneoplastic autonomic neuropathy, ganglinoic acetylcholine receptor antibody autonomic neuropathy, Lambert-Eaton myasthenic syndrome and myasthenia gravis; neuropathies associated with nervous system focal ischemia such as thalamic syndrome (anesthesia dolorosa); neuropathies associated with multiple neurotransmitter system dysfunction such as complex regional pain syndrome (CRPS); neuropathies associated with chronic/neuropathic pain such as osteoarthritis, low back pain, fibromyalgia, cancer bone pain, chronic stump pain, phantom limb pain, and paraneoplastic neuropathies; toxic neuropathies (*e.g.*, exposure to chemicals such as exposure to acrylamide, 3-chlorophene, carbamates, carbon disulfide, ethylene oxide, n-hexane, methyl n-butylketone, methyl bromide, organophosphates, polychlorinated biphenyls, pyriminil, trichlorethylene, or dichloroacetylene), focal traumatic neuropathies, phantom and stump pain, monoradiculopathy, and trigeminal neuralgia; and central neuropathies including ischemic cerebrovascular injury (stroke), multiple sclerosis, spinal cord injury, Parkinson's disease, amyotrophic lateral sclerosis, syringomyelia, neoplasms, arachnoiditis, and post-operative pain; mixed neuropathies such as diabetic neuropathies (including symmetric polyneuropathies such as sensory or sensorimotor polyneuropathy, selective small-fiber polyneuropathy, and autonomic neuropathy; focal and multifocal neuropathies such as cranial neuropathy, limb mononeuropathy, trunk mononeuropathy, mononeuropathy multiplex, and asymmetric lower limb motor neuropathy) and sympathetically maintained pain. Other neuropathies include focal neuropathy; glosopharyngeal neuralgia; ischemic pain; trigeminal neuralgia; atypical facial pain associated with Fabry's disease, Celiac disease, hereditary sensory neuropathy, or $B_{12}$-deficiency; mono-neuropathies; polyneuropathies; hereditary peripheral neuropathies such as Carcot-Marie-Tooth disease, Refsum's disease, Strumpell-Lorrain disease, and retinitis pigmentosa; acute polyradiculoneuropathy; and chronic polyradiculoneuropathy. Paraneoplastic neuropathies include paraneoplastic subacute sensory neuropathy, paraneoplastic motor neuron disease, paraneoplastic neuromyotonia, paraneoplastic demyelinating neuropathies, paraneoplastic vasculitic neuropathy, and paraneoplastic autonomic insufficiency. Prodrugs of $GABA_B$ agonists such as *R*-baclofen prodrug (**1**) can be used to treat any of the foregoing types of neuropathic pain. In certain embodiments, the neuropathic pain is chosen from post-herpetic neuralgia, peripheral neuropathy, trigeminal neuralgia, painful diabetic neuropathy, HIV-related neuropathic pain, cancer-related pain, and fibromyalgia. In certain embodiments, the neuropathic pain is chosen from post-herpetic neuralgia and trigeminal neuralgia.

The $GABA_B$ agonist (*R*,*S*)-baclofen has long been known to have antinociceptive activity in models of acute pain and recent studies have shown that baclofen inhibits allodynia and hyperalgesia in the chronic constriction injury and spinal nerve ligation models of persistent neuropathic pain at doses lower than those required to produce sedation and impairment of motor activity *(see e.g.,* Hwang and Yaksh, Pain 1997, 70(1), 15-22; Smith et al., Neruopharmacology 1994, 33(9), 1103-8; Patel et al., Pain 2001, 90(3), 217-26; Balerio and Rubio, Pharmacol Res 2002, 46(3), 281-6; and Reis and Duarte, Br J Pharmacol 2006,149(6), 733-9).

In clinical studies, intrathecal baclofen administration has been shown to be effective in treating neuropathic pain associated with spinal-cord injury and multiple sclerosis (Herman et al., Clin J Pain 1992, 8(4), 338-45), painful extremity paresthesias (Gatscher et al., Acta Neurochir Suppl 2002, 79, 75-76), sympathetically maintained pain (Van Hilten et al., N Engl J Med 2000, 343(9), 625-30; Becker et al., J Clin Neurosci 2000, 7(4), 316-9; and Zuniga et al., Reg Anesth Pain Med 2002, 27(1), 90-3). $GABA_B$ agonists such as baclofen have also been shown to be effective in trigeminal, gloospharyngeal, vagoglossopharyngeal, and ophthalmic-postherpetic neuralgias (Fromm et al., Neurology 1981, 31 (6), 683-7; and Ringel and Roy, Ann Neurol 1987, 21(5), 514-5); and in patients with diabetic neuropathy (Anghinah et al., Muscle Nerve 1994, 17(8), 958-59). Doses of baclofen from about 50 mg/day to about 60 mg/day have been shown to be effective in treating trigeminal neuralgia (Fromm et al., Ann Neurol 1984, 15(3), 240-4).

The efficacy of prodrugs of $GABA_B$ agonists provided by the present disclosure for treating one or more types of neuropathic pain can be assessed in animal models of neuropathic pain and in clinical trials *(see e.g.,* Beggs and Salter, Drug Dev Res 2006, 67, 829-301). Useful animal models of neuropathic pain include peripheral nerve injury by ligation or transection include dorsal rhizotomy (Lombard et al., Pain 1979, 6(2), 163-174); spinal nerve ligation (Kim and Chung, Pain 1992, 50, 355-363; and Hwang and Yaksh, Pain 1997, 70(1), 15-22); sciatic nerve transaction (Wall et al., Pain 1979, 7, 103-111); sciatic nerve cuff (Mosconi and Kruger, Pain 1996, 64, 37-57); partial nerve ligation (Seltzer et al., Pain 1990, 43, 205-218); chronic constriction (Bennett and Xie, Pain 1988, 33, 87-107); rat spinal cord ischemia model (Hao et al., Pain 1991, 45, 175-185; and von Heijne et al., Eur J Pain 2001, 5, 1-10); and spared nerve injury (Decosterd and Woolf, Pain 2000, 87, 149-158). Other animal models of neuropathies involving immune system activation, and metabolic and chemically induced neuropathies include sciatic cyroneurolysis (DeLeo et al., Pain 1994, 56, 9-16); zymosan-induced neuritis (Chacur et al., Pain 2001, 94, 231-244); HIV gp120-induced pain model (Herzberg and Sagen, J Neuroimmunol 2001, 116, 29-39); photochemical ischemia (Kupers et al., Pain 1998, 76(1-2), 45-59); antiganglioside antibody (Slart et al., Pain 1997, 69, 119-125); streptozotocin-neuropathy (Fox et al., Pain 1999, 81, 307-316*);* DDI-induced myelinopathy *(*Joseph et al., Pain 2004, 107, 147-158); formalin phase 2 model of hyperalgesic pain (Dirig and Yaksy, J Pharmacology Exper Ther 1995, 275, 219-227); vincristine-induced pain model (Aley et al., Neuroscience 1996, 73, 259-265); paclitaxel-induced pain model (Cavaletti et al., Exp Neurol 1995, 133, 64-72); and cisplatin-induced pain model (Authier et al., Neurosci Lett 2000, 25, 2576-2585).

The efficacy of prodrugs of $GABA_B$ agonists provided by the present disclosure for treating various types of neuropathic pain can also be assessed in clinical trials using, for example, randomized double-blind placebo controlled methods. End points used in clinical trials for neuropathic pain can be determined using validated neuropathic pain criteria such as the Brief Pain Inventory, Categorical Scale, Gracely Pain Scale, Likert Scale, Neuropathic Pain Scale, Numerical Pain Scale, Short Form McGill Pain Questionnaire, Verbal Pain Scale, Visual Analog Scale (VAS), VAS Pain Intensity Scale, and/or VAS Pain Relief Scale.

*Musculoskeletal Pain*

Musculoskeletal conditions causing tenderness and musculoskeltal pain and muscle spasms include fibromyalgia, tension headaches, myofascial pain syndrome, facet joint pain, internal disk disruption, somatic dysfunction, spinal fractures, vertebral osteomyelitis, polymyalgia rheumatica, atlantoaxial instability, atlanto-occipital joint pain, osteoporotic

vertebral compression fracture, Scheuermann's disease, spondyloysis, spondylolisthesis, kissing spines, sacroiliac joint pain, sacral stress fracture, coccygodynia, failed back syndrome, and mechanical low back or neck pain (*see, e.g.,* Meleger and Krivickas, Neurol Clin 2007, 25, 419-438. In these conditions, muscle spasm is related to local factors involving the affected muscle groups without the increased tone or reflex characteristic of spasticity. Muscle, tendon, ligament, intervertebral disc, articular cartilage, and bone can be involved in musculoskeletal pain. Disorders that can produce neck and back pain include muscle strain, ligament sprain, myofascial pain, fibromyalgia, facet joint pain, internal disc disruption, somatic dysfunction, spinal fracture, vertebral osteomyelitis, and polymyalgia rheumatica, atlantoaxial instability and atlanto-occipital joint pain. *(see e.g.,* Meleger and Krivickas, Neurological Clinics 2007, 25(2), 419-438).

Studies have shown that $GABA_B$ agonists can be effective in treating muscular pain and/or spasms associated with peripheral musculoskeletal conditions. Baclofen has been shown effective in treating migraine (Hering-Hanit, Cephalalgia 1999, 19(6), 589-91; and Hering-Hanit and Gadoth, Headache 2000, 40(1), 48-51) and specifically in tension-type headaches (Freitag, CNS Drugs 2003, 17(6), 373-81); and in low-back pain and radiculopathy (Slonimski et al., Reg Anesth Pain Med 2004, 29(3), 269-76; Dapas et al., Spine 1985, 10(4), 345-9; and Raphael et al., BMC Musculoskeletal Disorders 2002, 3(17).

The efficacy of prodrugs of $GABA_B$ agonists provided by the present disclosure for treating one or more types of musculoskeletal pain can be assessed in animal models of neuropathic pain and in clinical trials. Kehl *et al.*, disclose an animal model of muscle hyperplasia that employs intramuscular injection of carrageenan as useful for assessing the mechanisms and management of musculoskeletal pain (Kehl et al., Pain 2000, 85, 333-343).

Low back pain generally occurs in the lumbar region of the back in the location of lumbar vertebrae L1-L5. Pain in the lower back can be caused by a sprain, strain, or spasm to one of the muscles, ligaments, facet joints, and/or sacroiliac joints in the back; spinal sprain or overcompression; or disc rupture or bulge. Low back pain may also reflect nerve or muscle irritation or bone lesions. Most low back pain follows injury or trauma to the back, but pain may also be caused by degenerative conditions such as arthritis or disc disease, osteoporosis, or other bone diseases, viral infections, irritation to joints and discs, or congenital abnormalities in the spine. Obesity, smoking, weight gain during pregnancy, stress, poor physical condition, posture inappropriate for the activity being performed, and poor sleeping position also may contribute to low back pain. Additionally, scar tissue created when the injured back heals itself does not have the strength or flexibility of normal tissue. Buildup of scar tissue from repeated injuries eventually weakens the back and can lead to more serious injury. Occasionally, low back pain may indicate a more serious medical problem. Pain accompanied by fever or loss of bowel or bladder control, pain when coughing, and progressive weakness in the legs may indicate a pinched nerve or other serious condition. People with diabetes may have severe back pain or pain radiating down the leg related to neuropathy. Low back pain can be caused by bulging disc (e.g., protruding, herniated, or ruptured disc), sciatica, spinal degeneration, spinal stenosis, osteoporosis, osteoarthritis, compression fractures, skeletal irregularities, fibromyalgia, spondylolysis and/or spondylolisthesis. Less common spinal conditions that can cause low back pain include ankylosing spondylitis, bacterial infections, osteomyelitis, spinal tumors, Paget's disease, and Scheuermann's disease. Clinical results suggest that $GABA_B$ agonists such as baclofen can be effective in treating low back pain (Dapas et al., Spine 1985, 10(4), 345-9; and Raphael et al., BMC Musculoskeletal Disorders 2002, 3(17*).* For example doses of baclofen from about 20 mg/day to about 80/mg day have been shown to be effective in treating acute low back pain (Dapas et al., Spine 1985, 10(4), 345-9).

In certain embodiments, methods of treating low back pain provided by the present disclosure comprises treating disorders, conditions, and/or symptoms associated with low back pain such as muscle spasms. Symptoms of low back pain can depend on the cause. For example, symptoms of back sprain or back strain include muscle spasms, cramping, stiffness, and pain centered in the back and buttocks. Symptoms of nerve-root pressure include leg pain, also referred to as sciatica, and nerve-related manifestations such as tingling, numbness, or weakness in one leg or in the foot, lower leg, or both legs. Symptoms of arthritis of the spine include pain and stiffness that are worse in the back and hip.

Fibromyalgia is a condition characterized by aching and pain in muscles, tendons and joints all over the body, but especially along the spine. The body also is tender to touch in specific areas referred to as tender or trigger points. Other symptoms of fibromyalgia include sleep disturbance, depression, daytime tiredness, headaches, alternating diarrhea and constipation, numbness and tingling in the hands and feet, feelings of weakness, memory difficulties, and dizziness. Although the etiology of fibromyalgia is not known, stress, disordered sleep patterns, abnormal production of pain-related chemicals in the nervous system, and/or low levels of growth hormone are believed to contribute to the onset of fibromyalgia.

Fibromyalgia usually occurs in people between 20 and 60 years of age and is estimated to affect 3.4% of women and 0.5% of men. The incidence of juvenile primary fibromyalgia in school age girls is estimated to be about 1.2%.

Current treatment of fibromyalgia is based on symptoms, with the goal of alleviating pain, restoring sleep, and improving general quality of life. Several nonpharmacologic treatments include exercise, education, behavioral and physical therapy. Pharmacologic treatments include tricyclic compounds, serotonin reuptake inhibitors, analgesics, muscle relaxants, and ACE inhibitors. There is evidence suggesting that $GABA_B$ agonists such as baclofen may be useful in improving fibromyalgia symptoms (Taylor-Gjevre and Gjevre, Lupus 2005, 14(6), 486-8).

The efficacy of administering compounds provided by the present disclosure for treating fibromyalgia may be assessed using animal models of fibromyalgia and in clinical results (*see e.g.,* Dooley et al., U.S. Application Publication Nos. 2004/0180959 and 2004/0138305; Crofford et al., Arthritis & Rheumatism 2005, 52, 4, 1264-1273; Eaton, J Rehabilitation Research and Development 2003, 40(4), 41S-54S; Guay, Am J Geriatr Pharmacother 2005, 3, 274-287; Freynhagen et al., Pain 2005,115, 254-263; Backonja et al., Clin Ther. 2003, 25, 81-104; Gidal et al., Am J Manag Care. 2006,12, S269-S278; and Argoff, JAOA, 2002, Suppl. 3, 102(9), S21-S26). In particular, animal models of neuropathic pain or clinically relevant studies of different types of neuropathic pain have been found useful in assessing therapeutic activity for treating fibromyalgia, such as are disclosed, for example, in Bennett and Xie, Pain 1988, 33, 87-107; Chaplan et al., J Neurosci. Meth. 1994, 53, 55-63; Fox et al., Pain 2003, 105, 355-362; Milligan et al., Brain Res. 2000, 861, 105-116; De Vry et al., Eur. J Pharmacol. 2004, 491, 137-148; and Polomano et al., Pain 2001, 94, 293-304.

## Dosing

The amount of a compound (**1**) that will be effective in the treatment of a particular disease disclosed herein will depend, at least in part, on the nature of the disease, and may be determined by standard clinical techniques known in the art as previously described. In addition, *in vitro* or *in vivo* assays can optionally be employed to help identify optimal dosage ranges. Dosage regimens and dosing intervals may also be determined by methods known to those skilled in the art. The amount of compound (**1**) administered may depend on, among other factors, the subject being treated, the weight of the subject, the severity of the disease or disorder, the manner of administration, and the judgment of the prescribing physician.

A dose of compound (**1**) can be adjusted to provide an equivalent molar quantity or mass equivalent dose of *R*-baclofen. A dose can comprise multiple dosage forms provided by the present disclosure. Therapeutically effective doses of *R*-baclofen are generally from about 0.03 mg to about 1 mg per kilogram body weight per day. In certain embodiments, a daily dose can comprise a mass equivalent of *R*-baclofen ranging from about 1 mg to about 100 mg, in certain embodiments, from about 5 mg to about 80 mg, in certain embodiments, from about 5 mg to about 60 mg, and in certain embodiments, from about 10 mg to about 40 mg. In certain embodiments, a dose of compound (**1**) is less than a dose that causes moderate sedation and impairment of motor activity in a patient. The dose of compound (**1**) and appropriate dosing intervals can be selected to maintain a sustained therapeutically effective concentration of *R*-baclofen in the blood of a patient, and in certain embodiments, without exceeding a minimum adverse concentration.

In certain embodiments, dosage forms provided by the present disclosure may be administered once per day, twice per day, and in certain embodiments at intervals greater than once per day. Dosing may be provided alone or in combination with other drugs and may continue as long as required for effective treatment of the disease. Dosing includes administering a dosage form to a mammal, such as a human, in a fed or fasted state.

## Combination Therapy

Dosage forms provided by the present disclosure may further comprise one or more pharmaceutically active compounds in addition to compound (**1**). Such compounds may be provided to treat the same disease or a different disease than the disease being treated with compound (**1**).

In certain embodiments, compound (**1**) may be used in combination with at least one other therapeutic agent. In certain embodiments, compound (**1**) may be administered to a patient together with another compound for treating movement disorders such as spasticity, digestive disorders such as gastro-esophageal reflux disease and emesis, or addictive or abuse disorders such as nicotine addiction or abuse, alcohol addiction or abuse, narcotic addiction or abuse, cough, neuropathic pain, or musculoskeletal pain. In certain embodiments, the at least one other therapeutic agent may be a different *R*-baclofen prodrug. Compound (**1**) and the at least one other therapeutic agent may act additively or, and in certain embodiments, synergistically. The at least one additional therapeutic agent may be included in the same dosage form comprising compound (**1**) or may be in a separate dosage form. Accordingly, methods provided by the present disclosure can further include, in addition to administering compound (**1**), administering one or more therapeutic agents effective for treating the same or different disease than the disease being treated by compound (**1**). Methods provided by the present disclosure include administration of compound (**1**) and one or more other therapeutic agents provided that the combined administration does not inhibit the therapeutic efficacy of compound (**1**) and/or does not produce adverse combination effects.

In certain embodiments, dosage forms comprising compound (**1**) may be administered concurrently with the administration of another therapeutic agent, which may be part of the same dosage form as, or in a different dosage form than that comprising compound (**1**). Compound (**1**) may be administered prior or subsequent to administration of another therapeutic agent. In certain embodiments of combination therapy, the combination therapy may comprise alternating between administering compound (**1**) and a composition comprising another therapeutic agent, e.g., to minimize adverse drug effects associated with a particular drug. When compound (**1**) is administered concurrently with

another therapeutic agent that potentially may produce an adverse drug effect including, but not limited to, toxicity, the other therapeutic agent may advantageously be administered at a dose that falls below the threshold at which the adverse drug reaction is elicited.

In certain embodiments, dosage forms comprising compound (**1**) may be administered with one or more substances to enhance, modulate and/or control release, bioavailability, therapeutic efficacy, therapeutic potency, stability, and the like of compound (**1**). For example, to enhance the therapeutic efficacy of compound (**1**) or its metabolite, *R*-baclofen, compound (**1**) may be co-administered with or a dosage form comprising compound (**1**) may comprise one or more active agents to increase the absorption or diffusion of compound (**1**) or *R*-baclofen from the gastrointestinal tract to the systemic circulation, or to inhibit degradation of compound (**1**) or *R*-baclofen in the blood of a patient. In certain embodiments, a dosage form comprising compound (**1**) may be co-administred with an active agent having pharmacological affects that enhance the therapeutic efficacy of compound (**1**).

Additionally, dosage forms provided by the present disclosure may be used in combination with other drugs that are themselves known to cause spasticity, gastro-esophageal reflux disease, narcotic addiction or abuse, alcohol addiction or abuse, nicotine addiction or abuse, emesis, cough, neuropathic pain, and/or musculoskeletal pain as an adverse effect, thereby preventing or reducing the occurrence of such adverse effects.

In certain embodiments, dosage forms provided by the present disclosure may be administered to a patient for treating a movement disorder such as spasticity in combination with a therapy or another therapeutic agent known or believed to be effective in treating a movement disorder such as spasticity. Examples of drugs for treating movement disorders such as spasticity and which may be administered in conjunction with compound (**1**) include levodopa, mild sedatives such as benzodiazepines including alprazolam, chlordiazepoxide, clonazepam, clorazepate, diazepam, lorazepam, and oxazepam; muscle relaxants such as baclofen, anticholinergic drugs such as trihexyphenidyl and diphenhydramine; antipsychotics such as chlorpromazine, fluphenazine, haloperidol, loxapine, mesoridazine, molindone, perphenazine, pimozide, thioridazine, thiothixene, trifluoperazine, aripiprazole, clozapine, olanzapine, quetiapine, risperidone, and ziprasidone; and antidepressants such as amitriptyline.

In certain embodiments, dosage forms provided by the present disclosure may be administered to a patient for treating a gastrointestinal disorder such as gastro-esophageal reflux disease in combination with a therapy or another therapeutic agent known or believed to be effective in treating a gastrointestinal disorder such as gastro-esophageal reflux disease. Examples of drugs for treating gastrointestinal disorders such as gastro-esophageal reflux disease and which may be administered in conjunction with compound (**1**) include H2 inhibitors such as cimetidine, famotidine, nizatidine, and ranitidine; proton pump inhibitors such as omeprazole, lansoprazole, pantoprazole, rabeprazole, and exomeprazole; and prokinetics such as cisparide, bethanechol, and metoclopramide.

In certain embodiments, dosage forms provided by the present disclosure may be administered to a patient for treating emesis in combination with a therapy or another therapeutic agent known or believed to be effective in treating emesis. Examples of drugs for treating emesis (nausea and vomiting) and which may be administered in conjunction with compound (**1**) include benzamines such as metoclopramide; phenothiazines such as prochlorperazine, perphenazine, chlorpromazine, promethazine, and thiethylperazine; butyrophenones such as droperidol and haloperidol; dopamine 2 antagonists such as metoclorpamide; 5-HT3 antagonists such as ondansetron, granisetron, dolasetron, palonosetron; NK-1 receptor antagonists such as aprepitant, corticosteroids such as dexamethazone; antihistamines such as diphenhydramine and hydroxyzine; cannabinoids such as dronabinol; and benzodiazepines such as lorazepam, midazolam, alprazolam, and olanzapine.

In certain embodiments, dosage forms provided by the present disclosure may be administered to a patient for treating alcohol addiction or abuse in combination with a therapy or another therapeutic agent known or believed to be effective in treating alcohol addiction or abuse. Examples of drugs for treating alcohol addiction or abuse and which may be administered in conjunction with compound (**1**) include disulfiram, naltrexone, clonidine, methadone, 1-alpha-acetylmethadol, buprenorphine, and bupropion.

In certain embodiments, dosage forms provided by the present disclosure may be administered to a patient for treating narcotic addiction or abuse in combination with a therapy or another therapeutic agent known or believed to be effective in treating narcotic addiction or abuse. Examples of drugs for treating narcotic addiction or abuse and which may be administered in conjunction with compound (**1**) include buprenorphine, tramadol, methadone, and naltrexone.

In certain embodiments, dosage forms provided by the present disclosure may be administered to a patient for treating nicotine addiction or abuse in combination with a therapy or another therapeutic agent known or believed to be effective in treating nicotine addiction or abuse. Examples of drugs for treating nicotine addiction or abuse and which may be administered in conjunction with compound (**1**) include bupropion, clonidine, and nicotine.

In certain embodiments, dosage forms provided by the present disclosure may be administered to a patient for treating cough in combination with a therapy or another therapeutic agent known or believed to be effective in treating cough. Examples of drugs for treating cough and which may be administered in conjunction with compound (**1**) include dextromethorphan, guaifenesin, hydrocodone, benzonatate, diphenhydramine, pseudoephedrine, acetaminophen, and carbinoxamine.

In certain embodiments, dosage forms provided by the present disclosure may be administered to a patient for treating neuropathic pain in combination with a therapy or another therapeutic agent known or believed to be effective in treating neuropathic pain. Examples of drugs useful for treating pain include opioid analgesics such as morphine, codeine, fentanyl, meperidine, methadone, propoxyphene, levorphanol, hydromorphone, oxycodone, oxymorphone, tramadol and pentazocine; nonopioid analgesics such as aspirin, ibuprofen, ketoprofen, naproxen, and acetaminophen; non-steroidal anti-inflammatory drugs such as aspirin, choline magnesium trisalicylate, diflunisal, salsalate, celecoxib, rofecoxib, valdecoxib, diclofenac, etodolac, fenoprofen, flubiprofen, ibuprofen, indomethacin, ketoprofen, ketorolac, meclofanamate, mefenamic acid, meloxicam, nabumetone, naproxen, oxaprozin, piroxicam, sulindac, and tometin; antiepileptics such as gabapentin, pregabalin, carbamazepine, phenytoin, lamotrigine, and topiramate; antidepressants such as duloxetine, amitriptyline, venlafaxine, nortryptyline, imipramine, and desipramine; local anesthetics such as lidocaine, and mexiletine; NMDA receptor antagonists such as dextropethorphan, memantine, and ketamine; N-type calcium-channel blockers such as ziconotide; vanilloid receptor-1 modulators such as capsaicin; cannabinoid receptor modulators such as sativex; neurokinin receptor antagonists such as lanepitant; other analgesics such as neurotropin; and other drugs such as desipramine, clonazepam, divalproex, oxcarbazepine, divalproex, butorphanol, valdecoxib, vicoprofen, pentazocine, propoxyhene, fenoprofen, piroxicam, indometnacin, hydroxyzine, buprenorphine, benzocaine, clonidine, flurbiprofen, meperidine, lacosamide, desvenlafaxine, and bicifadine.

In certain embodiments, a drug useful for treating neuropathic pain is chosen from propoxyphene, meperidine, hydromorphone, hydrocodone, morphine, codeine, 2-piperidinol-1-alkanol, eliprodil, ifenprodil, rofecoxib, celecoxib, salicylic acid, diclofenac, piroxicam indomethacin, ibuprofen, naproxen, gabapentin, carbemazepine, pregabalin, topiramate, valproic acid, sumatriptan, elitriptan, rizatriptan, zolmitriptan, naratriptan, flexeril, carisoprodol, robaxisal, norgesic, dantrium, diazepam, chlordiazepoxide, alprazolam, lorazepam, acetaminophen, nitrous oxide, halothane, lidocaine, etidocaine, ropivacaine, chloroprocaine, sarapin, bupivacaine, capsicin, desipramine, amitriptyline, doxepin, perphenazine, protriptyline, tranylcypromine, baclofen, clonidine, mexelitine, diphenhydramine, hydroxyzine, caffeine, prednisone, methyl-prednisone, decadron, sertraline, paroxetine, fluoxetine, tramadol, levodopa, dextromethorphan, substance P antagonists, and botulinum toxin.

In certain embodiments, a drug useful for treating neuropathic pain can be chosen from a nicotine receptor partial agonist and an analgesic agent as disclosed by Coe et al., U.S. Application Publication No. 2003/0133951; a 1-aryl-3-azabicyclo[3.1.0] hexane as disclosed by Lippa et al., U.S. Application Publication No. 2007/00892939; and a nitro(cyano)vinylpiperazine compound as disclosed by Sun and Tafesse, U.S. Application Publication No. 2007/0032500.

Non-pharmacological therapies for treating neuropathic pain include transcutaneous electrical nerve stimulation, percutaneous electrical nerve stimulation, and acupuncture.

In certain embodiments, dosage forms provided by the present disclosure may be administered to a patient for treating fibromyalgia in combination with a therapy or another therapeutic agent known or believed to be effective in treating fibromyalgia, or in certain embodiments, a disease, disorder, or condition associated with fibromyalgia. Drug therapy for fibromyalgia may be tailored to the severity and frequency of fibromyalgia episodes. For occasional episodes, acute treatment may be indicated. For fibromyalgia episodes occurring two or more times per month, or when attacks greatly impact the patient's daily life, chronic therapy on an ongoing basis may be appropriate.

Treatments for fibromyalgia that reduce the frequency of episodes and include non-steroidal anti-inflammatory agents (NSAIDs), adrenergic beta-blockers, calcium channel blockers, tricyclic antidepressants, selective serotonin reuptake inhibitors, anticonvulsants, NMDA receptor antagonists, dopamine agonists, selective 5-$HT_3$ receptor antagonists, opioids, muscle relaxants, sedative hypnotics, and other therapy. Examples of NSAIDs useful for treating fibromyalgia include aspirin, ibuprofen, fenoprofen, flurbiprofen, ketoprofen, mefenamic acid, and naproxen. Examples of adrenergic beta-blockers useful for treating fibromyalgia include acebutolol, atenolol, imilol, metoprolol, nadolol, pindolol, propranolol, and timolol. Examples of calcium channel blockers useful for treating fibromyalgia include amlodipine, diltiazem, dotarizine, felodipine, flunarizine, nicardipine, nifedipine, nimodipine, nisoldipine, and verapamil. Examples of tricyclic antidepressants useful for treating fibromyalgia include amitriptyline, desipramine, doxepin, imipramine, nortriptyline, cyclobenzaprine, and protriptyline. Examples of selective serotonin reuptake inhibitors useful for treating fibromyalgia include fluoxetine, methysergide, nefazodone, paroxetine, sertraline, citalopram, and venlafaxine. Examples of other antidepressants useful for treating g fibromyalgia include bupropion, nefazodone, norepinephrine, venlafaxine, duloxetine, and trazodone. Examples of anticonvulsants (antiepileptics) useful for treating fibromyalgia include divalproex sodium, felbamate, gabapentin, lamotrigine, levetiracetam, oxcarbazepine, tiagabine, topiramate, valproate, and zonisamide. Examples of NMDA receptor antagonists useful for treating fibromyalgia include dextromethorphan, magnesium, and ketamine. Examples of dopamine agonists useful for treating fibromyalgia include $\alpha$-dihydroergocryptine. Examples of opioids useful for preventing fibromyalgia are tramadol, oxycodone, and methadone. An example of a muscle relaxant useful for treating fibromyalgia is cyclobenzaprine. Examples of therapies useful for treating fibromyalgia include exercise, interferon, growth hormone, hormone therapy, diet low in animal fat and high in fiber, and complementary therapies such as counseling/psychotherapy, relaxation training, progressive muscle relaxation, guided imagery, diaphragmatic breathing, biofeedback, acupuncture, and physical and massage therapy.

Acute fibromyalgia treatments intended to eliminate or reduce the severity of muscular/skeletal pain and any associated symptoms include serotonin receptor agonists, such as triptans (5-hydroxytryptophan (5-HT) agonists), for example, almotriptan, eletriptan, frovatriptan, naratriptan, rizatriptan, sumatriptan, and zolmitriptan; ergotamine-based compounds such as dihydroergotamine and ergotamine; antiemetics such as metoclopramide and prochlorperazine; and compounds that provide analgesic effects.

Other examples of drugs useful in treating fibromyalgia include acetaminophen-aspirin, caffeine, cyproheptadine, methysergide, valproic acid, NSAIDs such as diclofenac, flurbiprofen, ketaprofen, ketorolac, ibuprofen, indomethacin, meclofenamate, and naproxen sodium; opioids such as codeine, meperidine, and oxycodone; and glucocorticoids such as dexamethasone, prednisone, and methylprednisolone.

In certain embodiments, dosage forms provided by the present disclosure may be administered to a patient for treating musculoskeletal pain in combination with a therapy or another therapeutic agent known or believed to be effective in treating musculoskeletal pain. Examples of drugs useful for treating musculoskeletal pain include cyclobenzaprine, dantrolene, methocarbamol, orphenadrine, tizanidrine, metaxalone, carisoprodol, chlorphenesin, chlorzoxazone, alprazolam, bromazepam, chlordiazepoxide, clorazepate, diazepam, flunitriazepam, lorazepam, medazepam, midazolam, oxazepam, prazepam, triazolam, temazepam, and botulinum toxin. In certain embodiments, any of the drugs useful for treating neuropathic pain may be coadminstered with a prodrug of a $GABA_B$ agonist for treating musculskeletal pain.

In certain embodiments, dosage forms provided by the present disclosure may be administered to a patient for treating low back pain in combination with a therapy or another therapeutic agent known or believed to be effective in treating low back pain. Examples of drugs useful for treating low back pain include NSAIDs such as aspirin, naproxen, and ibuprofen; anticonvulsants, antidepressants such as amitriptyline and desipramine; and opioids such as codeine, oxycodone, hydrocodone, and morphine. In certain embodiments, any of the drugs useful for treating neuropathic pain may be coadminstered with a prodrug of a $GABA_B$ agonist for treating low back pain. Therapies for low back pain include the use of cold and hot compresses, bed rest, exercise, spinal manipulation, acupuncture, biofeedback, interventional therapy, traction, transcutaneous electrical nerve stimulation, ultrasound, vertebroplasty, kyphoplasty, discectomy, foraminotomy, intradiscal electrothermal therapy, nucleoplasty, radiofrequency lesioning, spinal fusion, and spinal laminectomy.

In certain embodiments, dosage forms provided by the present disclosure may be administered to a patient for treating low back pain in combination with a therapy or other therapeutic agent for treating muscle spasms, for example muscle spasms associated with low back pain, such as muscle relaxants. Examples of drugs useful as muscle relaxants for treating muscle spasms include baclofen, carisoprodol, chlorzoxazone, cyclobenzaprine, diazepam, metaxalone, methocarbamol, orphenadrine, and tizanidine.

**Examples**

The following examples describe in detail preparation of oral sustained release dosage forms comprising compound (**1**), methods of evaluating the properties of such dosage forms, and methods of using such dosage forms. It will be apparent to those skilled in the art that many modifications, both to materials and methods, may be practiced.

In the examples below, the following abbreviations have the following meanings. If an abbreviation is not defined, the generally accepted meaning applies.

| | |
|---|---|
| g = | gram |
| h = | hour |
| kg = | kilogram |
| kp = | kilopond |
| L = | liter |
| LC/MS = | liquid chromatography/mass spectroscopy |
| mg = | milligram |
| min = | minute |
| mL = | milliliter |
| mm = | millimeter |
| mM = | millimolar |
| nm = | nanometer |
| rpm = | revolutions per minute |
| μL = | microliter |
| w% = | weight percent |

**Reference Example 1**

**Controlled Release Capsules**

Controlled release (CR) particles comprising *R*-baclofen prodrug (**1**) were prepared by coating cores comprising *R*-baclofen prodrug (**1**) with a pH independent release coating. 20/25 mesh sugar spheres (sugar spheres NF, Paulaur, Cranbury, NJ) were added to a fluid-bed coater bowl and heated to 29-31 °C. A coating formulation was prepared by dissolving *R*-baclofen prodrug (1) and binder (Plasdone® K29/32 Povidone, USP/NF, ISP Corporation) in 409 g of a 50: 50 mixture of isopropyl alcohol and acetone. The coating formulation was sprayed onto the sugar spheres while maintaining the outlet temperature at 29-31 °C to form the cores. The amounts of the components forming the cores are provided in Table 1.

Table 1: Composition of cores.

| Component | Amount/Capsule (mg) | %Composition (w/w) | Ingredient category |
|---|---|---|---|
| Compound (**1**) | 2.00 | 2.64 | Drug substance |
| 20/25 Sugar sphere, NF | 73.65 | 97.21 | Inert core |
| Plasdone® K29/32 Povidone USP/NF | 0.11 | 0.15 | Binder |
| Isopropyl alcohol, USP | - | - | Solvent |
| Acetone, NF | - | - | Solvent |
| Total | 75.76 | 100.00 | - |

Controlled release particles were prepared by coating the immediate release cores with a pH independent release polymer. The cores were spray coated with a mixture comprising which 9.7 g ammonioalkyl methacrylic acid copolymer type A (Eudragit® RL 100, Rohm Pharma) and 0.3 g glyceryl monostearate were dissolved in 125 g of a 60: 40 mixture of isopropyl alcohol and acetone.

The controlled release particles, coated with the pH independent release polymer, were then loaded into size #0 hydroxypropylmethyl cellulose (HPMC) capsules in a quantity to provide 10 mg of compound (1), equivalent to 5.35 mg of *R*-baclofen. The CR particles were then loaded into HPMC capsules. The relative amounts of the components forming controlled release capsules are provided in **Table 2**.

Table 2: Composition of controlled-release capsules.

| | Amount/Capsule (mg) | %Composition (w/w) | Ingredient category |
|---|---|---|---|
| Cores / Compound (**1**) | 353.16 | 82.64 | Drug substance coated beads |
| Ammonioalkyl Methacrylate Copolymer Type A (Eudragit® RL 100), USP, NF | 71.97 | 16.84 | pH-independent release controlling polymer |
| Glyceryl Monostearate, USP, NF | 2.22 | 0.52 | Antistatic Agent |
| Size #0 white, opaque HPMC capsule shell | - | - | Capsule Shell |
| Total | 427.35 | 100.00 | - |

**Reference Example 2**

**Sustained Release Tablet Dosage Form (SR1)**

Sustained release tablets comprising compound (1) and poly(ethylene) oxide were prepared according to the following procedure based on a 150 g batch size.

Compound (**1**) (6 g), poly(ethylene)oxide (Polyox® WSR-N750, Dow Chemical Co.) (60 g), and microcrystalline cellulose (Avicel® PH200, FMC Biopolymer) (53.25 g), hydroxypropylmethyl cellulose (Methocel®-E4M, Dow Chemical Co.) (30 g) were weighed, screened through a #20 mesh stainless steel screen, and mixed in a V-blender (2 quart, Model MB-1, Globe Pharma, New Brunswick, NJ) for 5 minutes.

The blend was discharged and wet granulated at high shear using a KG-5 Mixer/Granulator with a 1L bowl

(Key International, Englishtown, NJ). Wet granulation was performed using 15 mL of water, a tubing dimension of 1 mm, an impeller speed of 250 rpm, and a chopper speed of 1500 rpm.

The granulate was wet milled and then dried in a Fluid Bed Model 0002 (Fluid Air, Aurora, IL) granulator/drier using an inlet from of 25 SCFM, an inlet air temperature of 45°C, an outlet air temperature less than 30 °C, a filter pressure of 200-900 mm $H_2O$. The target weight loss on drying was less than about 3%.

The dried product was passed through a Comil Model U5 mill (Quadro Engineering, Inc., Millburn, NJ) using a 0.079-inch Grater Type screen (ID No. 7L079G03123-(2007)0503) and a stainless steel, 150 grit (Ra 1.06) surface finish, at an operating speed of 2500 rpm to obtain the milled material for further compression.

Magnesium stearate (0.75 g) was weighed and passed through a #40 mesh screen. The milled material and the magnesium stearate were added to the V-blender and blended for 5 minutes at 25 rpm.

The blended material was discharged and compressed to form tablets having a total weight of 250 mg and a compound (1) loading of 10 mg (4 wt%). A 10 station, Mini Press-IIBD (Globe Pharma, New Brunswick, NJ) fitted with 5/16-in diameter standard concentric upper and lower punches and a 5/16-inch (ID) × 1.1875 OD straight bore steel die was used to compress the tablets. The tablets had a mean final hardness from about 6 kp to about 9 kp (59 to 88 Newtons).

Tablets prepared according to the above procedure exhibited an average hardness of 7.1 ± 0.7 kp, a bulk density of 0.321 g/mL, a tap density of 0.422 g/mL, and a compressibility index of 24%.

The amount of the components in sustained release tablets (SAR1) comprising 10 mg *R*-baclofen prodrug and poly(ethylene) oxide is provided in Table 3.

Table 3. Composition of SR1 sustained release tablets.

| Ingredient | Source | Amount (mg/ tablet) | Composition (wt%) | Ingredient Category |
|---|---|---|---|---|
| Compound (1) | XenoPort (Santa Clara, CA) | 10.0 | 4.0 | Prodrug |
| Poly(ethylene) oxide | Union Carbide (Danbury, CT) | 100.0 | 40.0 | pH independent release control polymer |
| Microcrystalline cellulose PA) | FMC Corp. (Philadelphia, | 88.8 | 35.5 | Matrix material |
| Hydroxypropylmethyl cellulose | Dow Chemical | 50.0 | 20.0 | Binding agent |
| Magnesium stearate, NF | Mallinckrodt (phillipsburg, NJ) | 1.2 | 0.5 | Lubricant |
| Total | | 250.0 | 100 | |

**Reference Example 3**

**Sustained Release Tablet Dosage Form (SR2)**

Sustained release tablets comprising compound (**1**) and polyvinyl acetate phthalate (SR2) were prepared according to the following procedure based on a 150 g batch size.

Compound (**1**) (6 g), microcrystalline cellulose (Avicel® PH200, FMC Biopolymer) (75.75 g), and hydroxypropylmethyl cellulose (Methocel® E4M, Dow Chemical Co.) (37.5 g) were weighed, screened through a #20 mesh stainless steel screen, and mixed in a V-blender (2 quart, Model MB-1, Globepharma, New Brunswick, NJ) for 5 minutes.

The blend was discharged and wet granulated at high shear using a KG-5 Mixer/Granulator with a 1 L bowl (Key International, Englishtown, NJ). Wet granulation was performed using 100 mL of water, a tubing dimension of 1 mm, an impeller speed of 250 rpm, and a chopper speed of 1500 rpm.

The wet granulate was then dried in a Fluid Bed Model 0002 (Fluid Air, Aurora, IL) granulator/drier using an inlet from of 25 SCFM, an inlet air temperature of 45 °C, an outlet air temperature of less than 30 °C, and a filter pressure of 200-900 mm $H_2O$. The target weight loss on drying was less than about 3%.

The dried product was passed through a Comil Model U5 mill (Quadro Engineering, Inc., Millburn, NJ) using a 0.079 inch Grater Type screen (ID No. 7L079G03123-(2007)0503) and a stainless steel, 150 grit (Ra 1.06) surface finish, at an operating speed of 2500 rpm to obtain the milled material for further compression.

The granulate was returned to the KG-5 Mixer/Granulator and coated with a blend comprising polyvinyl acetate

phthalate and excipients by adding an aqueous solution of polyvinyl acetate phthalate (Sureteric®, Colorcon, West Point, PA) (30 g) at 2.4 mL/min while mixing at an impeller speed of 250 rpm and a chopper speed of 1500 rpm.

Magnesium stearate (10.75 g) (Hyqual® vegetable source) was weighed and passed through a #40 mesh screen. The milled material and the magnesium stearate were added to the V-blender and blended for 5 minutes at 25 rpm.

The blended material was discharged and compressed to form tablets having a total weight of 250 mg and a compound (1) loading of 10 mg (4 wt%). A 10 station, Mini Press-IIBD (Globepharma, New Brunswick, NJ) fitted with 5/16-in diameter IPT standard concentric upper and lower punches and a 5/16-in (ID) $\times$ 1.18875 OD straight bore steel die was used to compress the tablets. The tablets had a mean final hardness of from about 6 kp to about 9 kp (59 to 88 Newtons).

Tablets prepared according to the above procedure exhibited an average hardness of 7.1 $\pm$ 0.7 kp, a bulk density of 0.321 g/mL, a tap density of 0.422 g/mL, and a compressibility index of 24%.

The amount of the components in sustained release tablets (SR2) comprising 10 mg compound (**1**) and polyvinyl acetate phthalate is provided in Table 4.

Table 4. Composition of SR2 sustained release tablets.

| Ingredient | Source | Amount (mg/ tablet) | Composition (wt%) | Ingredient Category |
|---|---|---|---|---|
| Compound (1) | XenoPort (Santa Clara, CA) | 10.00 | 4.0 | Prodrug |
| Sureteric® (polyvinyl acetate phthalate) | Colorcon (West Point, PA) | 50.00 | 20.0 | pH dependent release control polymer |
| Microcrystalline cellulose | FMC Corp. (Philadelphia, PA) | 126.25 | 50.5 | Matrix material |
| Hydroxypropylmethyl cellulose | Dow Chemical | 62.50 | 25.0 | Binding agent |
| Magnesium stearate | Mallinckrodt (Phillipsburg, NJ) | 1.25 | 0.5 | Lubricant |
| Total | | 250.00 | 100.0 | |

**Example 4**

**<u>Sustained Release Tablet Dosage Form (SR3)</u>**

Sustained release tablets comprising compound (**1**) and an ammonioalkyl methacrylate polymer (SR3) were prepared in a 300 g batch size using the procedure described in Example 3 and replacing Sureteric® with Eudragit® RL 30D, and replacing Methocel® E4M with Methocel® K4M. The following amounts of the components were used to prepare a 300 g batch: 12.0 g of compound (**1**), 128.7 g of microcrystalline cellulose (Avicel® PH200), 106.5 g of hydroxypropylmethyl cellulose (Methocel® K4M), 51.3 g of ammonioalkyl methacrylate copolymer (Eudragit® RL 30D), and 1.5 g of magnesium stearate (Hyqual® vegetable source). The amount of the components in sustained release tablets (SR3) comprising 10 mg compound (1) and an ammonioalkyl methacrylate polymer is provided in Table 5.

Table 5. Composition of SR3 sustained release tablets.

| Ingredient | Source | Amount (mg/ tablet) | Composition (wt%) | Ingredient Category |
|---|---|---|---|---|
| Compound (1) CA) | XenoPort (Santa Clara, | 10.00 | 4.0 | Prodrug |
| Eudragit® RL 30D Ammonioalkyl methacrylate copolymer | Degussa 42.75 | | 17.1 | pH-independent release control polymer, granulating fluid |
| Microcrystalline cellulose cellulose | FMC Corp. (Philadelphia, PA) | 107.25 | 42.9 | Matrix material |
| Hydroxypropylmethylcellulose | Dow Chemical | 88.75 | 35.5 | Binding agent |

(continued)

| Ingredient | Source | Amount (mg/ tablet) | Composition (wt%) | Ingredient Category |
|---|---|---|---|---|
| Magnesium stearate | Mallinckrodt (Phillipsburg, NJ) | 1.25 | 0.5 | Lubricant |
| Total | | 250.00 | 100.0 | |

**Example 5**

***In Vitro* Dissolution Profiles for Dosage Forms**

*In vitro* dissolution profiles for the dosage forms prepared according to Examples 1-4 were determined according to USP Method 2 (Type II, paddle method) using a Model Evolution 4300-7 Vessel USP II bath (Distek Inc., New Brunswick, NJ). Dosage forms were placed into a dissolution vessel containing 500 mL of 10 mM monobasic potassium phosphate buffer ($KH_2PO_4$) at pH 7.4, 37 °C. The dissolution medium was agitated at 75 rpm (USP, Type II). Samples were withdrawn at intervals up to about 20 hours and the content of compound (**1**) in solution was determined by reverse phase HPLC using a C 18 column and a phosphate buffer/acetonitrile/water isocratic mobile phase with photodiode detection at 210 nm. An *in vitro* dissolution profile for controlled release capsules prepared according to Example 1 is shown in **FIG. 1.** *In vitro* dissolution profiles for sustained release tablet dosage forms prepared according to Examples 2-4 are shown in **FIGS. 2-4,** respectively, and in Table 6.

Table 6. Dissolution profiles for sustained release tablet dosage forms.

| Time (h) | Dosage Form SR1 Amount Dissolved (%) | Dosage Form SR2 Amount Dissolved (%) | Dosage Form SR3 Amount Dissolved (%) |
|---|---|---|---|
| 0.5 | 7 (5.1)§ | 89 (1.9) | 4 (23.0) |
| 1 | 12 (3.6) | 96 (2.8) | 6 (20.2) |
| 2 | 22 (4.3) | 97 (1.9) | 9 (16.5) |
| 4 | 41 (4.1) | 98(1.1) | 14(11.1) |
| 6 | 57 (3.7) | 99 (1.0) | 18 (9.1) |
| 8 | 69 (4.4) | 99(1.1) | 22 (8.3) |
| 12 | 86(1.5) | 99(1.0) | 31 (9.5) |
| 24 | 93 (2.3) | 100 (1.2) | 43 (12.6) |

§ The numbers represent the mean and in parentheses, the %SD.

**Example 6**

**Pharmacokinetics of *R*-Baclofen in Dogs**

Dosage forms comprising compound (1) were administered by oral gavage to groups of four adult male Beagle dogs (weight approx 8 kg) at a dose of 10 mg compound (**1**). The dogs were fasted overnight before the study and for 4 hours post-dosing. Blood samples (1 mL) were obtained via the femoral vein at intervals over 24 hours after oral dosing. Blood was quenched immediately using acetonitrile with 1 % formic acid and then frozen at -20 °C until analyzed.

The concentration of *R*-baclofen in quenched whole blood was determined using an API 2000 LC/MS/MS instrument equipped with a Shimadzu binary pump and a Leap CTC autosampler. A Phenomenex Hydro-RP 4.6 × 50 mm column operating at room temperature was used. The mobile phases were (A) water with 0.1 % formic acid, and (B) acetonitrile with 0.1 % formic acid. The gradient condition was: 5% B for 0.5 min, then to 95% B in 1.8 min; then maintained at 95% B for 1.2 min. The mobile phase was then returned to 5% B for 2 min. A TurbolonSpray source was used on the API 2000. The analysis was done in positive ion mode and an MRM transition of m/z 214/151 was used in the analysis of *R*-baclofen. Ten (10) μL of the blood sample was injected. The peaks were integrated using Analyst™ Software (Agilent Technologies) to provide the concentration of *R*-baclofen in the blood sample.

Pharmacokinetic profiles of sustained release oral dosage forms prepared according to Examples 1-4 at a dose of 10 mg compound (**1**) following oral administration to dogs is shown in **FIG. 5**. The corresponding pharmacokinetic parameters are provided in Table 7. The bioavailability for each formulation is determined relative to 10 mg-eq/kg *R*-baclofen administered intravenously ($AUC_{inf}$ = 1.98 μg·h/mL).

Table 7. Pharmacokinetics of *R*-baclofen following oral administration of sustained release oral dosage forms to fasted dogs at a dose of 10 mg compound (**1**).

| Formulation | $C_{max}$ (ng/mL) | $T_{max}$ (h) | $C_{12h}$ (ng/mL) | $C_{max}/C_{12h}$ | $T_{1/2}$ (h) | $AUC_{(0-Tast)}$ (ng·h/mL) | $AUC_{(0-inf)}$ (ng·h/mL) | $\%F_{po}$ |
|---|---|---|---|---|---|---|---|---|
| **CR** | 41 (9) | 5.0 (2.6) | 15 | 2.7 | 4.5 (0.5) | 340 (50) | 408 (20) | 42 (8) |
| **SR1** | 119 (26) | 3.0 (2.0) | 23 | 5.2 | 3.6 (1.0) | 836 (157) | 882 (194) | 91(6) |
| **SR1** | 100 (2) | 3.5 (1.0) | 19 | 5.3 | 4.6(1.7) | 760 (115) | 852 (159) | 90(11) |
| **SR2** | (116) | 4.3 (2.9) | 36 | 4.6 | 4.1 (0.6) | 1080 (155) | 1110 (155) | 100 (0) |
| **SR3** | 48 (26) | 4.0 (2.8) | 14 | 3.4 | 8.1 (4.4) | 359 (126) | 483 (168) | 55 (24) |
| **SR3** | 29 (5) | 8.5 (7.9) | 13 | 2.2 | 7.4 (0.2) | 360 (153) | 554 (136) | 42 (15) |

**Reference Example 7**

**Pharmacokinetics of *R*-Baclofen in Human Patients Following Administration of CR Capsules Comprising Compound (1)**

The pharmacokinetics of *R*-baclofen in healthy human patients following oral administration of CR capsules comprising compound (**1**) was determined.

Fasted human patients were randomized to receive single oral doses of CR capsules or matching placebo in a double-blind fashion. The study investigated 6 dose levels of compound (**1**), 10, 20, 30, 40, 60, and 80 mg, in capsules with each capsule comprising controlled release particles and comprising 10 mg compound (**1**). Six (6) groups of 10 subjects each were enrolled sequentially (10 subjects per dose level). Eight subjects in each dose group received CR capsules and two received placebo.

Blood samples were collected from patients prior to dosing and at 0.5, 1, 1.5, 2, 3, 4, 5, 6, 8, 10, 12, 14, 18, 24, 30, and 36 hours post-dosing for all treatments. Blood sample aliquots were quenched immediately with methanol to prevent further hydrolysis of compound (**1**). Blood sample aliquots were stored in a freezer at -70 °C. The blood sample aliquots were analyzed for *R*-baclofen and compound (**1**) in whole blood supernatant using sensitive and specific LC-MS/MS methods.

Concentration data for *R*-baclofen and compound (**1**) in blood were analyzed by noncompartmental methods using WinNonlin™ Software version 4.1 (Pharsight Corporation, Mountain View, CA). Concentration data and pharmacokinetics parameters were plotted using SigmaPlot™ version 9.0 (Systat Software Inc., Point Richmond, CA). Actual time points were used for the calculation of pharmacokinetic parameters. The maximum concentration ($C_{max}$) and time to $C_{max}$ ($T_{max}$) were obtained by observation. The apparent elimination half-life ($T_{1/2}$) was determined by linear regression of three or more log-transformed data points in the terminal phase. The area under the concentration versus time curve (AUC) was determined by the linear trapezoidal method using concentration data over the dosing interval. The AUC value extrapolated to infinity ($AUC_{inf}$) was calculated as:

$$AUC_{inf} = AUC_{(0\text{-}tlast)} + C_{last}/\lambda_z$$

where $t_{last}$ is the time of the last quantifiable concentration ($C_{last}$) and $\lambda_z$ is the rate constant of the apparent terminal elimination phase. Using the data from doses 10, 20, 30, 40, 60, and 80 mg, linear regression models were fit for $AUC_{inf}$ versus dose and for $C_{max}$ versus dose using SAS™ version 9.1 for Windows (SAS Institute, Cary, NC). In both models, the dose effect was parameterized using orthogonal polynomial coefficients for unequally spaced values.

The blood concentration and pharmacokinetic parameters for *R*-baclofen and *R*-baclofen prodrug (**1**) following oral administration of CR capsules to healthy human patients is shown in **Figures 6-9**, and a summary of the pharmacokinetic parameters for different doses of *R*-baclofen prodrug (**1**) is provided in Table 8.

Table 8. Pharmacokinetic parameters of *R*-baclofen following oral administration of CR capsules at doses of 10 mg (1 10 mg), 20 mg (2 10 mg), 30 mg (3 10 mg) , 40 mg (4 10 mg), 60 mg (6 10 mg), and 80 mg (8 10 mg) compound (**1**) to fasted dogs.

| Dose Compound (1) (mg) | C $C_{max}$ (ng/mL) | $C_{max}$/dose (ng/mL·mg) | $C_{max}/C_{12}$ | $T_{max}$ (h) | $T_{1/2}$ (h) | $AUC_{inf}$ (ng·h/mL) | $AUC_{inf}$/ dose (ng·h/mL · mg) | $\%F_{rel}$ |
|---|---|---|---|---|---|---|---|---|
| **10** | 23 (10) | 2.27 | 2.6 | (3.8) | 10.3 (3.6) | 243 (66) | 24 | 31 (7) |
| **20** | 35 (17) | 1.74 | 2.7 | (1.1) | 9.6 (1.7) | 338 (83) | 17 | 33 (10) |
| **30** | 63 (19) | 2.09 | 2.3 | (0.9) | 9.3 (2.7) | 810 (169) | 27 | 33 (7) |
| **40** | 82 (49) | 2.06 | 2.2 | (4.5) | 11.3 (4.7) | 1020 (300) | 26 | 28 (9) |
| **60** | 139 (56) | 2.31 | 2.3 | (3.9) | 10.5 (2.6) | 1540 (603) | 26 | 35 (6) |
| **80** | 193 (89) | 2.41 | 2.8 | (4.0) | 9.7 (1.0) | 2020 (787) | 26 | 34 (18) |

The pharmacokinetic parameters for *R*-baclofen following oral administration of CR capsules comprising 10 mg compound (**1**) to dogs and humans is compared in Table 9.

Table 9. Comparison of pharmacokineticparameters of *R*-baclofen following oral administration of CR capsules comprising 10 mg compound (**1**) to dogs and humans.

| Patient | $C_{max}$ (ng/mL) | $C_{max}/C_{12}$ | $T_{max}$ (h) | $T_{1/2}$ (h) | $AUC_{inf}$ (ng·h/mL) | F (%) |
|---|---|---|---|---|---|---|
| **dog** | 41 (9) | 2.2 (-) | 5.0 (2.6) | 4.5 (0.5) | 408 (20) | 42 (8) |
| **human** | 23 (10) | 2.6 (-) | 5.0 (3.8) | 10.3 (3.6) | 243 (66) | 31 (7) |

**Example 8**

**<u>Pharmacokinetics of *R*-Baclofen in Human Patients Following Administration of Tablet Dosage Forms Comprising Compound (1) to Human Patients</u>**

The pharmacokinetics of *R*-baclofen in healthy human patients following oral administration of 20 mg dose of compound (**1**) as two tablet dosage forms comprising 10 mg compound (**1**) was determined.

Fed or fasted human patients were randomized to receive single oral doses of 20 mg compound (**1**) as two SR tablets comprising 10 mg compound (**1**).

Blood samples (approximately 4 mL) were collected from patients prior to dosing and at time intervals post-dosing into tubes containing $K_2EDTA$. Blood sample aliquots were quenched immediately with methanol to prevent further hydrolysis of compound (**1**). Two aliquots (1 mL each) were immediately transferred to Nalgene tubes and quenched with 3 mL methanol. Blood sample aliquots were stored in a freezer at -80 °C. The blood sample aliquots were analyzed for R-baclofen and compound (**1**) in whole blood supernatant using sensitive and specific LC-MS/MS methods.

Concentration data for R-baclofen and compound (**1**) in blood was analyzed by noncompartmental methods using WinNonlin™ Software version 4.1 (Pharsight Corporation, Mountain View, CA). Concentration data and pharmacokinetics parameters were plotted using SigmaPlot™ version 9.0 (Systat Software Inc., Point Richmond, CA). Actual time points were used for the calculation of pharmacokinetic parameters. The maximum observed drug concentration ($C_{max}$) and time to $C_{max}$ ($T_{max}$) were obtained by observation. The apparent elimination half-life ($T_{1/2}$) was determined by linear regression of three or more log-transformed data points in the terminal phase (calculated as $\ln(2)/K_{el}$ where $K_{el}$ is the terminal elimination rate constant calculated by linear regression of the terminal linear portion of the log concentration vs. time curve). The area under the linear regression models were fit for $AUC_{inf}$ versus dose and for $C_{max}$ versus dose using SAS™ version 9.1 for Windows (SAS Institute, Cary, NC). In both models, the dose effect was parameterized using orthogonal polynomial coefficients for unequally spaced values.

The blood concentration and pharmacokinetic parameters for *R*-baclofen following oral administration of a 20 mg dose of compound (1) as two tablet dosage forms comprising 10 mg compound (**1**) to fed and fasted healthy human

patients is shown in Figures 10 and 11, and a summary of the pharmacokinetic parameters is provided in Tables 10-11. In the tables, AUC is the area under the drug concentration-time curve calculated using linear trapezoidal summation form time zero to time t, where t is the time of the last measurable concentration ($C_t$); AUC($_{0\text{-inf}}$) is the area under the drug concentration-time curve from time zero to infinity, $AUC_{(0\text{-inf})} = AUC_{(0\text{-t})} + C_{t/kel}$.; and $\%F_{rel}$ is the percent bioavailability relative to R-baclofen $AUC_{0\text{-}24}$ of 279 ng·h/mL after oral dosing of 20 mg compound (1) as controlled release capsule dosage forms (2 10 mg) (Example 1) to fasted healthy patients.

Table 10. Mean (SD) pharmacokinetic data for *R*-baclofen in blood following oral administration of 20 mg compound (**1**) as sustained release tablet formulations (2 10 mg) to fasted healthy human patients (n=9,10).

| Formulation | $C_{max}$ (ng/mL) | $T_{max}$ (h) | $C_{12\,h}$ (ng/mL) | $C_{max}/C_{12h}$ | $T_{1/2}$ (h) | $AUC_{(0\text{-tlast})}$ (ng·h/mL) | $AUC_{(0\text{-inf})}$ (ng·h/mL) | $\%F_{rel}$ |
|---|---|---|---|---|---|---|---|---|
| **SR1** | 105 (43) | 3.3 (1.1) | 24 (7) | 4.6 (1.6) | 4.7 (1.0) | 802 (166) | 825 (164) | 284 (60) |
| **SR2** | 82 (35) | 6.8 (1.4) | 31 (12) | 2.9 (1.4) | 5.6 (1.5) | 705 (225) | 729 (225) | 253 (96) |
| **SR3** | 33 (12) | 4.1 (2.3) | 17 (9) | 2.1 (0.8) | 7.7 (4.1) | 499 (134) | 546 (124) | 150 (48) |

Table 11. Mean (SD) pharmacokinetics data for *R*-baclofen in blood following oral administration of 20 mg compound (**1**) as sustained release tablet formulations (2 10 mg) to fed healthy human patients (n=10).

| Formulation | $C_{max}$ (ng/mL) | $T_{max}$ (h) | $C_{12\,h}$ (ng/mL) | $C_{max}/C_{12\,h}$ | $T_{1/2}$ (h) | $AUC_{(0\text{-tlast})}$ (ng·h/mL) | $AUC_{(0\text{-inf})}$ (ng·h/mL) | $\%F_{rel}$ |
|---|---|---|---|---|---|---|---|---|
| **SR1** | 158 (38) | 5.0 (1.6) | 31 (15) | 5.9 (2.4) | 5.2 (0.9) | 919 (16.1) | 941 (161) | 323 (56) |
| **SR2** | 183 (64) | 7.3 (2.0) | 51 (25) | 4.3 (2.6) | 5.5 (1.6) | 1050 (206) | 1070 (207) | 370 (50) |
| **SR3** | 66 (18) | 8.0 (2.7) | 49 (27) | 1.6 (0.7) | 6.1 (1.6) | 830 (281) | 865 (280) | 273 (94) |

The pharmacokinetic profiles of *R*-baclofen in blood following oral administration of sustained release tablet formulation SR3 to fasted dogs (10 mg compound (**1**)) and fasted humans (2 10 mg compound (**1**)) is compared in **Figure 12.**

**Example 9**

**Steady State Pharmacokinetics of *R*-Baclofen in Human Patients Following Administration of Tablet Dosage Forms Comprising Compound (1) to Human Patients**

A randomized, double-blind, placebo-controlled, ascending multiple-dose study of the steady state pharmacokinetics of the SR3 tablet formulation (Example 4) in healthy adult human patients was performed.

All doses were administered as multiples of SR3 tablets comprising 10 mg compound (**1**) or matching placebo. Patients were titrated to a target once daily dose level and maintained at the target dose for 7 days. Patients were then escalated to twice daily dosing of the same compound (**1**) dose, maintained at the new target dose for 7 days, and then tapered off the drug. In Cohort 1,11 patients receved 30 mg (3 10 mg) once daily (QD) compound (**1**) or matching placebo for 7 days followed by 30 mg twice daily (BID) for 7 days. In Cohort 2, 12 patients received 60 mg (36 10 mg) QD compound (**1**) or matching placebo for 7 days followed by 60 mg BID for 7 days. In Cohort 3,12 subjects received 90 mg (9 10 mg) QD compound (**1**) or matching placebo for 7 days flowed by 90 mg BID for 7 days. In Cohort 4, subjects received 120 mg (12 10 mg) QD for 7 days. All treatments were administered with a moderate fat meal.

Blood concentrations of *R*-baclofen were measured and analyzed as described in Example 7. The steady state concentrations of *R*-baclofen in blood following repeated QD dosing of compound (**1**) as SR3 tablets are provided in Table 12 and in **Figure 16.** The steady state concentrations of *R*-baclofen in blood following repeated BID dosing of compound (**1**) as SR3 tablets are provided in Table 12 and in **Figure 14.** The correlation between the compound (**1**) dose and the $C_{max,ss}$ and $AUC_{0\text{-}24}$ for QD and BID dosing is shown in **Figures 15 and 16.** The mean (SD) steady state trough concentration, $C_{SS,\,min}$,of *R*-baclofen during QD and BID dosing of compound (**1**) as SR3 tablets is provided in **Figures 17** and **18,** respectively.

Table 12. Mean (SD) pharmacokinetic parameters for *R*-baclofen in blood determined at steady state after once (QD) or twice daily (BID) oral dosing of 30 mg (3 10 mg), 60 mg (6 10 mg), 90 mg (9 10 mg), or 120 mg (12 10 mg) compound (**1**) as SR3 tablet formulations in healthy human patients.

| Compound (1) Dose (mg) | $C_{SS, max}$ (ng/mL) | $C_{SS, min}$ (ng/mL) | $C_{SS, max}/C_{SS, min}$ | $T_{max}$ (h) | $T_{1/2}$ (h) | $AUC_{0-24}$ (ng·h/mL) |
|---|---|---|---|---|---|---|
| **30 QD** | 69 (25) | 18 (5) | 4.4 (2.3) | 5.4 (2.9) | 11.4 (4.4) | 838 (198) |
| **60 QD** | 133 (28) | 22 (7) | 6.7 (3.7) | 4.8 (2.2) | 8.1 (2.3) | 1530 (286) |
| **90 QD** | 194 (52) | 40 (17) | 5.2 (1.6) | 4.4 (1.6) | 10.7 (3.6) | 2140 (528) |
| **120 QD** | 250 (70) | 63 (26) | 4.2 (1.0) | 4.1 (0.4) | 12.1 (5.2) | 3050 (698) |
| **30 BID** | 132 (46) | 56 (11) | 2.4 (0.7) | 2.2(2.2) | 9.6(4.2) | 2050 (508)* |
| **60 BID** | 222 (39) | 103 (49) | 2.5 (1.1) | 4.1 (1.8) | 8.1 (2.3) | 3500 (591)* |
| **90 BID** | 275 (35) | 143 (35) | 2.0 (0.4) | 3.9 (0.8) | 11.0 (2.0) | 4510 (887)* |

* $AUC_{0-24}$ on Day 14 after BID dosing was calculated from 2 × $AUC_{0-12}$.

**Example 10**

**Animal Model for Assessing Therapeutic Efficacy for Treating Spasticity**

The mutant spastic mouse is a homozygous mouse that carries an autosomal recessive trait of genetic spasticity characterized by a deficit of glycine receptors throughout the central nervous system (Chai et al., Proc. Soc. Exptl. Biol. Med. 1962, 109, 491). The mouse is normal at birth and subsequently develops a coarse tremor, abnormal gait, skeletal muscle rigidity, and abnormal righting reflexes at two to three weeks of age. Assessment of spasticity in the mutant spastic mouse can be performed using electrophysiological measurements or by measuring the righting reflex (any righting reflex over one second is considered abnormal), tremor (holding mice by their tails and subjectively rating tremor), and flexibility.

Models of acute spasticity including the acute decerebrate rat, the acute or chronic spinally transected rat, and the chronically spinal cord-lesioned rat (*see e.g.,* Wright and Rang, Clin Orthop Relat Res 1990, 253, 12-19; Shimizu et al., JPharmacol Sci 2004, 96, 444-449; and Li et al., J Neurophysiol 2004, 92, 2694-2703). The acute models, although valuable in elucidating the mechanisms involved in the development of spasticity, have come under criticism due to the fact that they are acute. The animals usually die or have total recovery from spasticity. The spasticity develops immediately upon intervention, unlike the spasticity that evolves in the human condition of spasticity, which most often initially manifests itself as a flaccid paralysis. Only after weeks and months does spasticity develop in humans. Some of the more chronic-lesioned on spinally transected models of spasticity do postoperatively show flaccid paralysis. At approximately four weeks post-lesion/transection, the flaccidity changes to spasticity of variable severity. Although all of these models have their own particular disadvantages and lack of true representation of the human spastic condition, they are shown useful in developing treatments for spasticity in humans. Many of these models have also made use of different species, such as cats, dogs, and primates. Baclofen, diazepam, and tizanidine, effective antispastic agents in humans, are effective on different parameters of electrophysiologic assessment of muscle tone in these models.

The Irwin Test is used to detect physiological, behavioral, and toxic effects of a test substance, and indicates a range of dosages that can be used for later experiments (Irwin, Psychopharmacologia 1968, 13, 222-57). Typically, rats (three per group) are administered the test substance and are then observed in comparison with a control group given vehicle. Behavioral modifications, symptoms of neurotoxicity, pupil diameter, and rectal temperature are recorded according to a standardized observation grid derived from that of Irwin. The grid contains the following items: mortality, sedation, excitation, aggressiveness, Straub tail; writhes, convulsions, tremor, exophthalmos, salivation, lacrimation, piloerection, defecation, fear, traction, reactivity to touch, loss of righting reflexes, sleep, motor incoordination, muscle tone, stereotypes, head-weaving, catalepsy, grasping, ptosis, respiration, corneal reflex, analgesia, abnormal gait, forepaw treading, loss of balance, head twitches, rectal temperature, and pupil diameter. Observations are performed at 15, 30, 60, 120, and 180 minutes following administration of a test compound, and also 24 hours later.

In the Rotarod Test (Dunham et al., J Am. Pharm. Assoc. 1957, 46, 208-09) rats or mice are placed on a rod rotating at a speed of eight turns per minute. The number of animals that drop from the rod before three minutes is counted and the drop-off times are recorded (maximum: 180 sec). Diazepam, a benzodiazepine, can be administered at 8 mg/kg, i.p., as a reference substance.

**Example 11**

**Animal Model of Gastroesophageal Reflux Disease**

A method described by Stakeberg and Lehmann, *Neurogastroenterol. Mot.* 1999, *11*, 125-132 can be used to assess the efficacy for treating gastroesophageal reflux disease associated with transient lower esophageal sphincter relaxation. Dogs are equipped with an esophagostomy. After recovery, the dogs are intubated with a water-perfused multi-lumen Dentsleeve assembly to record pressure of the esophagus, lower esophageal sphincter and stomach. A pH catheter is placed adjacent the manometer assembly to measure reflux episodes. A thin air-perfused catheter is placed retrogradely in the hypopharynx to measure swallows. Only pharyngeal contractions follwed by a peristaltic wave are included in the analysis. TLOSRs are stimulated by infusion of an acidified nutritious soup followed by insufflation of air. Test compoundis administered before infusion of soup.

**Example 12**

**Animal Model of Emesis**

Pica, reduced food intake, delayed gastric emptying, and the presence of gas in the stomach are considered behavioral correlates of emesis in the rat, which lacks the vomiting reflex (see e.g., Malik et al., Eur JPharmacology 2007, 555, 164-173).

Male rats are housed in cages for a 3 day habituation period before injection with ddrugs and rema in the same cages for the subsequent 2 days of the study. Food, water, and kaolin are provided *ad libitum.* Body weight, food and water intake, and kaolin are monitored daily throughout the study.

Gastric contents and the presence of gas is determined by killing the rats at the end of the study and removing the stomach. The stomach was immediately dropped into 100 mL of 150 mM NaCl and the presence of gas determined by whether the stomach sinks or rises to the surface. The stomach contents are then removed and the wet weight measured.

The animals are adapted for 3 days prior to evaluation of cisplatin-induced emesis. On day 1, rats are given an anti-emetic drug or vehicle and 1 h later a single intraperitoneal dose of cisplatin (6 mg/kg i.p.) or vehicle. Animals may receive further doses of an anti-emetic drug as appropriate and continued on subsequent days as appropriate. Cisplatin reduces food intake, increases kaolin consumption (pica), increases the weight of gastic contents, incresaes the presence of gas in the stomach, and reduces locomotor activity. Compounds that reduce these cisplatin-induced effects amay be useful in treating emesis, and in particular, emesis induced by chemotherapeutic agents.

**Example 13**

**Efficacy for Treating Cough**

Male guinea pigs are individually placed into a sealed perspex exposure chamber and allowed to acclimatize prior to administration of tussive stimuli or test compound by aerosol. Cough responses are induced by exposure to an aerosol of either citric acid (20%, 10 min) or capsaicin (15 μM, 4 min) at flow rates of 2 L/min and 3 L/min, respectively. An observer continuously monitors the animals, and the number of coughs counted over a 15 min period from commencement of the aerosol administration of the tussive stimuli. Guinea pigs are then randomly allocated to receive either test compound or control, and exposure to the tussive stimuli repeated and the number of coughs recorded.

Healthy, nonsmoking subjects who do not experience symptoms of respiratory tract infection or seasonal allergy for at least 4 weeks prior to evaluation and who demonstrate normal pulmonary function are enrolled. Subjects inhale single breaths of capsaicin solution (ranging from 0.98 μmol/L to 1,000 μmol/L) from a compressed-air driven nebulizer controlled by a dosimeter. Single breaths of capsaicin solution are given in ascending order, with inhalations of saline solution randomly interspersed to increase challenge blindness, until the concentration inducing five or more coughs is reached. Breaths are delivered at 1-min intervals. The number of coughs in response to each concentration of capsaicin during the 1-min period immediately after each inhalation is recorded by a blinded observer. Subjects are unaware that the end point of the study is the number of coughs induced. After undergoing baseline capsaicin cough challenge, subjects are randomly assigned, in a double-blind manner, and administered a test compound at an appropriate dose or placebo, after which the cough challenge is repeated. A significant response can be defined as a fourfold or greater increment in the capsaicin concentration required to elicit five or more coughs.

**Example 14**

**Use of Animal Models to Assess the Efficacy of Compounds for Treating Neuropathic Pain**

*Inflammatory Pain - Formalin test*

Fifty μL of a 5% formalin solution is injected subcutaneously into the dorsal aspect of the right hind paw and the rats are then individually placed into clear observation cages. Rats are observed for a continuous period of 60 min or for periods of time corresponding to phase I (from 0 to 10 min following formalin injection) and phase II (from 30 to 50 min following formalin injection) of the formalin test (Abbott et al., Pain 1995, 60, 91-102*)*. The number of flinching behaviors of the injected paw is recorded using a sampling technique in which each animal is observed for one 60-sec period during each 5-min interval. Test compound is administered 30 min or other appropriate interval prior to formalin injection.

*Inflammatory Pain - Carrageenan-induced acute thermal hyperalgesia and edema*

Paw edema and acute thermal hyperalgesia are induced by injecting 100 μL of a 1% solution of λ-carrageenan in physiological saline into the plantar surface of the right hind paw of rats. Thermal hyperalgesia is determined 2 h following carrageenan injection using a thermal paw stimulafor as described by Hargreaves et al., Pain 1988, 32, 77-88. Rats are placed into plastic cubicles mounted on a glass surface maintained at 30 °C and a thermal stimulus in the form of radiant heat emitted from a focused projection bulb is then applied to the plantar surface of each hind paw. The maximum time of exposure is set to limit possible tissue damage. The elapsed time until a brisk withdrawal of the hind paw from the thermal stimulus is automatically recorded using photodiode motion sensors. The right and left hind paw of each rat is tested in three sequential trials at about 5-min intervals. Carrageenan-induced thermal hyperalgesia of paw withdrawal latency ($PWL_{thermal}$) is calculated as the mean of the two shortest latencies. Test compound is administered before assessment of thermal hyperalgesia.

The volume of paw edema is measured using water displacement with a plethysmometer 2 h following carrageenan injection by submerging the paw up to the ankle hairline (approx. 1.5 cm). The displacement of the volume is measured by a transducer and recorded. Test compound is administered at an appropriate time following carrageenan injection, such as for example, 30 min or 90 min.

*Visceral Pain*

Thirty min following administration of test compound, mice receive an injection of 0.6% acetic acid in sterile water (10 mL/kg, i.p.) as described by Mogil et al., Pain 1999, 80, 67-82. Mice are then placed in table-top Plexiglass observation cylinders (60 cm high × 40 cm diameter) and the number of constrictions/writhes (a wave of mild constriction and elongation passing caudally along the abdominal wall, accompanied by a slight twisting of the trunk and followed by bilateral extension of the hind limbs) is recorded during the 5-20 min following acetic acid injection for a continuous observation period of 15 min.

*Neuropathic Pain - Spinal nerve ligation*

Rats receive unilateral ligation of the lumbar 5 (L5) and lumbar 6 (L6) spinal nerves as described by Kim and Chung, Pain 1992, 50, 355-363. The left L5 and L6 spinal nerves of the rat are isolated adjacent to the vertebral column and tightly ligated with a 5-0 silk suture distal to the dorsal root ganglia, and care is taken to avoid injury of the lumbar 4 (L4) spinal nerve. Control rats undergo the same procedure but without nerve ligation. All animals are allowed to recover for at least 1 week and not more than 3 weeks prior to assessment of mechanical allodynia. Mechanical allodynia is measured using calibrated von Frey filaments as described by Chaplan et al., J Neurosci Methods 1994; 53, 55-63. Rats are placed into inverted plastic containers (20 cm × 12.5 cm x 20 cm) on top of a suspended wire mesh grid and acclimated to the test chamber for 20 min. The von Frey filaments are presented perpendicularly to the plantar surface of the selected hind paw and then held in this position for approximately 8 s with sufficient force to cause a slight bend in the filament. Positive responses include an abrupt withdrawal of the hind paw from the stimulus or flinching behavior immediately following removal of the stimulus. A 50% paw withdrawal threshold (PWT) is determined using a procedure described by Dixon, Rev Pharmacol Toxicol 1980, 20, 441-462. Rats with a PWT ≤ 5.0 g are considered allodynic and utilized to test the analgesic activity of a test compound. The test compound is administered 30 min or other appropriate interval prior to the assessment of mechanical allodynia.

*Neuropathic Pain - Chronic constriction injury of the sciatic nerve*

A model of chronic constriction injury of the sciatic nerve-induced neuropathic pain according to the method of Bennett and Xie, Pain 1988, 33, 87-107, is used. The right common sciatic nerve is isolated at mid-thigh level and loosely ligated by four chromic gut (4-0) ties separated by an interval of 1 mm. Control rats undergo the same procedure but without sciatic nerve constriction. All animals are allowed to recover for at least 2 weeks and for no more than 5 weeks prior to testing of mechanical allodynia. Allodynic PWT is assessed in the animals as described for animals with spinal nerve ligation. Only rats with a PWT ≤ 5.0 g are considered allodynic and utilized to evaluate the analgesic activity of a test compound. Test compound is administered 30 min or other appropriate time prior to the assessment of mechanical allodynia.

*Neuropathic Pain - Vincristine-induced mechanical allodynia*

A model of chemotherapy-induced neuropathic pain is produced by continuous intravenous vincristine infusion (Nozaki-Taguchi et al., Pain 2001, 93, 69-76). Anesthetized rats undergo a surgical procedure in which the jugular vein is catheterized and a vincristine-primed pump is implanted subcutaneously. Fourteen days of intravenous infusion of vincristine (30 μg/kg/day) results in systemic neuropathic pain of the animal. Control animals undergo the same surgical procedure, with physiological saline infusion. PWT of the left paw is assessed in the animals 14 days post-implantation as described for the spinal nerve ligation model. Test compound is administered 30 min or other appropriate interval prior to the test for mechanical allodynia in rats with PWT≤ 5.0 g before treatment.

*Post-Operative Pain*

A model of post-operative pain is performed in rats as described by Brennan et al., Pain 1996, 64, 493-501. The plantar aspect of the left hind paw is exposed through a hole in a sterile plastic drape, and a 1-cm longitudinal incision is made through the skin and fascia, starting 0.5 cm from the proximal edge of the heel and extending towards the toes. The plantaris muscle is elevated and incised longitudinally leaving the muscle origin and insertion points intact. After hemostasis by application of gentle pressure, the incision is closed. Animals are then allowed to recover for 2 h following surgery, at which time mechanical allodynia and thermal hyperalgesia are assessed.

Effects of test compound on mechanical allodynia are assessed following administration, with PWT being examined in these animals for both the injured and non-injured paw as described for the spinal nerve ligation model with the von Frey filament systematically pointing towards the medial side of the incision. In a separate experiment, the effects of test compound on thermal hyperalgesia are assessed following administration of test compound, with $PWL_{thermal}$ being determined as described for the carrageen-induced thermal hyperalgesia model with the thermal stimulus applied to the center of the incision of the paw planter aspect.

**Example 15**

**Use of Animal Models to Assess the Efficacy of Prodrugs of $GABA_B$ Agonists for Treating Musculoskeletal Pain**

An animal model of muscle hyperalgesia described by Kehl et al., Pain 2000, 85, 333-343, can be used to assess efficacy for treating musculoskeletal pain.

Male Sprague-Dawley rats are used in the study. Animals are housed for 1 week before each experiment and weigh approximately 100-150 g when carrageenan is injected. At the start of each experiment baseline forelimb and hindlimb grip force measurements are acquired. Each animal is then briefly anesthetized and carrageenan (4 mg/ 75 μL per triceps) or PBS vehicle (75 μL) is injected into the triceps muscles bilaterally. To determine whether grip force reduction is specifically mediated by carrageenan, various doses of carrageenan or an equal volume of PBS vehicle are injected into the triceps muscles bilaterally. The forelimb and hindlimb grip force are then measured at various intervals following the injections and compared to pre-carrageenan levels.

Measurement of forelimb grip force is made using a computerized grip force meter. The apparatus measures the neuromuscular performance of rodents as displayed in their forelimb and hindlimb grip force responses. Two separate force gauges are used to measure the responses, with one gauge for measuring forelimb grip force located at the front of the apparatus, and the other gauge, that measures hindlimb grip force, located at the rear of the apparatus. During testing, each rat is held by its tail and gently passed (about 10 cm/sec) over the wire mesh grids and the grip force measured by the strain gauges. The length of time each animal applies force to the mesh grid is determined by the animal, and therefore the amplitude and duration of force exerted are subject to factors, such as hyperalgesia, influencing the behavioral performance of the animal.

To test the anatomical specificity of carrageenan-evoked grip force reduction, the force measurement apparatus

is modified to position both force transducers with attached wire mesh grids side-by-side at the front of the apparatus. Rats are held by their tails and gently passed (about 10 cm/sec) over the side-by-side wire mesh grids to obtain separate baseline forelimb grip force measurements from the right and left forelimbs simultaneously. Rats are then injected bilaterally with carrageenan (4 mg) or PBS (75 μL) into the triceps to obtain the following three treatment groups: (1) bilateral PBS (75 μL); (2) bilateral carrageenan (4 mg); and (3) PBS (75 μL) in one triceps and carrageenan (4 mg) in the contralateral triceps. The side selected for carrageenan injection is randomized and the observer is unaware of the treatment allocation. Bilateral grip force measurements are then obtained at intervals over the next 48 h and compared to baseline measurements.

To evaluate compounds for effectiveness in treating clinical muscle pain, baseline grip force measurements are first obtained. Carrageenan is then injected bilaterally and grip force measured, at a time determined in the first experiment to exhibit peak reduction in grip force. Immediately after testing, an appropriate amount of a test compound is administered. After an appropriate time, grip force is measured and compared to the baseline levels for each animal. Test compounds that inhibit carrageenan-evoked reduction in grip force may be efficacious in treating musculoskeletal pain in humans.

Finally, it should be noted that there are alternative ways of implementing the embodiments disclosed herein. Accordingly, the present embodiments are to be considered as illustrative and not restrictive.

## Claims

**1.** A sustained release oral dosage form comprising a tablet, wherein the tablet comprises:

(3*R*)-4-{[(1S)-2-methyl-1-(2-methylpropanoyloxy)propoxy]carbonylamino}-3-(4-chlorophenyl)butanoic acid or a pharmaceutically acceptable salt thereof,
ammonioalkyl methacrylate copolymer, and
hydroxypropylmethyl cellulose.

**2.** An oral dosage from according to claim 1, wherein the (3*R*)-4-{[(1*S*)-2-methyl-1-(2-methylpropanoyloxy)propoxy] carbonylamino}-3-(4-chlorophenyl)butanoic acid is present in a therapeutically effective amount.

**3.** An oral dosage from according to claim 2, wherein the therapeutically effective amount comprises form 2 mg-equivalents *R*-baclofen to 40 mg-equivalents *R*-baclofen.

**4.** An oral dosage from according to claim 2, wherein the therapeutically effective amount is less than an amount that causes moderate sedation and impairment of motor activity in a patient.

**5.** An oral dosage from according to claim 1, wherein the (3*R*)-4-{[(1*S*)-2-methyl-1-(2-methylpropanoyloxy)propoxy] carbonylamino}-3-(4-chlorophenyl)butanoic acid is present in an amount from 5 mg to 80 mg.

**6.** An oral dosage from according to claim 1, which following oral administration to a fasted human patient at a dose of (3*R*)-4-{[(1*S*)-2-methyl-1-(2-methylpropanoyloxy)propoxy]carbonylamino}-3-(4-chlorophenyl)butanoic acid ranging from 5 mg to 140 mg provides a relative oral bioavailability of (*R*)-3-amino-3-(4-chlorophenyl)butanoic acid ranging from 220% to 340%, wherein the oral bioavailability is relative to that following oral administration of an equivalent amount of (*R*)-3-amino-3-(4-chlorophenyl)butanoic acid in at least one immediate release dosage form.

**7.** An oral dosage from according to claim 1, which following oral administration to a fasted human patient at a dose of (3*R*)-4-{[(1*S*)-2-methyl-1-(2-methylpropanoyloxy)propoxy]carbonylamino}-3-(4-chlorophenyl)butanoic acid ranging from 5 mg to 140 mg provides a pharmacokinetic profile of (*R*)-3-amino-3-(4-chlorophenyl)butanoic acid in the blood of a fasted human patient **characterized by**:

a $C_{max}$ / dose ratio ranging from 3.0 $(10^6 \cdot mL)^{-1}$ to 7.5 $(10^6.mL)^{-1}$ ;
a $C_{max}$ / $C_{12}$ ratio ranging from 3.0 to 6.2; and
an $AUC_{0\text{-}int}$ / dose ratio ranging from 33 $h/10^6 \cdot mL$ to 50 $h/10^6 mL$.

**8.** An oral dosage from according to claim 1, wherein the dosage form comprises:

from 3 wt% to 5 wt% of the (3*R*)-4-{[(1*S*)-2-methyl-1-(2-methylpropanoyloxy)propoxy]carbonylamino}-3-(4-chlorophenyl)butanoic acid,

from 12 wt% to 22 wt% of the ammonioalkyl methacrylate copolymer, and
from 30 wt% to 40 wt% of the hydroxypropylmethyl cellulose.

**9.** An oral dosage from according to claim 8, which following oral administration to a fasted human patient at a dose of (3*R*)-4-{[(1*S*)-2-methyl-1-(2-methylpropanoyloxy)propoxy]carbonylamino}-3-(4-chlorophenyl)butanoic acid ranging from 5 mg to 140 mg provides a relative oral bioavailability of (*R*)-3-amino-3-(4-chlorophenyl)butanoic acid ranging from 100% to 200%, wherein the oral bioavailability is relative to that following oral administration of an equivalent amount of (*R*)-3-amino-3-(4-chlorophenyl)butanoic acid in at least one immediate release dosage form.

**10.** An oral dosage from according to claim 8, which following oral administration to a fasted human patient at a dose of (3*R*)-4-{[(1*S*)-2-methyl-1-(2-methylpropanoyloxy)propoxy]carbonylamino}-3-(4-chlorophenyl)butanoic acid ranging from 5 mg to 140 mg provides a pharmacokinetic profile of (*R*)-3-amino-3-(4-chlorophenyl)butanoic acid in the blood of a fasted human patient **characterized by**:

a $C_{max/}$ / dose ratio ranging from 1.0 $(10^6{\cdot}mL)^{-1}$ to 2.2 $(10^6{\cdot}mL)^{-1}$;
a $C_{max}$ $C_{12}$ ratio ranging from 1.3 to 2.9; and
an $AUC_{0\text{-}inf}$ / dose ratio ranging from 21 h/$10^6{\cdot}$mL to 34 h/$10^6{\cdot}$mL.

**11.** An oral dosage from according to claim 8, which following once daily oral administration to a human patient at a dose of (3*R*)-4-{[(1*S*)-2-methyl-1-(2-methylpropanoyloxy)propoxy]carbonylamino}-3-(4-chlorophenyl)butanoic acid ranging from 5 mg to 140 mg provides a steady state pharmacokinetic profile of (*R*)-3-amino-3-(4-chlorophenyl) butanoic acid in the blood of the human patient **characterized by**:

a $C_{SS,max}$ / dose ratio ranging from 1.4 $(10^6{\cdot}mL)^{-1}$ to 3.0 $(10^6{\cdot}ml)^{-1}$
a $C_{SS,min}$ / dose ratio ranging from 0.26 $(10^6{\cdot}mL)^{-1}$ to 0.70 $(10^6{\cdot}mL)^{-1}$;
a $C_{SS,max}$ / $C_{SS,min}$ ratio ranging from 1.1 to 9.1; and
an $AUC_{0\text{-}24}$/ dose ratio ranging from 16 h/$10^6$ mL to 35 h/$10^6{\cdot}$mL.

**12.** An oral dosage from according to claim 11, wherein:

the $C_{SS,max}$ / dose ratio ranges from 1.8 $(10^6{\cdot}mL)^{-1}$ to 2.6 $(10^6{\cdot}mL)^{-1}$;
the $C_{SS,min}$ / dose ratio ranges from 0.37 $(10^6{\cdot}mL)^{-1}$ to 0.59 $(10^6{\cdot}mL)^{-1}$;
the $C_{SS,max}$ / $C_{SS,min}$ ratio ranges from 3.1 to 7.1; and
the $AUC_{0.24}$ / dose ratio ranges from 21 h/$10^6$ mL to 30 h/$10^6$.mL.

**13.** An oral dosage from according to claim 8, which following twice daily oral administration to a human patient at a dose of (3*R*)-4-{[(1*S*)-2-methyl-1-(2-methylpropanoyloxy)propoxy]carbonylamino}-3-(4-chlorophenyl)butanoic acid ranging from 5 mg to 140 mg provides a steady state pharmacokinetic profile of (R)-3-amino-3-(4-chlorophenyl) butanoic acid in the blood of the human patient **characterized by**:

a $C_{ss,max}$ / dose ratio ranging from 2.2 $(10^6{\cdot}mL)^{-1}$ to 5.2 $(10^6{\cdot}mL)^{-1}$;
a $C_{SS,min}$ / dose ratio ranging from 1.2 $(10^6{\cdot}mL)^{-1}$ to 2.2 $(10^6{\cdot}mL)^{-1}$ ;
a $C_{SS,max}$ / $C_{SS,min}$ ratio ranging from 1.1 to 3.5; and
an $AUC_{0\text{-}24}$ / dose ratio ranging from 42 h/$10^6$ mL to 76 h/$10^6{\cdot}$mL.

**14.** An oral dosage from according to claim 12, wherein:

the $C_{SS,max}$ / dose ratio ranges from 3.0 $(10^6{\cdot}mL)^{-1}$ to 4.4 $(10^6{\cdot}mL)^{-1}$;
the $C_{SS,min}$ / dose ratio ranges from 1.4 $(10^6.mL)^{-1}$ to 2.0 $(10^6{\cdot}ml)^{-1}$;
the $C_{SS,max}$ / $C_{SS,min}$ ratio ranges from 1.7 to 2.9; and
the $AUC_{0\text{-}24}$ / dose ratio ranges from 51 h/$10^6$ mL to 67 h/$10^6{\cdot}$mL.

**15.** An oral dosage from according to claim 1 or 8, wherein the (3*R*)-4-{[(1*S*)-2-methyl-1-(2-methylpropanoyloxy)propoxy] carbonylamino}-3-(4-chlorophenyl)butanoic acid is present in an amount ranging from 5 mg to 80 mg.

**16.** An oral dosage form according to any one of claims 1 to 15 for use in a method of treatment of the human or animal body by therapy.

**17.** An oral dosage form according to any one of claims 1 to 15 for use in a method of treatment of spasticity, gastro-esophageal reflux disease, emesis, cough, narcotic addiction or abuse, alcohol addiction or abuse, nicotine addiction or abuse, neuropathic pain, or musculoskeletal pain.

**18.** An oral dosage form according to any one of claims 1 to 15 for use in a method of treatment of spasticity.

**19.** An oral dosage form according to any one of claims 1 to 15 for use in a method of treatment of gastro-esophageal reflux disease.

**20.** An oral dosage form according to any one of claims 1 to 15 for use in a method of treatment of neuropathic pain, wherein the neuropathic pain is chosen from post-herpetic neuralgia, peripheral neuropathy, trigeminal neuralgia, painful diabetic neuropathy, HIV-related neuropathic pain, cancer-related pain, and fibromyalgia.

**21.** An oral dosage form according to any one of claims 1 to 15 for use in a method of treatment of musculoskeletal pain, wherein the musculoskeletal pain is chosen from tension headache and low back pain.

**22.** Use of (3*R*)-4-{[(1S)-2-methy)-1-(2-methy)propanoy)oxy)propoxy]carbony)amino}-3-(4-chlorophenyl)butanoic acid or a pharmaceutically acceptable salt thereof in the manufacture of a dosage form according to any one of claims 1 to 15 for the treatment of spasticity, gastro-esophageal reflux disease, emesis, cough, narcotic addiction or abuse, alcohol addiction or abuse, nicotine addiction or abuse, neuropathic pain, or musculoskeletal pain.

**23.** Use of (3*R*)-4-{[(1S)-2-methyl-1-(2-methylpropanoyloxy)propoxy]carbonylamino}-3-(4-chlorophenyl)butanoic acid or a pharmaceutically acceptable salt thereof in the manufacture of a dosage form according to any one of claims 1 to 15 for the treatment of spasticity.

**24.** Use of (3*R*)-4-{[(1S)-2-methyl-1-(2-methylpropanoyloxy)propoxy]carbonylamino}-3-(4-chlorophenyl)butanoic acid or a pharmaceutically acceptable salt thereof in the manufacture of a dosage form according to any one of claims 1 to 15 for the treatment of gastro-esophageal reflux disease.

**25.** Use of (3R)-4-{[(1S)-2-methyl-1-(2-methylpropanoyloxy)propoxy]carbonylamino}-3-(4-chlorophenyl)butanoic acid or a pharmaceutically acceptable salt thereof in the manufacture of a dosage form according to any one of claims 1 to 15 for the treatment of neuropathic pain, wherein the neuropathic pain is chosen from post-herpetic neuralgia, peripheral neuropathy, trigeminal neuralgia, painful diabetic neuropathy, HIV-related neuropathic pain, cancer-related pain, and fibromyalgia.

**26.** Use of (3R)-4-{[(1S)-2-methy)-1-(2-methylpropanoyloxy)propoxy]carbonylamino}-3-(4-chlorophenyl)butanoic acid or a pharmaceutical acceptable salt thereof in the manufacture of a dosage form according to any one of claims 1 to 15 for the treatment of musculoskeletal pain, wherein the musculoskeletal pain is chosen from tension headache and low back pain.

## Patentansprüche

**1.** Orale Retard-Dosierungsform, umfassend eine Tablette, worin die Tablette Folgendes umfasst:

(3R)-4-{[(1S)-2-Methyl-1-(2-methylpropanoyloxy)propoxy]carbonylamino}-3-(4-chlorphenyl)butansäure oder ein pharmazeutisch annehmbares Salz davon,
Ammonioalkylmethacrylatcopolymer und
Hydroxypropylmethylcellulose.

**2.** Orale Dosierungsform nach Anspruch 1, worin die (3R)-4-{[(1S)-2-Methyl-1-(2-methylpropanoyloxy)propoxy]carbonylamino}-3-(4-chlorphenyl)butansäure in therapeutisch wirksamer Menge vorliegt.

**3.** Orale Dosierungsform nach Anspruch 2, worin die therapeutisch wirksame Menge 2-mg-Äquivalente R-Baclofen bis 40-mg-Äquivalente R-Baclofen umfasst.

**4.** Orale Dosierungsform nach Anspruch 2, worin die therapeutisch wirksame Menge geringer ist als die Menge, die bei einem Patienten leichte Beruhigung und Störung der motorischen Aktivität verursacht.

5. Orale Dosierungsform nach Anspruch 1, worin die (3R)-4-{[(1S)-2-Methyl-1-(2-methylpropanoyloxy)propoxy]carbonylamino}-3-(4-chlorphenyl)butansäure in einer Menge von 5 mg bis 80 mg vorliegt.

6. Orale Dosierungsform nach Anspruch 1, die nach oraler Verabreichung an einen nüchternen menschlichen Patienten einer von 5 mg bis 140 mg (3R)-4-{[(1S)-2-Methyl-1-(2-methylpropanoyloxy)propoxy]carbonylamino}-3-(4-chlorphenyl)butansäure reichenden Dosis eine relative orale Bioverfügbarkeit von (R)-3-Amino-3-(4-chlorphenyl)butansäure im Bereich von 220 % bis 340 % bewirkt, worin die orale Bioverfügbarkeit relativ zu jener angegeben ist, die nach oraler Verabreichung einer gleichwertigen Menge (R)-3-Amino-3-(4-chlorphenyl)butansäure in zumindest einer Dosierungsform mit sofortiger Freisetzung folgt.

7. Orale Dosierungsform nach Anspruch 1, die nach oraler Verabreichung an einen nüchternen menschlichen Patienten einer von 5 mg bis 140 mg (3R)-4-{((1S)-2-Methyl-1-(2-methylpropanoyloxy)propoxy]carbonylamino}-3-(4-chlorphenyl)butansäure reichenden Dosis ein pharmakokinetisches Profil von (R)-3-Amino-3-(4-chlorphenyl)butansäure im Blut eines nüchternen menschlichen Patienten bewirkt, das durch Folgendes **gekennzeichnet** ist:

ein Verhältnis $C_{max}$ / Dosis, das von 3,0 $(10^6 \cdot ml)^{-1}$ bis 7,5 $(10^6 \cdot m)^{-1}$ reicht;
ein Verhältnis $C_{max}$ / $C_{12}$, das von 3,0 bis 6,2 reicht; und
ein Verhältnis $AUC_{0\text{-unendl}}$ / Dosis, das von 33 h/$10^6$·ml bis 50 h/$10^6$·ml reicht.

8. Orale Dosierungsform nach Anspruch 1, worin die Dosierungsform Folgendes umfasst:

3 Gew.-% bis 5 Gew.-% der (3R)-4-{[(1S)-2-Methyl-1-(2-methylpropanoyloxy)-propoxy]carbonylamino}-3-(4-chlorphenyl)butansäure,
12 Gew.-% bis 22 % des Ammonioalkylmethacrylatcopolymers und
30 Gew.-% bis 40 Gew.-% der Hydroxypropylmethylcellulose.

9. Orale Dosierungsform nach Anspruch 8, die nach oraler Verabreichung an einen nüchternen menschlichen Patienten einer von 5 mg bis 140 mg (3R)-4-{[(1S)-2-Methyl-1-(2-methylpropanoyloxy)propoxy]carbonylamino}-3-(4-chlorphenyl)butansäure reichenden Dosis eine relative orale Bioverfügbarkeit von (R)-3-Amino-3-(4-chlorphenyl)butansäure im Bereich von 100 % bis 200 % bewirkt, worin die orale Bioverfügbarkeit relativ zu jener angegeben ist, die nach oraler Verabreichung einer gleichwertigen Menge (R)-3-Amino-3-(4-chlorphenyl)butansäure in zumindest einer Dosierungsform mit sofortiger Freisetzung folgt.

10. Orale Dosierungsform nach Anspruch 8, die nach oraler Verabreichung an einen nüchternen menschlichen Patienten einer von 5 mg bis 140 mg (3R)-4-{[(1S)-2-Methyl-1-(2-methylpropanoyloxy)propoxy]carbonylamino}-3-(4-chlorphenyl)butansäure reichenden Dosis ein pharmakokinetisches Profil von (R)-3-Amino-3-(4-chlorphenyl)butansäure im Blut eines nüchternen menschlichen Patienten bewirkt, das durch Folgendes **gekennzeichnet** ist:

ein Verhältnis $C_{max}$ / Dosis, das von 1,0 $(10^6 \cdot ml)^{-1}$ bis 2,2 $(10^6 \cdot ml)^{-1}$ reicht;
ein Verhältnis $C_{max}$ / $C_{12}$, das von 1,3 bis 2,9 reicht; und
ein Verhältnis $AUC_{0\text{-unendl}}$ / Dosis, das von 21 h/$10^6$·ml bis 34 h/$10^6$·ml reicht.

11. Orale Dosierungsform nach Anspruch 8, die nach oraler Verabreichung an einen menschlichen Patienten einer von 5 mg bis 140 mg (3R)-4-{[(1S)-2-Methyl-1-(2-methylpropanoyloxy)propoxy]carbonylamino}-3-(4-chlorphenyl)butansäure reichenden Dosis einmal täglich ein dauerndes pharmakokinetisches Profil von (R)-3-Amino-3-(4-chlorphenyl) butansäure im Blut des menschlichen Patienten bewirkt, das durch Folgendes **gekennzeichnet** ist:

ein Verhältnis $C_{SS,max}$ / Dosis, das von 1,4 $(10^6 \cdot ml)^{-1}$ bis 3,0 $(10^6 \cdot ml)^{-1}$ reicht;
ein Verhältnis $C_{SS,min}$ / Dosis, das von 0,26 $(10^6 \cdot ml)^{-1}$ bis 0,70 $(10^6 \cdot ml)^{-1}$ reicht;
ein Verhältnis $C_{SS,max}$ / $C_{SS,min}$, das von 1,1 bis 9,1 reicht; und
ein Verhältnis $AUC_{0\text{-}24}$ / Dosis, das von 16 h/$10^6$·ml bis 35 h/$10^6$·ml reicht.

12. Orale Dosierungsform nach Anspruch 11, worin
das Verhältnis $C_{SS,max}$ / Dosis von 1,8 $(10^6 \cdot ml)^{-1}$ bis 2,6 $(10^6 \cdot ml)^{-1}$ reicht;
das Verhältnis $C_{SS,min}$ / Dosis von 0,37 $(10^6 \cdot ml)^{-1}$ bis 0,59 $(10^6 \cdot ml)^{-1}$ reicht;
das Verhältnis $C_{SS,max}$ / $C_{SS,min}$ von 3,1 bis 7,1 reicht; und
das Verhältnis $AUC_0$-24 / Dosis von 21 h/$10^6$·ml bis 30 /h/$10^6$·ml reicht.

**13.** Orale Dosierungsform nach Anspruch 8, die nach oraler Verabreichung an einen menschlichen Patienten einer von 5 mg bis 140 mg (3R)-4-{[(1S)-2-Methyl-1-(2-methylpropanoyloxy)propoxy]carbonylamino}-3-(4-chlorphenyl)butansäure reichenden Dosis zweimal täglich ein dauerndes pharmakokinetisches Profil von (R)-3-Amino-3-(4-chlorphenyl)butansäure im Blut des menschlichen Patienten bewirkt, das durch Folgendes **gekennzeichnet** ist:

ein Verhältnis $C_{SS,max}$ / Dosis, das von 2,2 $(10^6 \cdot ml)^{-1}$ bis 5,2 $(10^6 \cdot ml)^{-1}$ reicht;
ein Verhältnis $C_{SS,min}$ / Dosis, das von 1,2 $(10^6 \cdot ml)^{-1}$ bis 2,2 $(10^6 \cdot ml)^{-1}$ reicht;
ein Verhältnis $C_{SS,max}$ / $C_{SS,min}$, das von 1,1 bis 3,5 reicht; und
ein Verhältnis $AUC_{0-24}$ / Dosis, das von 42 h/$10^6 \cdot$ml bis 76 h/$10^6 \cdot$ml reicht.

**14.** Orale Dosierungsform nach Anspruch 12, worin
das Verhältnis $C_{SS,max}$ / Dosis von 3,0 $(10^6 \cdot ml)^{-1}$ bis 4,4 $(10^6 \cdot ml)^{-1}$ reicht;
das Verhältnis $C_{SS,min}$ / Dosis von 1,4 $(10^6 \cdot ml)^{-1}$ bis 2,0 $(10^6.ml)^{-1}$ reicht;
das Verhältnis $C_{SS,max}$ / $C_{SS,min}$ von 1,7 bis 2,9 reicht; und
das Verhältnis $AUC_{0-24}$ / Dosis von 51 h/$10^6 \cdot$ml bis 67 h/$10^6 \cdot$m1 reicht.

**15.** Orale Dosierungsform nach Anspruch 1 oder 8, worin die (3R)-4-{[(1S)-2-Methyl-1-(2-methylpropanoyloxy)propoxy] carbonylamino}-3-(4-chlorphenyl)butansäure in einer Menge von 5 mg bis 80 mg vorliegt.

**16.** Orale Dosierungsform nach einem der Ansprüche 1 bis 15 zur Verwendung in einem Verfahren zur Behandlung des menschlichen oder tierischen Körpers durch Therapie.

**17.** Orale Dosierungsform nach einem der Ansprüche 1 bis 15 zur Verwendung in einem Verfahren zur Behandlung von spastischer Lähmung, gastroösophagealer Refluxkrankheit, Erbrechen, Husten, Rauschgiftabhängigkeit oder -missbrauch, Alkoholabhängigkeit oder - missbrauch, Nikotinabhängigkeit oder -missbrauch, neuropathischem Schmerz oder Schmerzen im Bewegungsapparat.

**18.** Orale Dosierungsform nach einem der Ansprüche 1 bis 15 zur Verwendung in einem Verfahren zur Behandlung von spastischer Lähmung.

**19.** Orale Dosierungsform nach einem der Ansprüche 1 bis 15 zur Verwendung in einem Verfahren zur Behandlung von gastroösophagealer Refluxkrankheit.

**20.** Orale Dosierungsform nach einem der Ansprüche 1 bis 15 zur Verwendung in einem Verfahren zur Behandlung von neuropathischem Schmerz, worin der neuropathische Schmerz aus postherpetischer Neuralgie, peripherer Neuropathie, Trigeminusneuralgie, schmerzhafter diabetischer Neuropathie, HIV-bedingtem neuropathischem Schmerz, krebsbedingtem Schmerz und Fibromyalgie ausgewählt ist.

**21.** Orale Dosierungsform nach einem der Ansprüche 1 bis 15 zur Verwendung in einem Verfahren zur Behandlung von Schmerzen des Bewegungsapparates, worin die Schmerzen des Bewegungsapparates aus Spannungskopfschmerz und Kreuzschmerzen ausgewählt sind.

**22.** Verwendung von (3R)-4-{[(1S)-2-Methyl-1-(2-methylpropanoyloxy)propoxy]-carbonylamino}-3-(4-chlorphenyl)butansäure oder einem pharmazeutisch annehmbaren Salz davon zur Herstellung einer Dosierungsform nach einem der Ansprüche 1 bis 15 zur Behandlung von spastischer Lähmung, gastroösophagealer Refluxkrankheit, Erbrechen, Husten, Rauschgiftabhängigkeit oder -missbrauch, Alkoholabhängigkeit oder - missbrauch, Nikotinabhängigkeit oder -missbrauch, neuropathischem Schmerz oder Schmerzen im Bewegungsapparat.

**23.** Verwendung von (3R)-4-{[(1S)-2-Methyl-1-(2-methylpropanoyloxy)propoxy]-carbonylamino}-3-(4-chlorphenyl)butansäure oder einem pharmazeutisch annehmbaren Salz davon zur Herstellung einer Dosierungsform nach einem der Ansprüche 1 bis 15 zur Behandlung von spastischer Lähmung.

**24.** Verwendung von (3R)-4-{[(1S)-2-Methyl-1-(2-methylpropanoyloxy)propoxy]-carbonylamino}-3-(4-chlorphenyl)butansäure oder einem pharmazeutisch annehmbaren Salz davon zur Herstellung einer Dosierungsform nach einem der Ansprüche 1 bis 15 zur Behandlung von gastroösophagealer Refluxkrankheit.

**25.** Verwendung von (3R)-4-{[(1S)-2-Methyl-1-(2-methylpropanoyloxy)propoxy]-carbonylamino}-3-(4-chlorphenyl)butansäure oder einem pharmazeutisch annehmbaren Salz davon zur Herstellung einer Dosierungsform nach einem

der Ansprüche 1 bis 15 zur Behandlung von neuropathischem Schmerz, worin der neuropathische Schmerz aus postherpetischer Neuralgie, peripherer Neuropathie, Trigeminusneuralgie, schmerzhafter diabetischer Neuropathie, HIV-bedingtem neuropathischem Schmerz, krebsbedingtem Schmerz und Fibromyalgie ausgewählt ist.

**26.** Verwendung von (3R)-4-{[(1S)-2-Methyl-1-(2-methylpropanoyloxy)propoxy]-carbonylamino}-3-(4-chlorphenyl)butansäure oder einem pharmazeutisch annehmbaren Salz davon zur Herstellung einer Dosierungsform nach einem der Ansprüche 1 bis 15 zur Behandlung von Schmerzen des Bewegungsapparates, worin die Schmerzen des Bewegungsapparates aus Spannungskopfschmerz und Kreuzschmerzen ausgewählt sind.

**Revendications**

**1.** Forme de dosage orale à libération prolongée comprenant une tablette, où la tablette comprend:

de l'acide (3R)-4-{(15)-2-méthyl-1-(2-méthylpropanoyloxy)propoxylcarbonylamino}-3- (4-chlorophényl)butanoique ou
un sel pharmaceutiquement acceptable de celui-ci,
un copolymère d'ammonioalkyle méthacrylate, et
de la cellulose d'hydroxypropylméthyle.

**2.** Forme de dosage orale selon la revendication 1, où l'acide (3R)-4-{[(1S)-2-méthyl-1-(2-méthylpropanoyloxy) propoxylcarbonylamino}-3- (4-chlorophényl)butanoique est présent en une quantité thérapeutiquement efficace.

**3.** Forme de dosage orale selon la revendication 2, où la quantité thérapeutiquement efficace comprend de 2 mg-équivalents de R-baclofen à 40 mg-équivalents de R-baclofen.

**4.** Forme de dosage orale selon la revendication 2, où la quantité thérapeutiquement efficace est inférieure à une quantité qui provoque une sédation modérée et une détérioration de l'activité motrice chez un patient.

**5.** Forme de dosage orale selon la revendication 1, où l'acide (3R)-4-{[(1S)-2-méthyl-1-(2-méthylpropano-yloxy) propoxylcarbonylamino}-3-(4-chlorophényl)butanoique est présent en une quantité de 5 mg à 80 mg.

**6.** Forme de dosage orale selon la revendication 1, qui à la suite d'une administration orale à un patient humain à jeun à une dose d'acide (3R)-4-{[(1S)-2-méthyl-1-(2-méthylpropanoyloxy)propoxylcarbonylamino}-3- (4-chlorophényl) butanoique de 5 mg à 140 mg, entraîne une biodisponibilité orale relative de l'acide (R)-3-amino-3-(4-chlorophényl) butanoïque de 220% à 340%, où la biodisponibilité orale est relative à celle suivant l'administration orale d'une quantité équivalente de l'acide (R)-3-amino-3-(4-chlorophényl) butanoïque en au moins une forme de dosage à liberation immediate.

**7.** Forme de dosage orale selon la revendication 1, qui à la suite de l'administration orale à un patient humain à jeun à une dose d'acide (3R)-4-[[(1S)-2-méthyl-1-(2-méthylpropanoyloxy) propoxy]carbonylamino]-3-(4-chlorophényl) butanoïque de 5 mg à 140 mg entraîne un profil pharmacocinétique de l'acide (R)-3-amino-3-(4-chlorophényl) butanoïque dans le sang d'un patient humain à jeun **caractérisé par**:

un rapport de $C_{max}$ / dose de 3,0 $(10^6 \cdot ml)^{-1}$ à 7,5 $(10^6 \cdot ml)^{-1}$
un rapport de $C_{max}$ $C_{12}$ de 3,0 à 6,2; et
un rapport de $Auc_{0-inf}$/dose de 33 h/$10^6$·ml à 50 h/$10^6$·ml.

**8.** Forme de dosage orale selon la revendication 1, où la forme de dosage comprend:

de 3% en poids à 5% en poids de l'acide (3R)-4-[[(1S)-2-méthyl-1-(2-méthylpropanoyloxy)propoxy] carbonylamino]-3-(4-chlorophényl)butanoïque,
de 12% en poids à 22% en poids du copolymère d'ammonioalkyl méthacrylate, et
de 30% en poids à 40% en poids de l'hydroxypropylméthyl cellulose.

**9.** Forme de dosage orale selon la revendication 8, qui à la suite de l'administration orale à un patient humain à jeun à une dose d'acide (3R)-4-[[(1S)-2-méthyl-1-(2-méthylpropanoyloxy) propoxy]carbonylamino]-3-(4-chloro-phényl) butanoïque, de 5 mg à 140 mg entraîne une biodisponibilité orale relative de l'acide (R)-3-amino-3-(4-chlorophényl)butanoïque de 100% à 200%, où la biodisponibilité orale est relative à celle suivant l'administration orale d'une quantité équivalente de l'acide (R)-3-amino-3-(4-chlorophényl)butanoïque en au moins une forme de dosage à libération immédiate.

**10.** Forme de dosage orale selon la revendication 8, qui à la suite de l'administration orale à un patient humain à jeun à une dose d'acide (3R)-4-[[(1S)-2-méthyl-1-(2-méthylpropanoyloxy)propoxy]carbonylamino]-3-(4-chlorophényl)butanoïque de 5 mg à 140 mg entraîne un profil pharmacocinétique de l'acide (R)-3-amino-3-(4-chlorophényl)butanoïque dans le sang d'un patient humain à jeun, **caractérisée par**:

un rapport de $C_{max}$ / dose de 1,0 $(10^6 \cdot m)^{-1}$ à 2,2 $(10^6 \cdot ml)^{-1}$;
un rapport de $C_{max}$ / $C_{12}$ de 1, 3 à 2,9; et
un rapport de $AUC_{0-inf}$/dose de 21 h/$10^6 \cdot$ml à 34 h/$10^6 \cdot$ml.

**11.** Forme de dosage oral e selon la revendication 8, qui à la suite d'une administration orale journalière à un patient humain à une dose d'acide (3R)-4-[[(1S)-2-méthyl-1-(2-méthylpropanoyloxy)propoxy]carbonylamino]-3-(4-chlorophényl)butanoïque de 5 mg à 140 mg entraîne un profil pharmacocinétique en conditions stationnaires de l'acide (R)-3-amino-3-(4-chlorophényl)butanoïque dans le sang du patient humain, **caractérisée par**:

un rapport de $C_{ss,max}$ / dose de 1,4 $(10^6$ ml$)^{-1}$ à 3,0 $(10^6$ ml$)^{-1}$
un rapport de $C_{ss,min}$ / dose de 0,26 $(10^6$ ml$)^{-1}$ à 0,70 $(10^6$, ml$)^{-1}$;
un rapport de $C_{ss,max}$ / $C_{ss,min}$ dose de 1,1 à 9,1; et
un rapport de $AUC_{0-24}$ / dose de 16 h/$10^6$ ml à 3,5 h/$10^6$ ml.

**12.** Forme de dosage orale selon la revendication 11,
où:

le rapport de $C_{ss,max}$ / dose est de 1,8 $(10^6$ml$)^{-1}$ à 2,6 $(10^6$ ml$)^{-1}$;
le rapport de $C_{ss,min}$ / dose est de 0,37 $(10^6$ml$)^{-1}$ à 0,59 $(10^6$ ml$)^{-1}$;
le rapport de $C_{ss,max}$ / $C_{ss,min}$ dose est de 3,1 à 7,1 et le rapport de $AUC_{0-24}$ / dose est de 21 h/$10^6$ ml à 30 h/$10^6$ ml.

**13.** Forme de dosage orale selon la revendication 8, qui à la suite d'une administration orale deux fois par jour à un patient humain a une dose d'acide (3R)-4-[[(1S)-2-méthyl-1-(2-méthylpropanoyloxy)propoxy]carbonylamino]-3-(4-chlorophényl)butanoïque de 5 mg à 140 mg entraîne un profil pharmacocinétique en conditions stationnaires de l'acide (R)-3-amino-3-(4-chlorophényl)butanoïque dans le sang du patient humain, **caractérisée par**:

un rapport de $C_{ss,max}$ / dose de 2,2 $(10^6$ ml$)^{-1}$ à 5,2 $(10^6$ ml$)^{-1}$
un rapport de $C_{ss,min}$ / dose de 1,2 $(10^6$ml$)^{-1}$ à 2,2 $(10^6$ml$)^{-1}$;
un rapport de $C_{ss,max}$ / $C_{ss,min}$ de 1,1 à 3,5; et
un rapport de $AUC_{0,24}$ / dose de 42 h/$10^6$ ml à 76 h/$10^6$ ml.

**14.** Forme de dosage orale selon la revendication 12, où:

le rapport de $C_{ss,max}$ / dose est de 3,0 $(10^6$ ml$)^{-1}$ à 4,4 $(10^6$ ml$)^{-1}$;
le rapport $C_{ss,min}$ / dose est de 1,4 $(10^6$ ml$)^{-1}$ à 2,0 $(10^6$ ml$)^{-1}$;
le rapport de $C_{ss,max}$ / $C_{ss,min}$ est de 1,7 à 2,9; et
le rapport de $AUC_{0-24}$ / dose est de 51 h/$10^6$ ml à 67 /$10^6$ ml.

**15.** Forme de dosage orale selon la revendication 1 ou 8, où l'acide (3R)-4-[[(1S)-2-méthyl-1-(2-méthylpropanoyloxy)propoxy]carbonylamino]-3-(4-chlorophényl)butanoïque est présent en une quantité de 5 mg à 80 mg.

**16.** Forme de dosage orale selon l'une quelconque des revendications 1 à 15 pour utilisation dans une méthode de traitement du corps humain ou animal par thérapie.

**17.** Forme de dosage orale selon l'une quelconque des revendications 1 à 15 pour utilisation dans une méthode de traitement de la spasticité, de la maladie de reflux gastrooesophagien, du vomissement, de la toux, de la narcomanie

ou de l'abus, de l'alcoolisme ou de l'abus, du tabagisme ou de l'abus, de la douleur neuropathique ou de la douleur musculosquelettique.

**18.** Forme de dosage orale selon l'une quelconque des revendications 1 à 15 pour utilisation dans une méthode de traitement de la spasticité.

**19.** Forme de dosage orale selon l'une quelconque des revendications 1 à 15 pour utilisation dans une méthode de traitement de la maladie de reflux gastro-oesophagien.

**20.** Forme de dosage orale selon l'une quelconque des revendications 1 à 15 pour utilisation dans une méthode de traitement d'une douleur neuropathique, où la douleur neuropathique est sélectionnée parmi la névralgie post-herpétique, la neuropathie périphérique, la névralgie faciale, la neuropathie diabétique douloureuse, la douleur neuropathique liée au VIH, la douleur liée au cancer et la fibromyalgie.

**21.** Forme de dosage orale selon l'une quelconque des revendications 1 à 15 pour utilisation dans une méthode de traitement de la douleur musculosquelettique, où la douleur musculosquelettique est sélectionnée parmi la céphalée de tension et la lombalgie.

**22.** Utilisation de l'acide (3R)-4-[[(1S)-2-méthyl-1-(2-méthylpropanoyloxy)propoxy]carbonylamino]-3-(4-chlorophényl) butanoïque ou d'un sel pharmaceutiquement acceptable de celui-ci dans la fabrication d'une forme de dosage selon l'une quelconque des revendications 1 à 15 pour le traitement de la spasticité, de la maladie de reflux gastrooesophagien, du vomissement, de la toux, de la narcomanie ou de l'abus, de l'alcoolisme ou l'abus, du tabagisme ou de l'abus, de la douleur neuropathique ou de la douleur musculosquelettique.

**23.** Utilisation de l'acide (3R)-4-[[(1S)-2-méthyl-1-(2-méthylpropanoyloxy)propoxy]carbonylamino]-3-(4-chlorophényl) butanoïque ou d'un sel pharmaceutiquement acceptable de celui-ci dans la fabrication d'une forme de dosage selon l'une quelconque des revendications 1 à 15 pour le traitement de la spasticité.

**24.** Utilisation de l'acide (3R)-4-[[(1S)-2-méthyl-1-(2-méthylpropanoyloxy)propoxy]carbonylamino]-3-(4-chlorophényl) butanoïque ou d'un sel pharmaceutiquement acceptable de celui-ci dans la fabrication d'une forme de dosage selon l'une quelconque des revendications 1 à 15 pour le traitement de la maladie de reflux gastro-oesophagien.

**25.** Utilisation de l'acide (3R)-4-[[(1S)-2-méthyl-1-(2-méthylpropanoyloxy)propoxy]carbonylamino]-3-(4-chlorophényl) butanoïque ou d'un sel pharmaceutiquement acceptable de celui-ci dans la fabrication d'une forme de dosage selon l'une quelconque des revendications 1 à 15 pour le traitement de la douleur neuropathique, où la douleur neuropathique est sélectionnée parmi la névralgie post-herpétique, la neuropathie périphérique, la névralgie faciale, la neuropathie diabétique douloureuse, la douleur neuropathique liée au VIH, la douleur liée au cancer et la fibromyalgie.

**26.** Utilisation de l'acide (3R)-4-[[(1S)-2-méthyl-1-(2-méthylpropanoyloxy)propoxy]carbonylamino]-3-(4-chlorophényl) butanoïque ou d'un sel pharmaceutiquement acceptable de celui-ci dans la fabrication d'une forme de dosage selon l'une quelconque des revendications 1 à 15 pour le traitement de la douleur musculosquelettique, où la douleur musculosquelettique est sélectionnée parmi la céphalée de tension et la lombalgie.

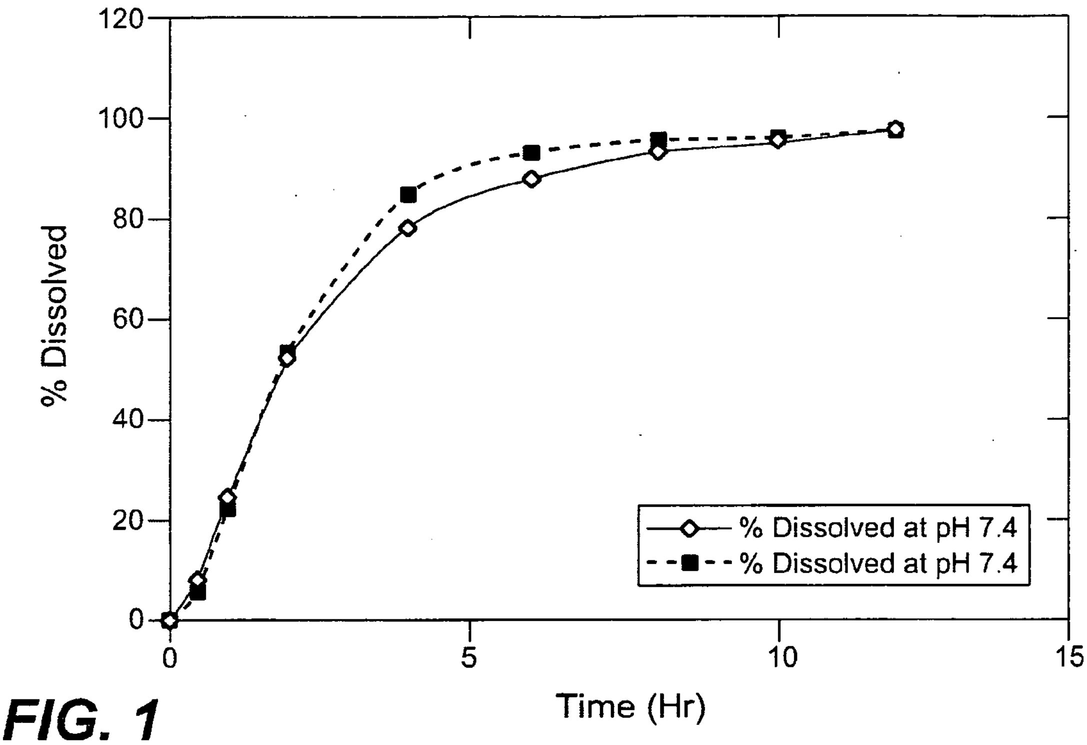
120
100
80
60
40
20
0
% Dissolved
0
5
10
15
Time (Hr)
% Dissolved at pH 7.4
% Dissolved at pH 7.4

*FIG. 1*

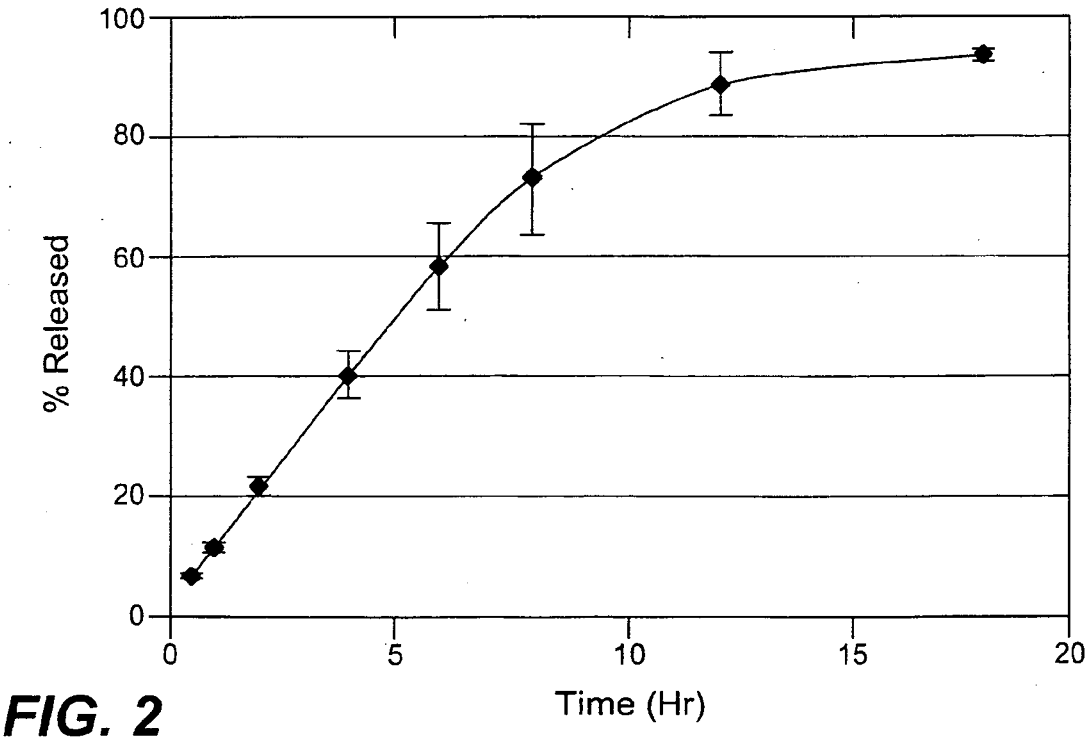
100
80
60
40
20
0
% Released
0
5
10
15
20
Time (Hr)

*FIG. 2*

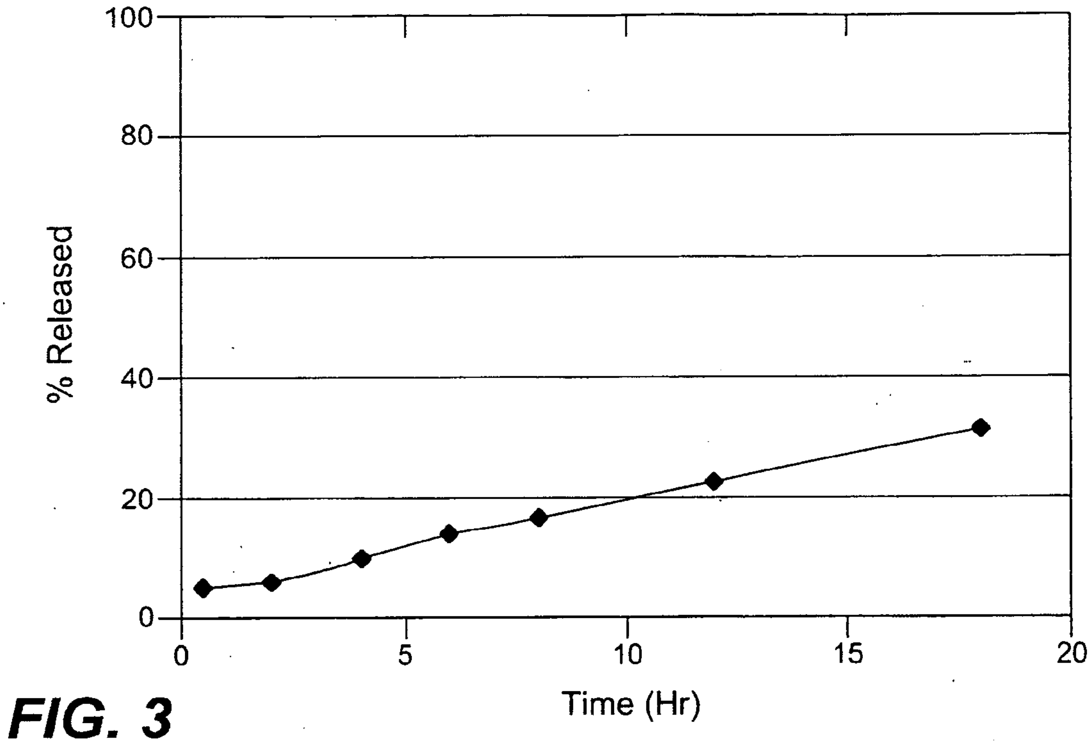
100
80
60
40
20
0
% Released
0
5
10
15
20
Time (Hr)

FIG. 3

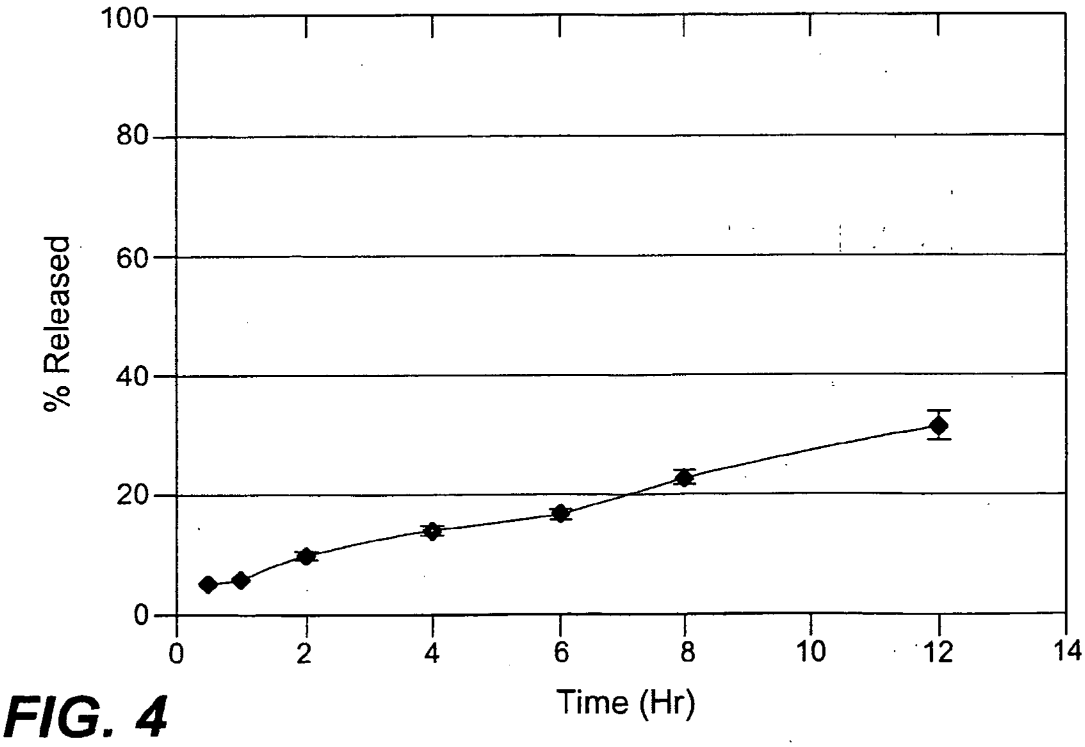
100
80
60
40
20
0
% Released
0
2
4
6
8
10
12
14
Time (Hr)

FIG. 4

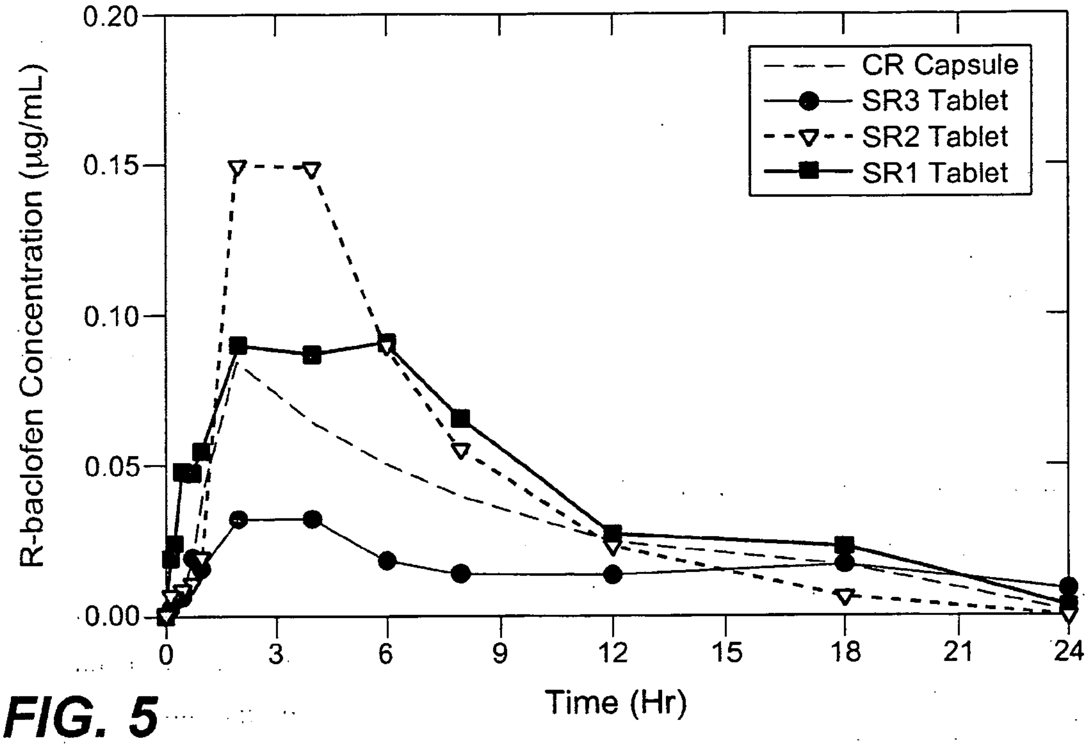
0.20
0.15
0.10
0.05
0.00
R-baclofen Concentration (µg/mL)
0
3
6
9
12
15
18
21
24
Time (Hr)
CR Capsule
SR3 Tablet
SR2 Tablet
SR1 Tablet

FIG. 5

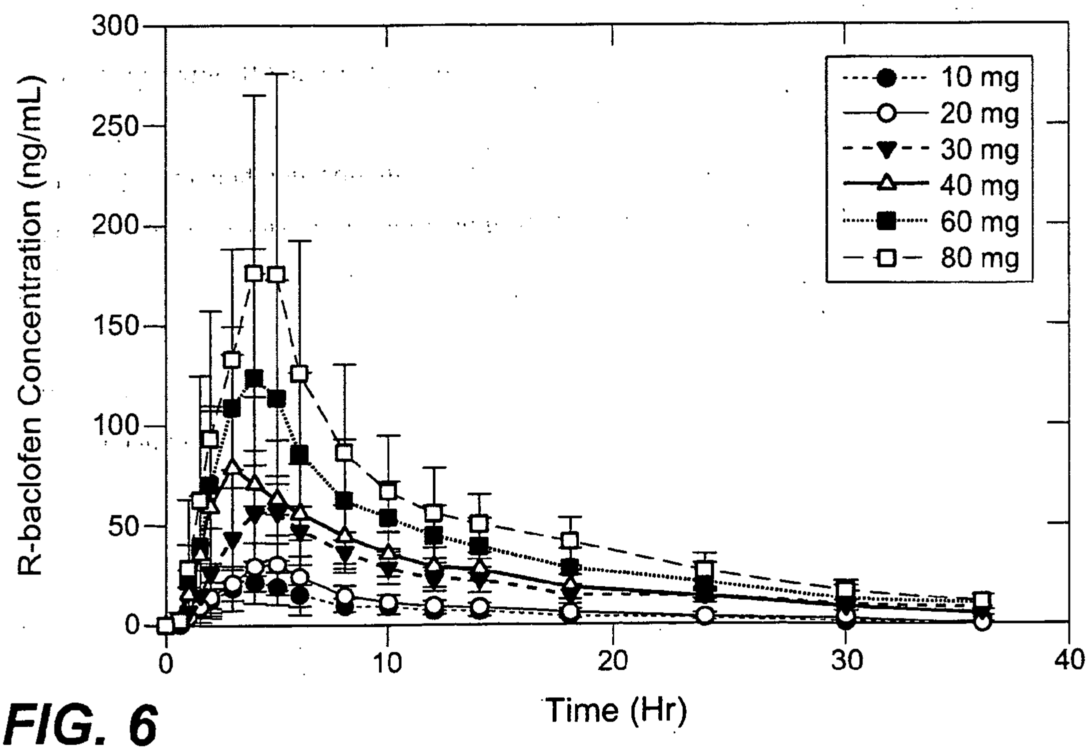
300
250
200
150
100
50
0
R-baclofen Concentration (ng/mL)
0
10
20
30
40
Time (Hr)
10 mg
20 mg
30 mg
40 mg
60 mg
80 mg

FIG. 6

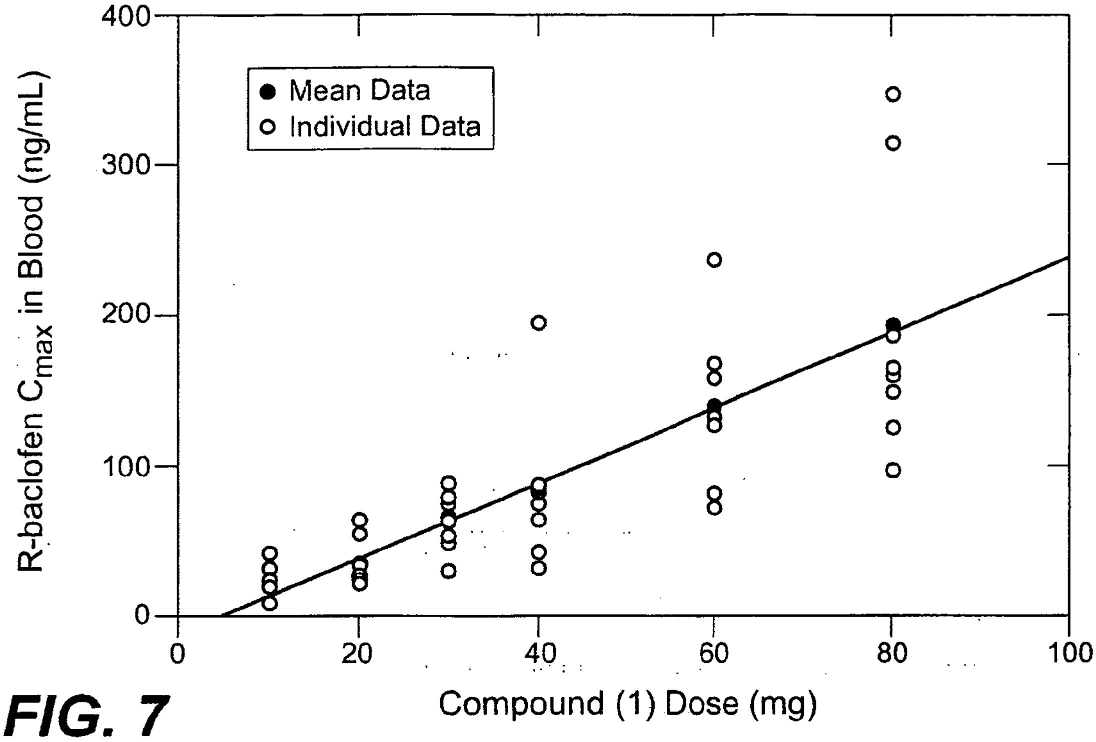
400
300
200
100
0
0
20
40
60
80
100
Mean Data
Individual Data
R-baclofen $C_{max}$ in Blood (ng/mL)
Compound (1) Dose (mg)

FIG. 7

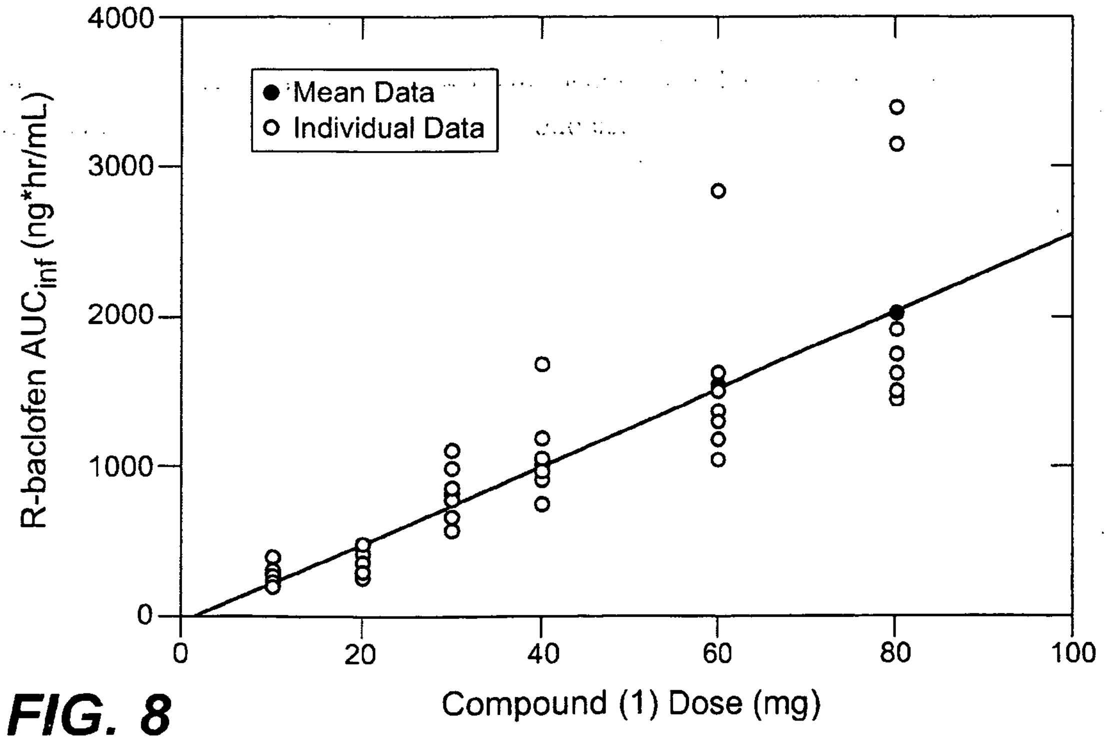
4000
3000
2000
1000
0
0
20
40
60
80
100
Mean Data
Individual Data
R-baclofen $AUC_{inf}$ (ng*hr/mL)
Compound (1) Dose (mg)

FIG. 8

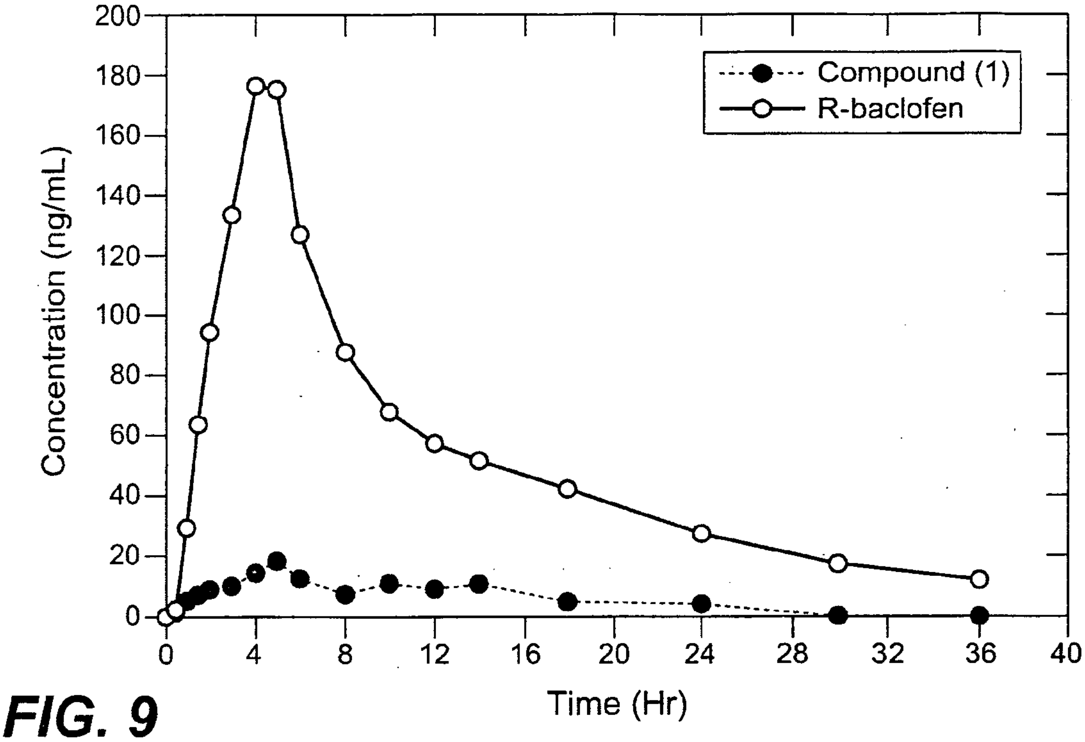
Compound (1)
R-baclofen
Concentration (ng/mL)
Time (Hr)
0
20
40
60
80
100
120
140
160
180
200
0
4
8
12
16
20
24
28
32
36
40

*FIG. 9*

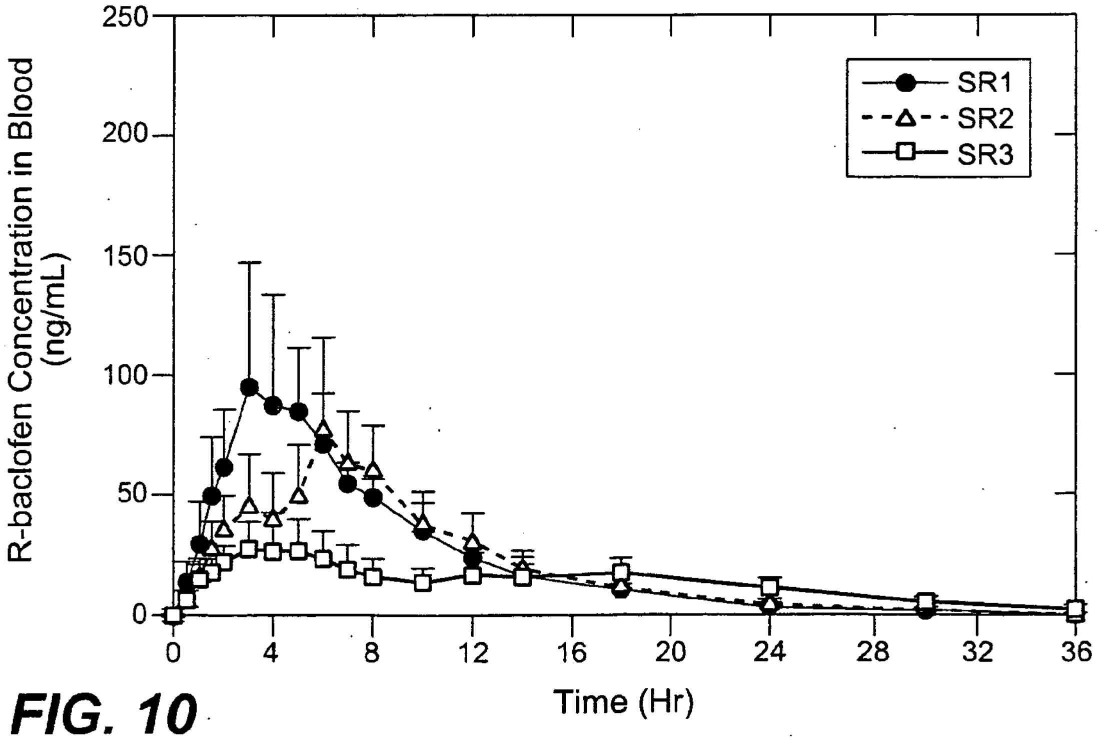
SR1
SR2
SR3
R-baclofen Concentration in Blood (ng/mL)
Time (Hr)
0
50
100
150
200
250
0
4
8
12
16
20
24
28
32
36

*FIG. 10*

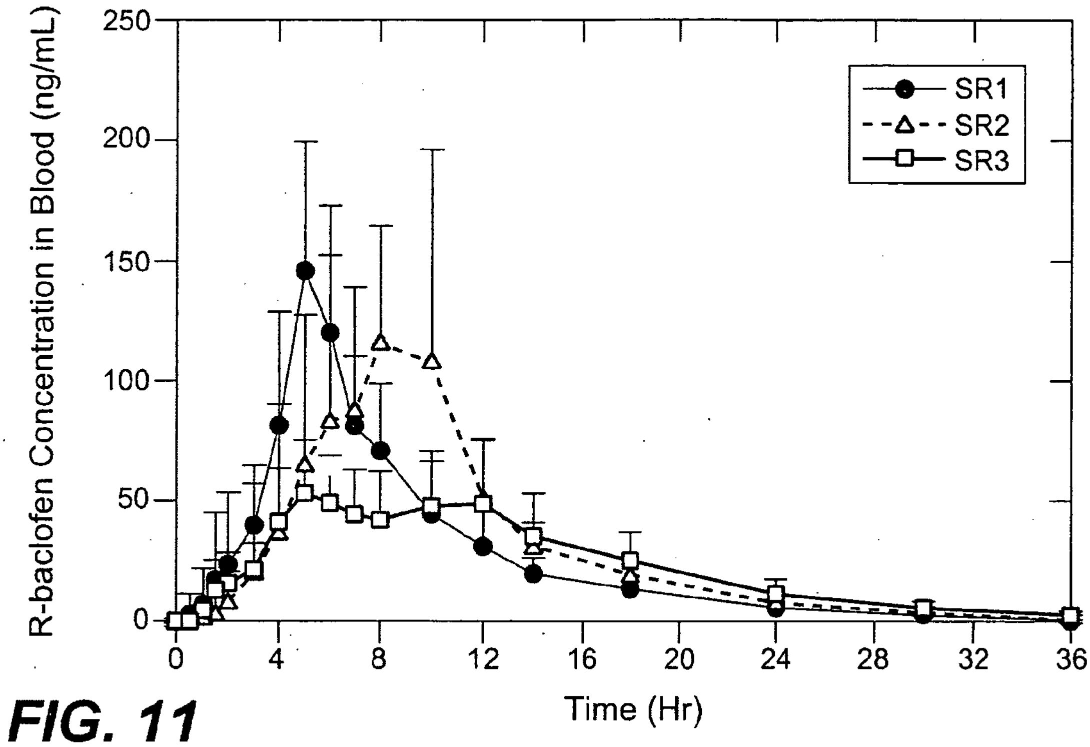
R-baclofen Concentration in Blood (ng/mL)
250
200
150
100
50
0
0
4
8
12
16
20
24
28
32
36
Time (Hr)
SR1
SR2
SR3

*FIG. 11*

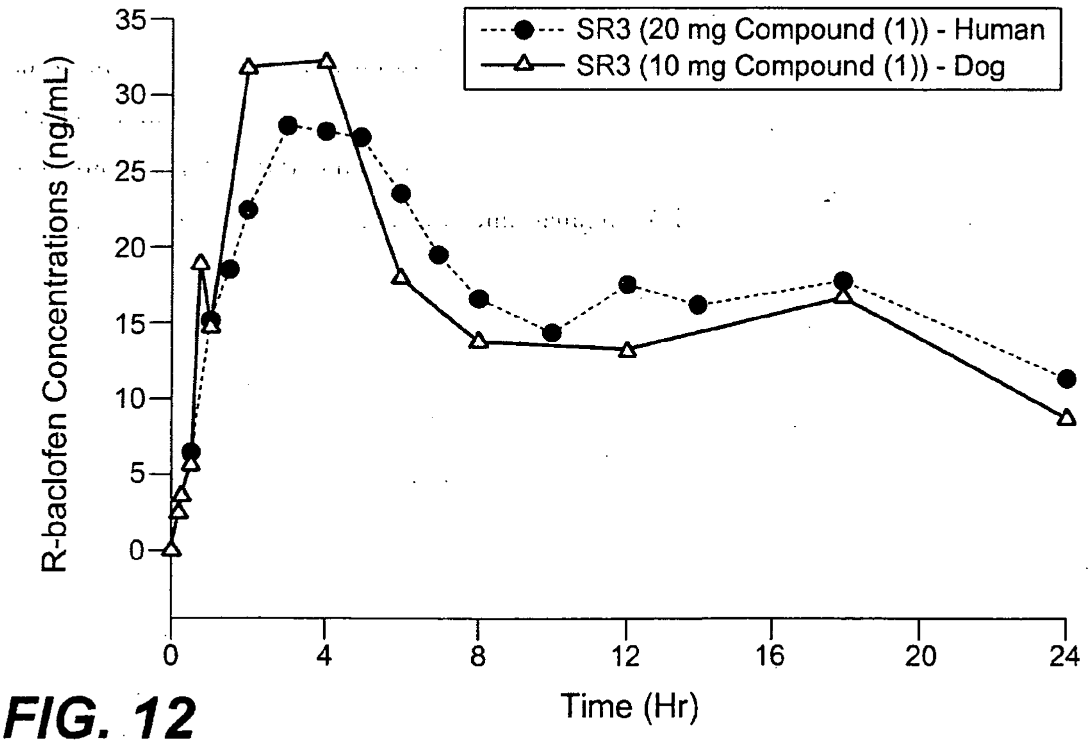
R-baclofen Concentrations (ng/mL)
35
30
25
20
15
10
5
0
0
4
8
12
16
20
24
Time (Hr)
SR3 (20 mg Compound (1)) - Human
SR3 (10 mg Compound (1)) - Dog

*FIG. 12*

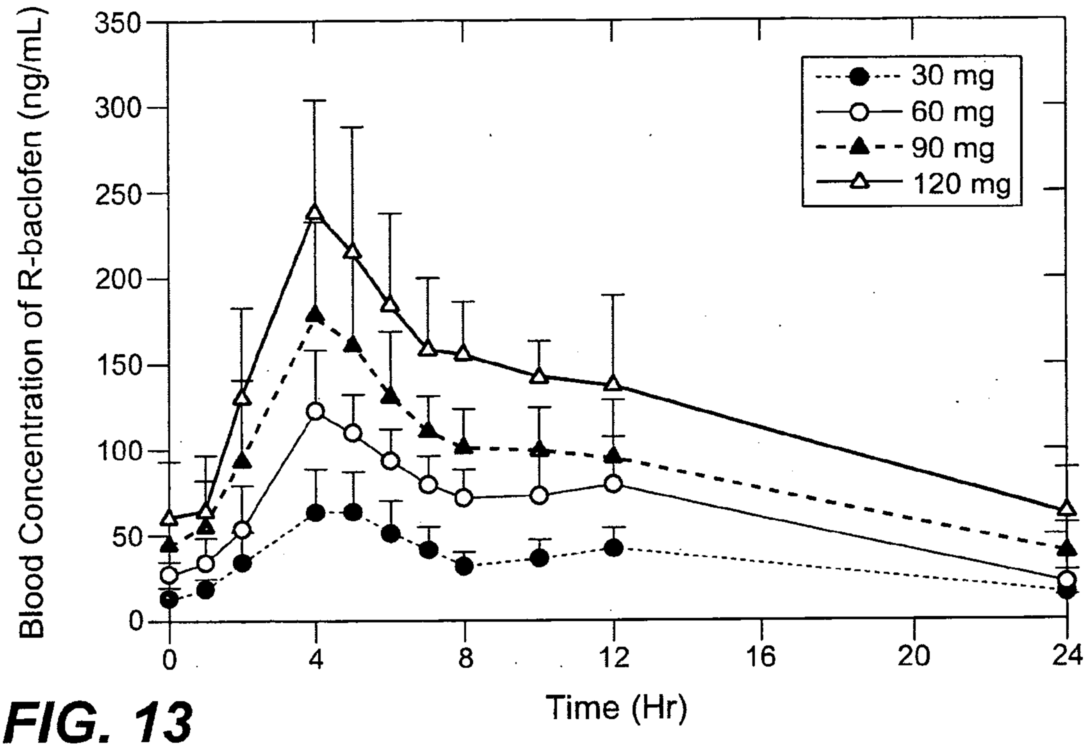

Blood Concentration of R-baclofen (ng/mL)
350
300
250
200
150
100
50
0
0
4
8
12
16
20
24
Time (Hr)
30 mg
60 mg
90 mg
120 mg


FIG. 13

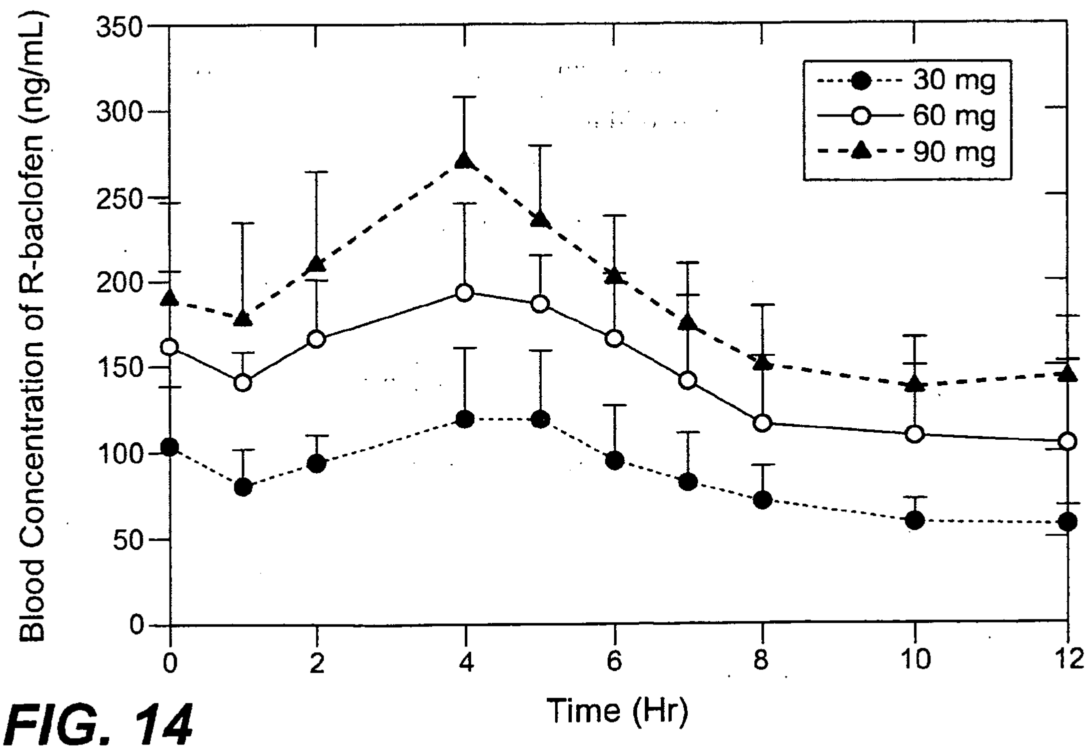

Blood Concentration of R-baclofen (ng/mL)
350
300
250
200
150
100
50
0
0
2
4
6
8
10
12
Time (Hr)
30 mg
60 mg
90 mg


FIG. 14

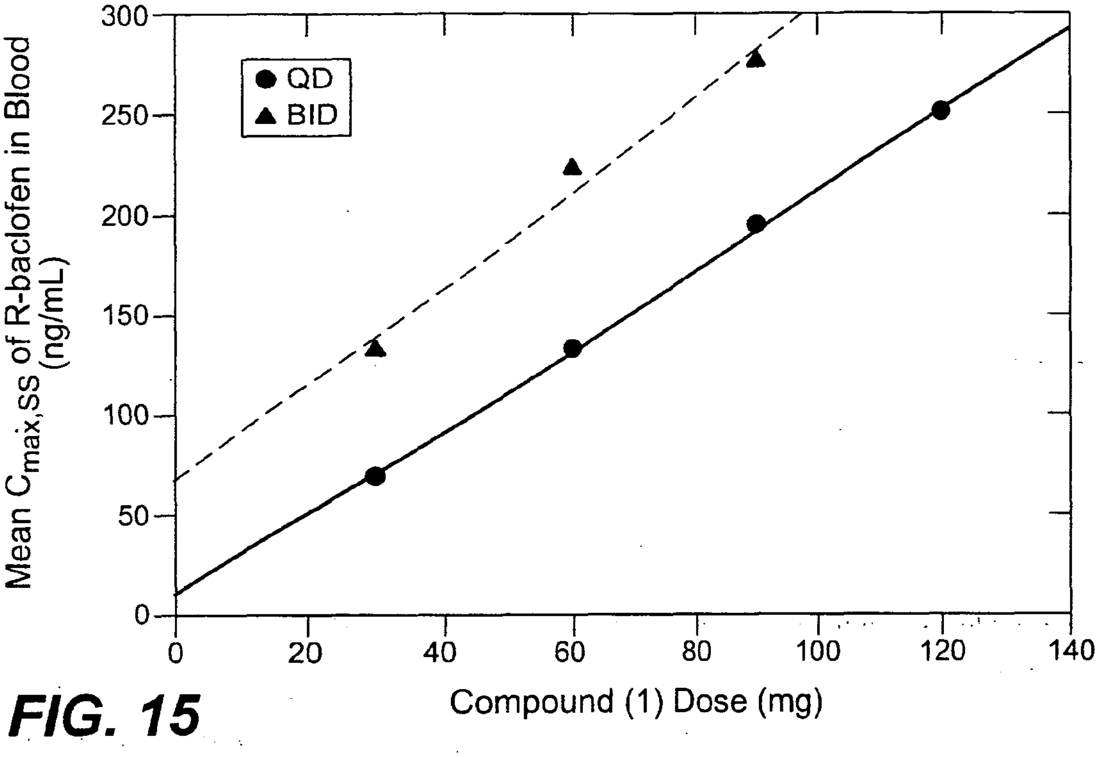
Mean $C_{max,SS}$ of R-baclofen in Blood (ng/mL)
QD
BID
0
50
100
150
200
250
300
0
20
40
60
80
100
120
140
Compound (1) Dose (mg)

*FIG. 15*

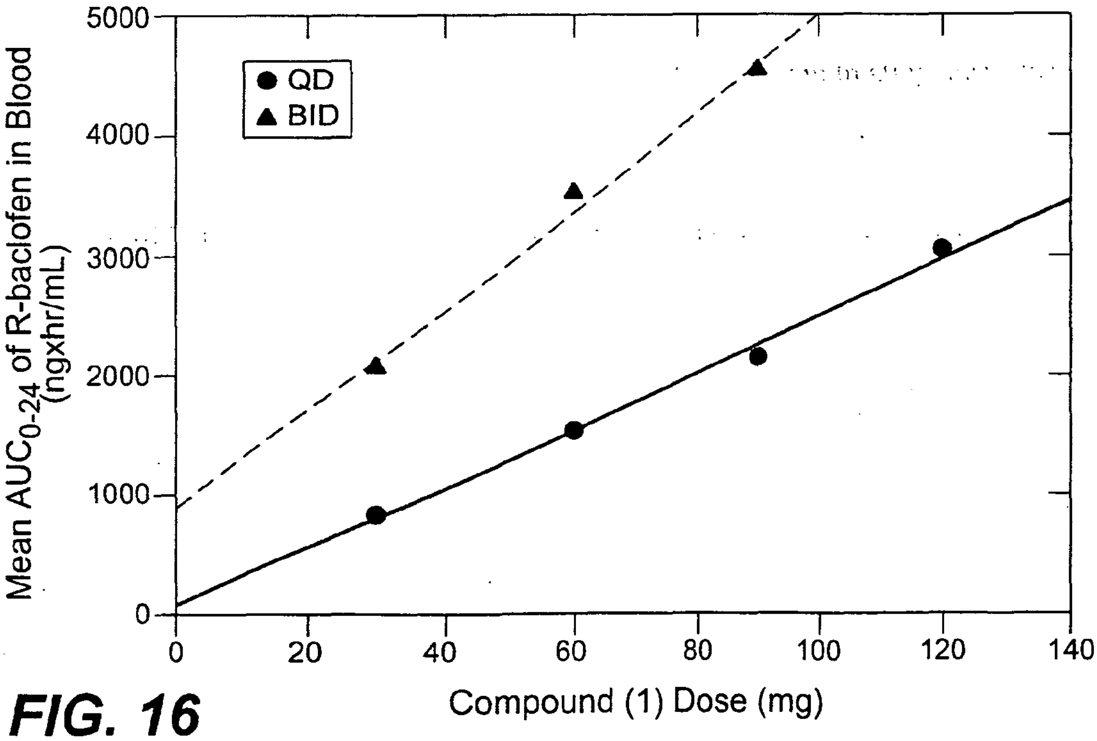
Mean $AUC_{0-24}$ of R-baclofen in Blood (ngxhr/mL)
QD
BID
0
1000
2000
3000
4000
5000
0
20
40
60
80
100
120
140
Compound (1) Dose (mg)

*FIG. 16*

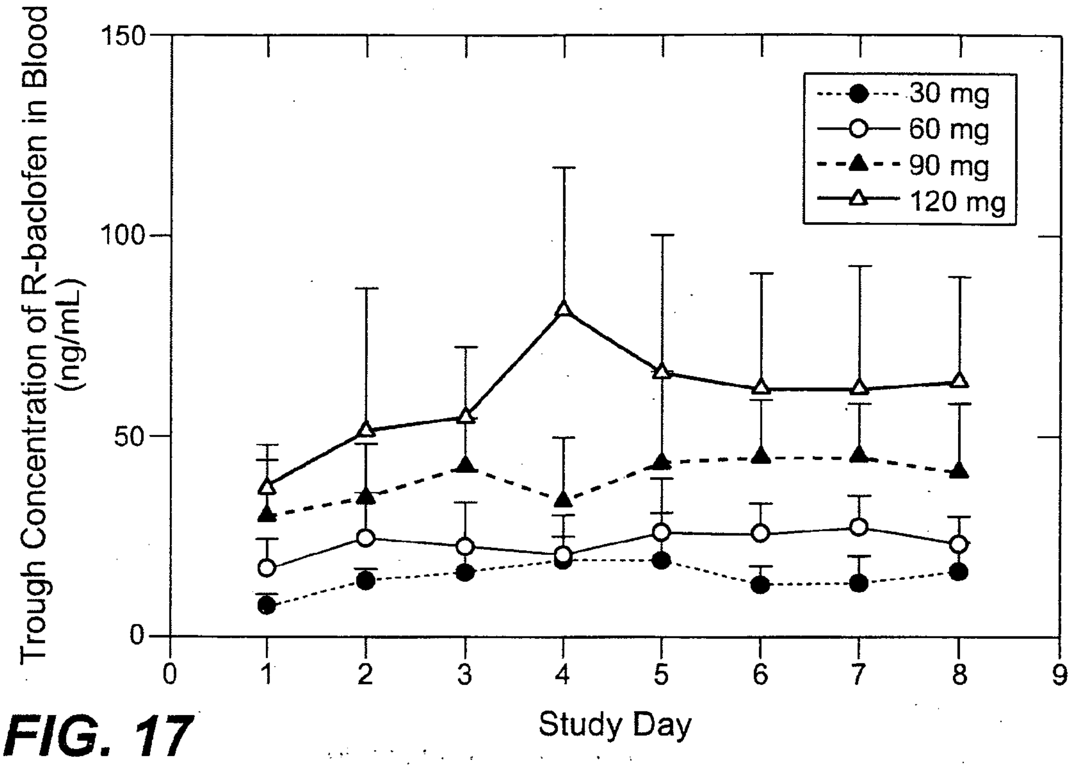
Trough Concentration of R-baclofen in Blood (ng/mL)
150
100
50
0
0
1
2
3
4
5
6
7
8
9
Study Day
30 mg
60 mg
90 mg
120 mg

*FIG. 17*

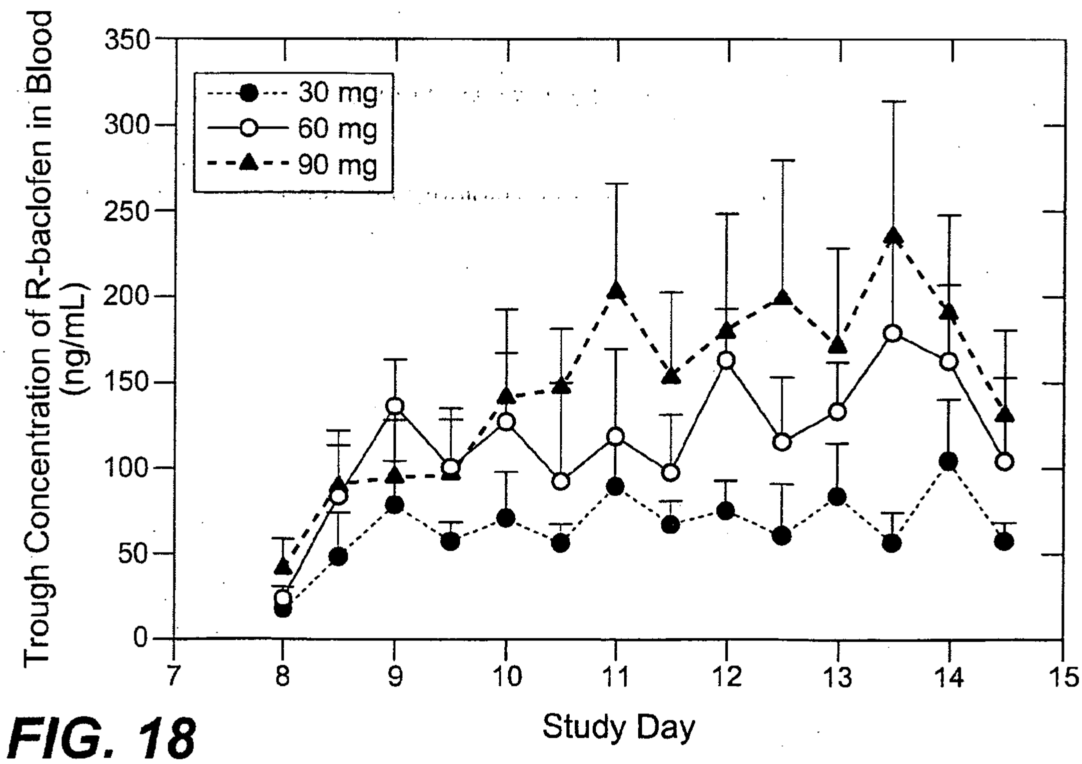
Trough Concentration of R-baclofen in Blood (ng/mL)
350
300
250
200
150
100
50
0
7
8
9
10
11
12
13
14
15
Study Day
30 mg
60 mg
90 mg

*FIG. 18*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6117908 A, Andrews
- WO 02096404 A
- WO 0126638 A
- WO 0108675 A
- US 4126684 A, Robson
- US 5719185 A, Bountra
- US 5006560 A, Kreutner
- WO 0190052 A
- EP 1178034 A, Bryans
- US 6992076 B, Cundy
- US 6818787 B, Gallop
- US 6927036 B
- US 6972341 B
- US 7232924 B, Raillard
- US 7109239 B, Gallop
- US 7227028 B
- WO 2005097079 A
- WO 2008011016 A
- US 20020151529 A, Cundy
- US 20030176398 A, Gallop
- US 20030171303 A, Gallop
- US 20040006132 A, Gallop
- US 20040014940 A, Raillard
- WO 9108740 A
- US 20040180959 A, Dooley
- US 20040138305 A
- US 20030133951 A, Coe
- US 200700892939 A, Lippa
- US 20070032500 A, Sun and Tafesse


### Non-patent literature cited in the description


- **Bowery.** *Trends Pharmacol. Sci.,* 1989, vol. 10, 401-407
- **Misgeld et al.** *Prog. Neurobiol.,* 1995, vol. 46, 423-462
- **van Herwaarden et al.** *Aliment. Pharmacol. Ther.,* 2002, vol. 16 (9), 1655-62
- **Ciccaglione ; Marzio.** *Gut,* 2003, vol. 52 (4), 464-70
- **Fromm et al.** *Neurology,* 1981, vol. 31 (6), 683-7
- **Ringel ; Roy.** *Ann Neurol,* 1987, vol. 21 (5), 514-5
- **Hering-Hanit.** *Cephalalgia,* 1999, vol. 19 (6), 589-591
- **Hering-Hanit ; Gadoth.** *Headache,* 2000, vol. 40 (1), 48-51
- **Freitag.** *CNS Drugs,* 2003, vol. 17 (6), 373-81
- **Slonimski et al.** *Reg Anesth Pain Med,* 2004, vol. 29 (3), 269-76
- **Katz.** *Am. J. Phys. Med. Rehabil.,* 1988, vol. 67 (3), 108-16
- **Krach.** *J Child Neurol.,* 2001, vol. 16 (1), 31-6
- **Sampathkumar et al.** *Anesth. Analg.,* 1998, vol. 87, 562-563
- **Terrence et al.** *Pharmacology,* 1983, vol. 27, 85-94
- **Sawynok et al.** *Pharmacology,* 1985, vol. 31, 248-259
- **Fromm et al.** *Neurology,* 1987, vol. 37 (11), 1725-8
- **van Bree et al.** *Pharm. Res.,* 1988, vol. 5, 369-371
- **Cercos-Fortea et al.** *Biopharm. Drug. Disp.,* 1995, vol. 16, 563-577
- **Deguchi et al.** *Pharm. Res.,* 1995, vol. 12, 1838-1844
- **Moll-Navarro et al.** *J. Pharm. Sci.,* 1996, vol. 85, 1248-1254
- **van Bree et al.** *Pharm. Res.,* 1991, vol. 8, 259-262
- **Ohtsuki et al.** *J Neurochem.,* 2002, vol. 83, 57-66
- **Wall et al.** *J. Med. Chem.,* 1989, vol. 32, 1340-1348
- Anesthesiology. American Society of Anesthesiologists, 2002, vol. 96, 1004-17
- **Ramsay et al.** *Br Med J,* 1974, vol. 2, 656-659
- **Chernik et al.** *J Clin Psychopharmacol,* 1990, vol. 10, 244-251
- **Sessler.** *Chest,* 2004, vol. 126, 1727-1730
- **Chisholm et al.** *Mayo Clin Proc,* 2006, vol. 81 (1), 46-52
- **Tonner et al.** *Best Pract Res Clin Anaesthesiol,* 2006, vol. 20 (1), 191-2000
- Handbook of Pharmaceutical Controlled Release Technology. Marcel Dekker, Inc, 2000

- Treatise on Controlled Drug Delivery, Fundamentals, Optimization, and Applications. Marcel Dekker, Inc, 1992
- **Ciccaglione ; Marzio.** *Gut,* 2003, vol. 52 (4), 464-470
- **Krach.** *J. Child Neurol.,* 2001, vol. 16 (1), 31-6
- **Lance et al.** *Trans Am. Neurol. Assoc.,* 1970, vol. 95, 272-274
- **Sanger et al.** *Pediatrics,* 2003, vol. 111, e89-e97
- **Eaton.** *J Rehab Res Dev,* 2003, vol. 40 (4), 41-54
- **Kakinohana et al.** *Neuroscience,* 2006, vol. 141, 1569-1583
- **Ligresti et al.** *British J Pharm,* 2006, vol. 147, 83-91
- **Zhang et al.** *Chinese J Clin Rehab,* 2006, vol. 10 (38), 150-151
- **Hefferan et al.** *Neuroscience Letters,* 2006, vol. 403, 195-200
- **Li et al.** *J Neurophysiol,* 2004, vol. 92, 2694-2703
- **Chai et al.** *Proc. Soc. Exptl. Biol. Med.,* 1962, vol. 109, 491
- **Wright ; Rang.** *Clin Orthop Relat Res,* 1990, vol. 253, 12-19
- **Shimizu et al.** *J Pharmacol Sci,* 2004, vol. 96, 444-449
- **Irwin.** *Psychopharmacologia,* 1968, vol. 13, 222-57
- **Dunham et al.** *J. Am. Pharm. Assoc.,* 1957, vol. 46, 208-09
- **Ligresti et al.** *British J Pharmacol,* 2006, vol. 147, 83-91
- **Zhang et al.** *Chinese J Clin Rehabilitation,* 2006, vol. 10 (38), 150-151
- **Loscher ; Schmidt.** *Epilepsy Res,* 1988, vol. 2 (3), 145-181
- **Priebe et al.** *Spinal Cord,* 1997, vol. 35 (3), 171-5
- **Gruenthal et al.** *Spinal Cord,* 1997, vol. 35 (10), 686-9
- **Tuszynski et al.** *Spinal Cord,* 2007, vol. 45, 222-231
- **Steeves et al.** *Spinal Cord,* 2007, vol. 45, 206-221
- **Hobart et al.** *Brain,* 2006, vol. 129 (1), 224-234
- **Lidums et al.** *Gastroenterology,* 2000, vol. 118 (1), 7-13
- **Vela et al.** *Aliment Pharmacol Ther,* 2003, vol. 17 (2), 243-51
- **Zhang et al.** *Gut,* 2002, vol. 50 (1), 19-24
- **Hornby et al.** *Gastroenterol Clin N Am,* 2002, vol. 31 (4), S11-S20
- **Castell et al.** *Am J. Gastroenterology,* 2006, vol. 101 (2), 59
- **Castell et al.** *Gastroenterology,* 2007, vol. A, 486
- **Blackshaw et al.** *Am. J. Physiol.,* 1999, vol. 277, G867-G874
- **Lehmann et al.** *Gastroenterology,* 1999, vol. 117, 1147-1154
- **Stakeberg ; Lehmann.** *Neurogastroenterol. Mot.,* 1999, vol. 11, 125-132
- **Suzuki et al.** *Neuropharmacology,* 2005, vol. 49 (8), 1121-31
- **Chan et al.** *Eur J Pharmacology,* 2007, vol. 559 (2-3), 196-201
- **Liu et al.** *Physiology & Behavior,* 2005, vol. 85, 271-277
- **Endo et al.** *Biogenic Amines,* 2004, vol. 18 (3-6), 419-434
- **Malik et al.** *Eur. J. Pharmacol,* 2007, vol. 555, 164-173
- **Rhodes et al.** *CA Cancer J Clin,* 2001, vol. 51, 232-248
- **Dicpinigaitis ; Dobkin.** *Chest,* 1997, vol. 111 (4), 996-9
- **Dicpinigaitis ; Rauf.** *Respiration,* 1998, vol. 65 (1), 86-8
- **Dicpinigaitis et al.** *J Clin Pharmacol,* 1998, vol. 38 (4), 364-7
- **Lewis et al.** *Pulmonary Pharmacology & Therapeutics,* 2007, vol. 20, 325-333
- **Brebner et al.** *Alcohol Alcohol,* 2002, vol. 37 (5), 478-84
- **Haney et al.** *Neuropsychopharmacology,* 2006, vol. 31, 1814-21
- **Heinzerling et al.** *Drug Alcohol Depend,* 2006, vol. 85 (3), 177-84
- **Assadi et al.** *BMC Psychiatry,* 2003, vol. 3 (16
- **Ahmadi-Abhari et al.** *J Clin Pharm Therapeutics,* 2001, vol. 26 (1), 67-71
- **Addolorato et al.** *Alcohol Alcohol,* 2002, vol. 37 (5), 504-8
- **Flannery et al.** *Alcohol Clin Exp Res,* 2004, vol. 28 (10), 1517-23
- **Markou et al.** *Ann N.Y. Acad Sci,* 2004, vol. 1025, 491-503
- **Cousins et al.** *Drug Alcohol Dependence,* 2002, vol. 65 (3), 209-20
- **Fattore et al.** *Alcohol & Alcoholism,* 2002, vol. 37 (5), 495-498
- **Spano et al.** *Neuropharmacology,* 2007, vol. 52, 1555-1562
- **Maccioni et al.** *Alcohol,* 2005, vol. 36, 161-168
- **Jensen et al.** *Eur J Pharmacol,* 2001, vol. 429, 1-11
- **Baron.** *Nat Clin Pract Neurol,* 2006, vol. 2, 95-106
- **Beggs ; Salter.** *Drug Dev Res,* 2006, vol. 67, 289-301

- **Sindrup ; Jensen.** *Pain,* 1999, vol. 83, 389-400
- **Hwang ; Yaksh.** *Pain,* 1997, vol. 70 (1), 15-22
- **Smith et al.** *Neruopharmacology,* 1994, vol. 33 (9), 1103-8
- **Patel et al.** *Pain,* 2001, vol. 90 (3), 217-26
- **Balerio ; Rubio.** *Pharmacol Res,* 2002, vol. 46 (3), 281-6
- **Reis ; Duarte.** *Br J Pharmacol,* 2006, vol. 149 (6), 733-9
- **Herman et al.** *Clin J Pain,* 1992, vol. 8 (4), 338-45
- **Gatscher et al.** *Acta Neurochir,* 2002, vol. 79, 75-76
- **Van Hilten et al.** *N Engl J Med,* 2000, vol. 343 (9), 625-30
- **Becker et al.** *J Clin Neurosci,* 2000, vol. 7 (4), 316-9
- **Zuniga et al.** *Reg Anesth Pain Med,* 2002, vol. 27 (1), 90-3
- **Anghinah et al.** *Muscle Nerve,* 1994, vol. 17 (8), 958-59
- **Fromm et al.** *Ann Neurol,* 1984, vol. 15 (3), 240-4
- **Beggs ; Salter.** *Drug Dev Res,* 2006, vol. 67, 829-301
- **Lombard et al.** *Pain,* 1979, vol. 6 (2), 163-174
- **Kim ; Chung.** *Pain,* 1992, vol. 50, 355-363
- **Wall et al.** *Pain,* 1979, vol. 7, 103-111
- **Mosconi ; Kruger.** *Pain,* 1996, vol. 64, 37-57
- **Seltzer et al.** *Pain,* 1990, vol. 43, 205-218
- **Bennett ; Xie.** *Pain,* 1988, vol. 33, 87-107
- **Hao et al.** *Pain,* 1991, vol. 45, 175-185
- **von Heijne et al.** *Eur J Pain,* 2001, vol. 5, 1-10
- **Decosterd ; Woolf.** *Pain,* 2000, vol. 87, 149-158
- **DeLeo et al.** *Pain,* 1994, vol. 56, 9-16
- **Chacur et al.** *Pain,* 2001, vol. 94, 231-244
- **Herzberg ; Sagen.** *J Neuroimmunol,* 2001, vol. 116, 29-39
- **Kupers et al.** *Pain,* 1998, vol. 76 (1-2), 45-59
- **Slart et al.** *Pain,* 1997, vol. 69, 119-125
- **Fox et al.** *Pain,* 1999, vol. 81, 307-316
- **Joseph et al.** *Pain,* 2004, vol. 107, 147-158
- **Dirig ; Yaksy.** *J Pharmacology Exper Ther,* 1995, vol. 275, 219-227
- **Aley et al.** *Neuroscience,* 1996, vol. 73, 259-265
- **Cavaletti et al.** *Exp Neurol,* 1995, vol. 133, 64-72
- **Authier et al.** *Neurosci Lett,* 2000, vol. 25, 2576-2585
- **Meleger ; Krivickas.** *Neurol Clin,* 2007, vol. 25, 419-438
- **Meleger ; Krivickas.** *Neurological Clinics,* 2007, vol. 25 (2), 419-438
- **Hering-Hanit.** *Cephalalgia,* 1999, vol. 19 (6), 589-91
- **Dapas et al.** *Spine,* 1985, vol. 10 (4), 345-9
- **Raphael et al.** *BMC Musculoskeletal Disorders,* 2002, vol. 3 (17
- **Kehl et al.** *Pain,* 2000, vol. 85, 333-343
- **Taylor-Gjevre ; Gjevre.** *Lupus,* 2005, vol. 14 (6), 486-8
- **Crofford et al.** *Arthritis & Rheumatism,* 2005, vol. 52 (4), 1264-1273
- **Eaton.** *J Rehabilitation Research and Development,* 2003, vol. 40 (4), 41S-54S
- **Guay.** *Am J Geriatr Pharmacother,* 2005, vol. 3, 274-287
- **Freynhagen et al.** *Pain,* 2005, vol. 115, 254-263
- **Backonja et al.** *Clin Ther.,* 2003, vol. 25, 81-104
- **Gidal et al.** *Am J Manag Care,* 2006, vol. 12, S269-S278
- **Argoff.** *JAOA,* 2002, vol. 102 (9), S21-S26
- **Chaplan et al.** *J Neurosci. Meth.,* 1994, vol. 53, 55-63
- **Fox et al.** *Pain,* 2003, vol. 105, 355-362
- **Milligan et al.** *Brain Res.,* 2000, vol. 861, 105-116
- **De Vry et al.** *Eur. J Pharmacol.,* 2004, vol. 491, 137-148
- **Polomano et al.** *Pain,* 2001, vol. 94, 293-304
- **Shimizu et al.** *JPharmacol Sci,* 2004, vol. 96, 444-449
- **Dunham et al.** *J Am. Pharm. Assoc.,* 1957, vol. 46, 208-09
- **Malik et al.** *JPharmacology,* 2007, vol. 555, 164-173
- **Abbott et al.** *Pain,* 1995, vol. 60, 91-102
- **Hargreaves et al.** *Pain,* 1988, vol. 32, 77-88
- **Mogil et al.** *Pain,* 1999, vol. 80, 67-82
- **Chaplan et al.** *J Neurosci Methods,* 1994, vol. 53, 55-63
- **Dixon.** *Rev Pharmacol Toxicol,* 1980, vol. 20, 441-462
- **Nozaki-Taguchi et al.** *Pain,* 2001, vol. 93, 69-76
- **Brennan et al.** *Pain,* 1996, vol. 64, 493-501